# EUROPEAN PATENT APPLICATION

(11) **EP 2 754 720 A1**
(43) Date of publication of application: **16.07.2014**
(21) Application number: 13153098.2
(22) Date of filing: 25.02.2008
(51) Int. Cl.: C12Q 1/68

(54) **Prostate cancer survival and recurrence**

(30) Priority: 23.02.2007 US 891477 P
(62) Divisional of application: 08730593.4
(71) Applicant: Aviaradx, Inc., San Diego, CA 92121 (US); General Hospital Corporation, Boston, MA 02114 (US)
(72) Inventor: Ma, Xiao-Jun, San Diego, CA 92130 (US); Wu, Chin-Lee, Newton, MA 02459 (US); Erlander, Mark G., Carlsbad, CA 92011 (US)
(74) Representative: Roques, Sarah Elizabeth

(57) **Abstract**

The disclosure includes the identification and use of gene expression profiles, or patterns, with clinical relevance to prostate cancer. In particular, the disclosure is based on the identities of genes that are correlated with patient survival and prostate cancer recurrence. The gene expression profiles may be embodied in nucleic acid expression, protein expression, or other expression formats and used to predict the survival of subjects afflicted with prostate cancer and to predict prostate cancer recurrence. The profiles may also be used in the study and/or diagnosis of prostate cancer cells and tissue as well as for the study and/or determination of prognosis of a patient. When used for diagnosis or prognosis, the profiles may be used to determine the treatment of prostate cancer based upon probable life expectancy and cancer recurrence and/or metastasis.

## Description

### RELATED APPLICATIONS

This application claims benefit of priority from U.S. Provisional Patent Application 60/891,477, filed February 23, 2007 (the 12-month anniversary of which is Saturday, February 23, 2008), which is hereby incorporated by reference as if fully set forth,.

### FIELD OF THE DISCLOSURE

The disclosure relates to the identification and use of gene (or sequence) expression profiles, or patterns, with clinical relevance to prostate cancer. In particular, the disclosure is based on the identification of genes, or expressed sequences, the expression of which are correlated with patient survival, prostate cancer recurrence, and/or occurrence of prostate cancer metastasis. The gene expression profiles, whether embodied in nucleic acid expression, protein expression, or other expression formats, may be used to predict the survival of subjects afflicted with prostate cancer, predict prostate cancer recurrence, and/or predict occurrence of prostate cancer metastasis. The profiles may also be used in the study and/or diagnosis of prostate cancer cells and tissue as well as for the study and/or determination of prognosis of a patient. When used for diagnosis or prognosis, the profiles are used to determine the treatment of prostate cancer based upon the likelihood of life expectancy, cancer recurrence, and/or cancer metastasis.

### BACKGROUND OF THE DISCLOSURE

Prostate cancer is the most commonly diagnosed cancer in men in the US, with approximately 240,000 newly diagnosed patients and 40,000 cancer death each year. Diagnosing and managing prostate cancer are clinically difficult due to lack of the knowledge of the cancer at molecular and genetic levels and a lack of understanding of the natural disease progression.

Prostate cancer research is limited by an inadequate resource of prostate cancer tissue specimens available for research use, particularly those specimens that are well characterized pathologically, appropriately preserved for RNA and protein studies, and linked with patient clinical, pathologic, and follow-up information.

Surgical treatment is used in treating some cases of prostate cancer, such as T2 prostate cancer. However, a fraction of patients will fail the treatment. This is judged by chemical failure (elevation of PSA after one year of surgery) or development of metastasis (commonly in lymph nodes and bone). The ability to identify, or predict, a post-surgery patient as likely to encounter PSA failure, recurrence of the prostate cancer, and/or development of metastasis, would provide multiple benefits to the patient.

### BRIEF SUMMARY OF THE DISCLOSURE

The disclosure includes the identification and use of gene (or sequence) expression patterns (or profiles or "signatures") which are clinically relevant to prostate cancer. The gene expression profiles, whether embodied in nucleic acid expression, protein expression, or other expression formats, may be used to predict prostate cancer recurrence and/or survival of subjects afflicted with prostate cancer. In embodiments involving use of prostatectomy, the disclosure includes methods to predict the likelihood of prostate cancer recurrence, cancer metastasis, and/or recurrence of elevated PSA levels.

In one aspect, the disclosure includes methods to identify, and then use, gene (or sequence) expression profiles which provide prognostic information related to prostate cancer. The expression profiles correlate with (and so are able to discriminate between) patients with good or poor cancer recurrence, cancer metastasis, and/or survival outcomes. In some embodiments, the disclosure includes a method to identify expression profiles relevant to cancer recurrence and/or metastasis in prostate cancer afflicted subjects. In other embodiments, the disclosure includes a method to compare gene (or sequence) expression in a sample of prostate cancer cells from a patient to an identified expression profile to determine the likely outcome for the patient, such as after prostatectomy. These embodiments of the disclosure may be advantageously used to meet an important unmet diagnostic need for the ability to predict whether a patient will likely benefit from surgery to treat prostate cancer or whether a patient will be better off with another type of treatment or need adjuvant treatment in addition to surgery.

One non-limiting example of a patient that will likely benefit from surgery, such as a radical prostatectomy, is one predicted, by the expression profile of the patient's prostate cancer cells as disclosed herein, to be free of cancer recurrence and/or metastasis following the surgery. In a related manner, a non-limiting example of a patient that may be expected to not benefit from surgery is one predicted, by the expression profile of the patient's prostate cancer cells as disclosed herein, to develop cancer metastasis following the surgery. By way of example, the recurrence of prostate cancer may be at the same location while metastasis is frequently to a different location or tissue, such as to a lymph node or bone as non-limiting examples.

In further embodiments, a method to identify a patient, from a population of patients with prostate cancer cells, or following prostatectomy, as belonging to a subpopulation of patients with a better cancer recurrence and/or survival outcome (such as lower risk of recurrence or metastasis), relative to another subpopulation, or as belonging to a subpopulation with a poorer cancer recurrence and/or survival outcome (such as elevated risk of recurrence or metastasis), relative to another subpopulation. The method is thus able to distinguish patients into at least two subpopulations or subtypes.

The disclosure includes a non-subjective means for the identification of patients with prostate cancer, optionally following prostatectomy, as likely to have a better or poorer cancer recurrence, cancer metastasis, and/or survival outcome by assaying for an expression pattern disclosed herein. Thus where subjective interpretation (such as that based upon immunohistochemical staining and subjective analysis) may have been previously used to determine the prognosis and/or treatment of prostate cancer patients, or post-prostatectomy patients, this disclosure introduces objective expression patterns, which may used alone or in combination with other (subjective and/or objective) criteria to provide a more accurate assessment of patient outcomes, including survival and the recurrence or metastasis of cancer. The expression patterns of the disclosure thus include a means to determine prostate cancer, or post-prostatectomy, prognosis.

The expression patterns of the disclosure comprise more than one gene, or expressed sequence, capable of discriminating between prostate cancer, or post-prostatectomy, outcomes with significant accuracy. The expression level(s) of the gene(s), or expressed sequence(s) are identified as correlated with outcomes such that the expression level(s) is/are relevant to a determination of the preferred treatment protocols of a patient. Thus, the disclosure includes a method to determine the outcome of a subject afflicted with prostate cancer, optionally post-prostatectomy, by assaying a prostate cancer cell containing sample from said subject for expression levels that are correlated with patient outcomes as disclosed herein.

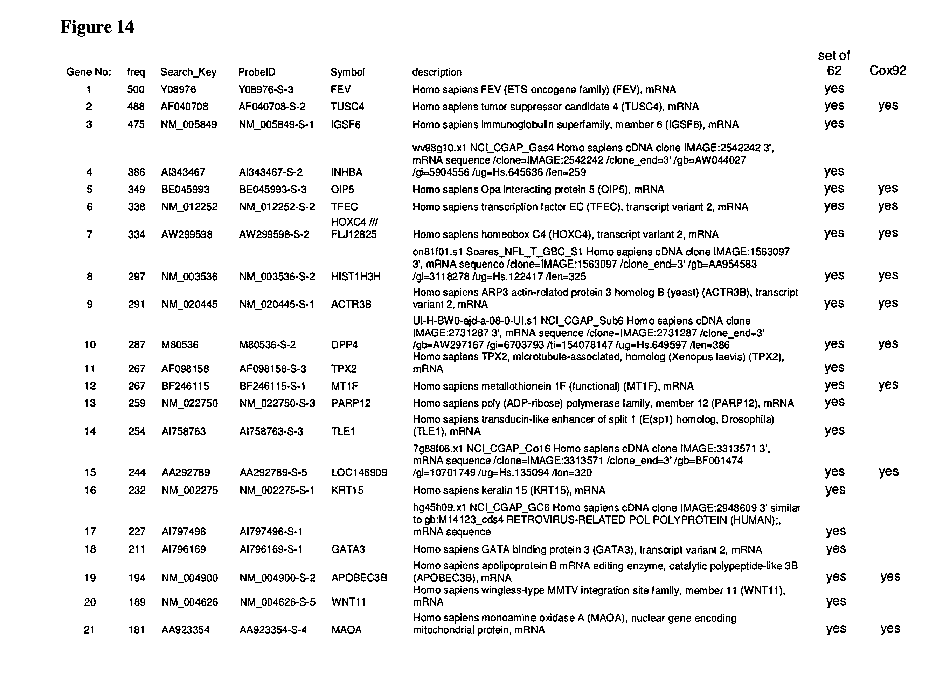

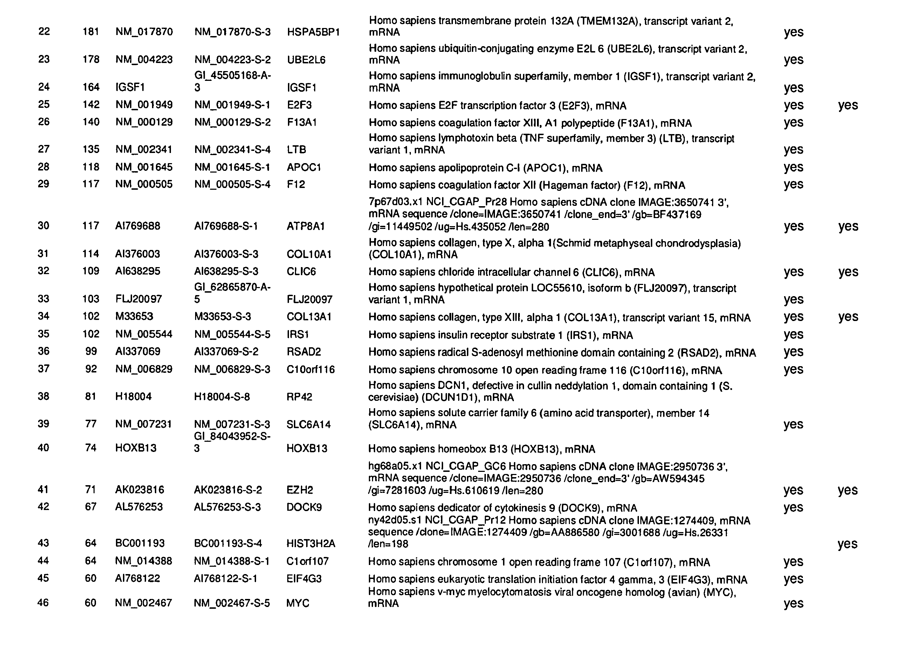

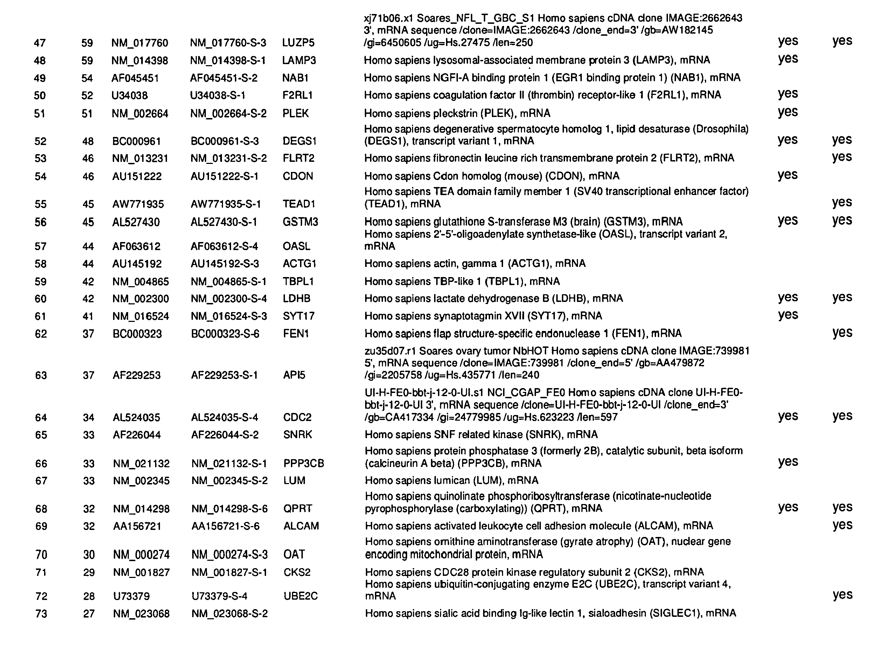

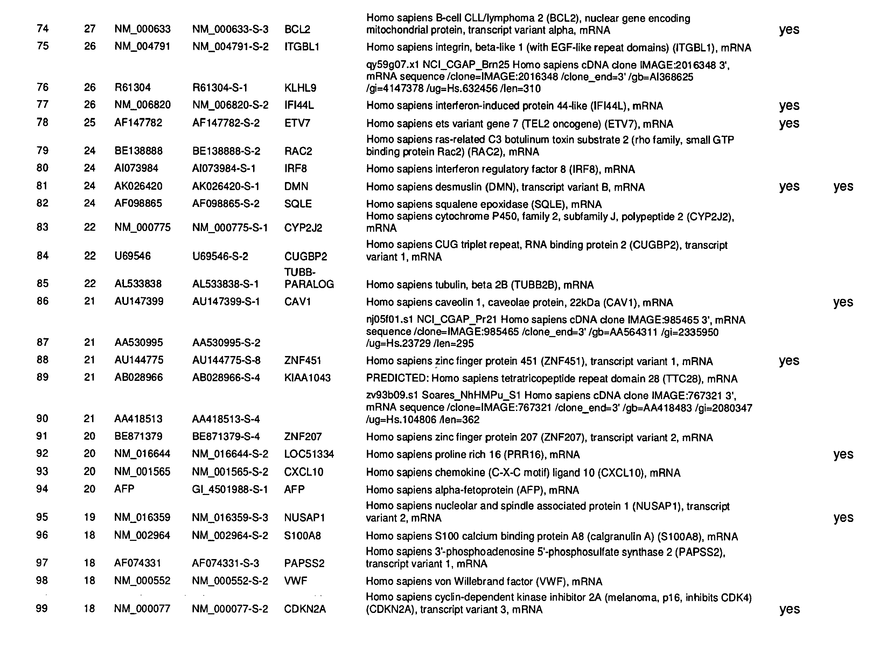

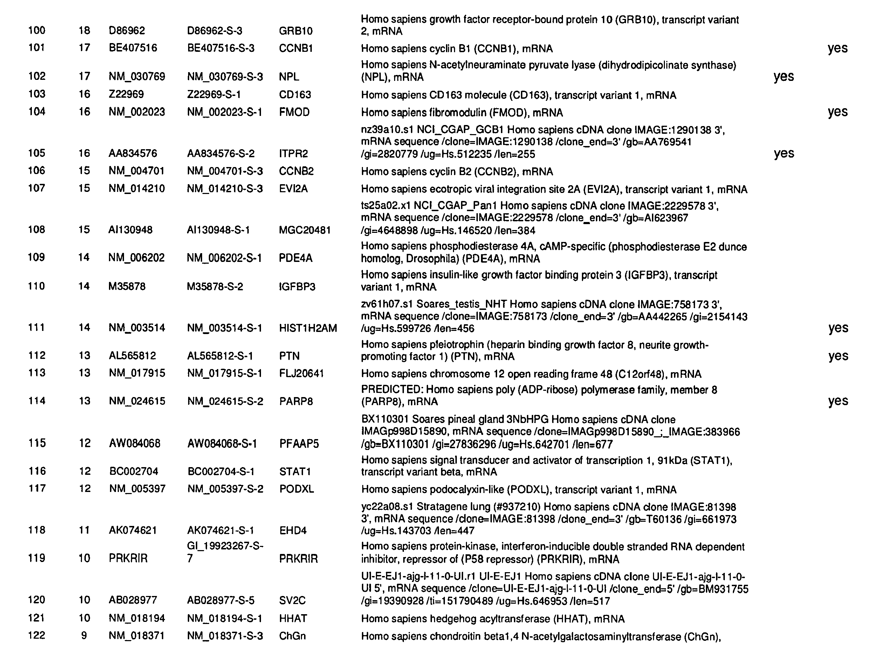

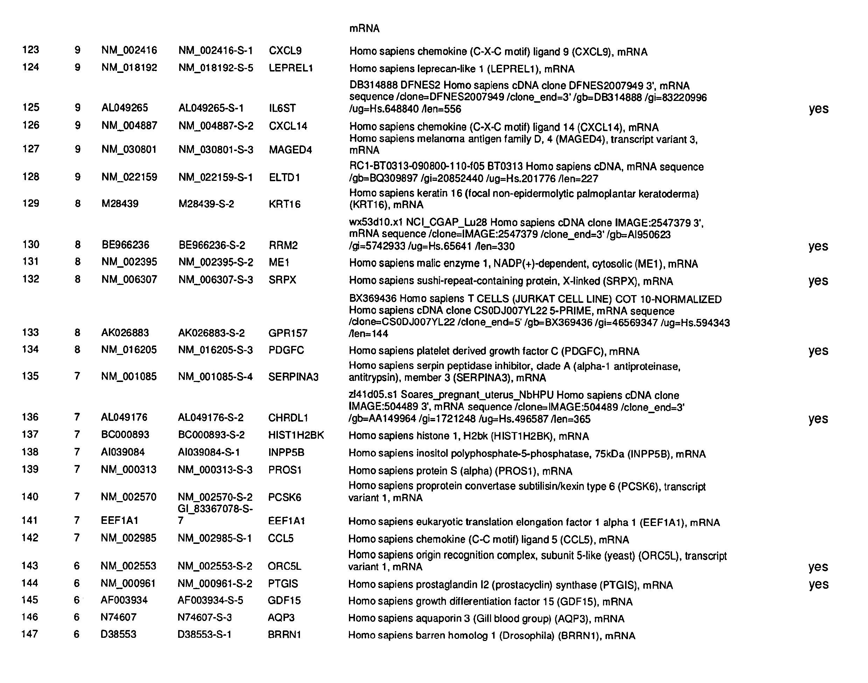

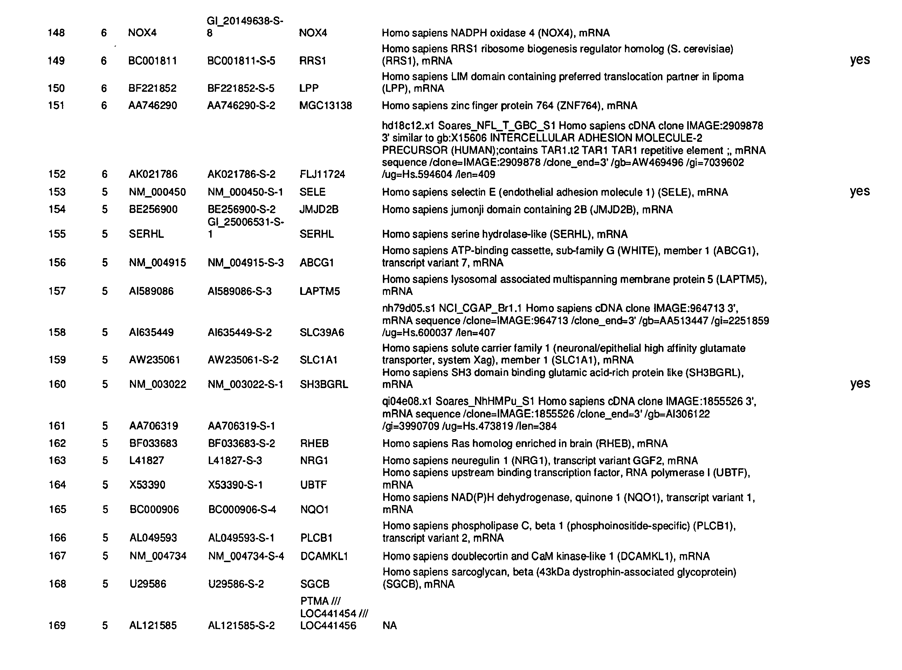

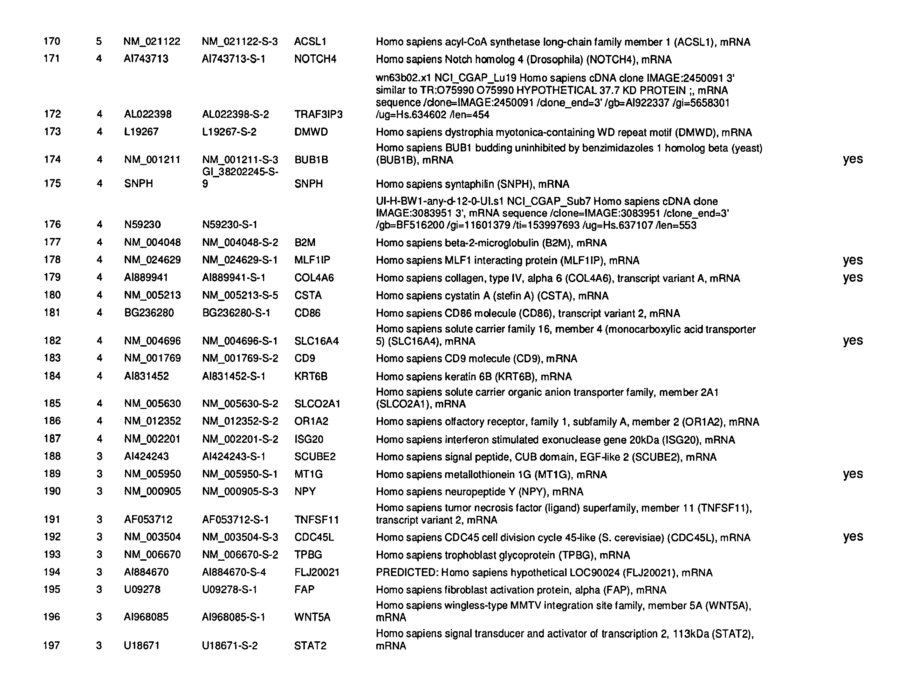

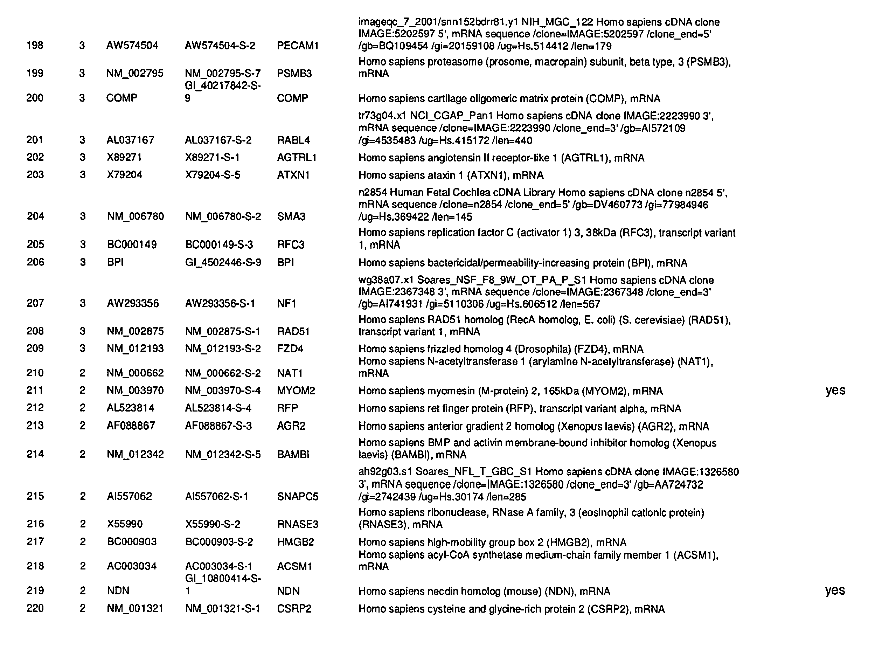

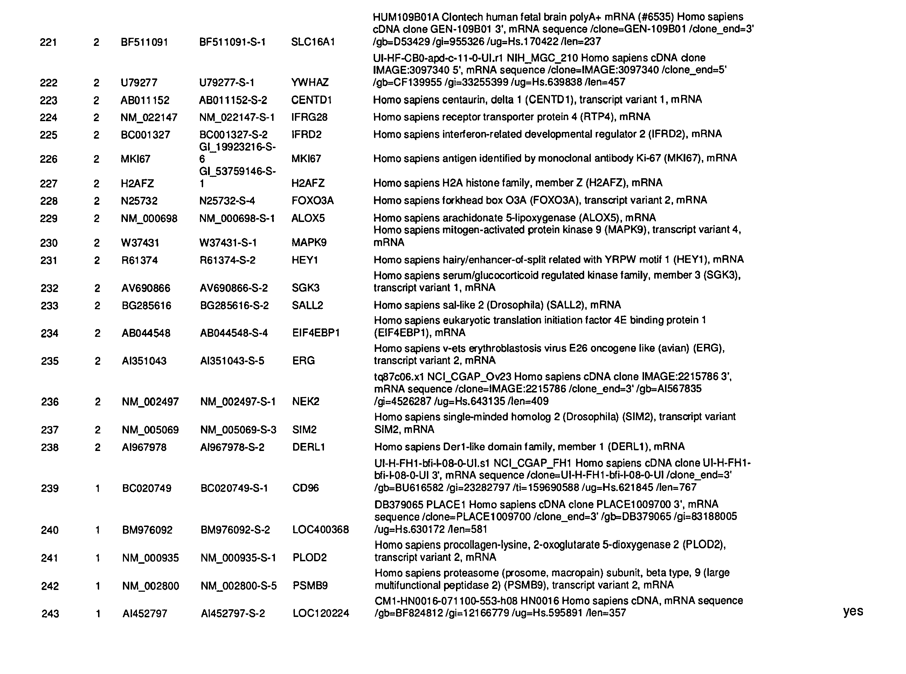

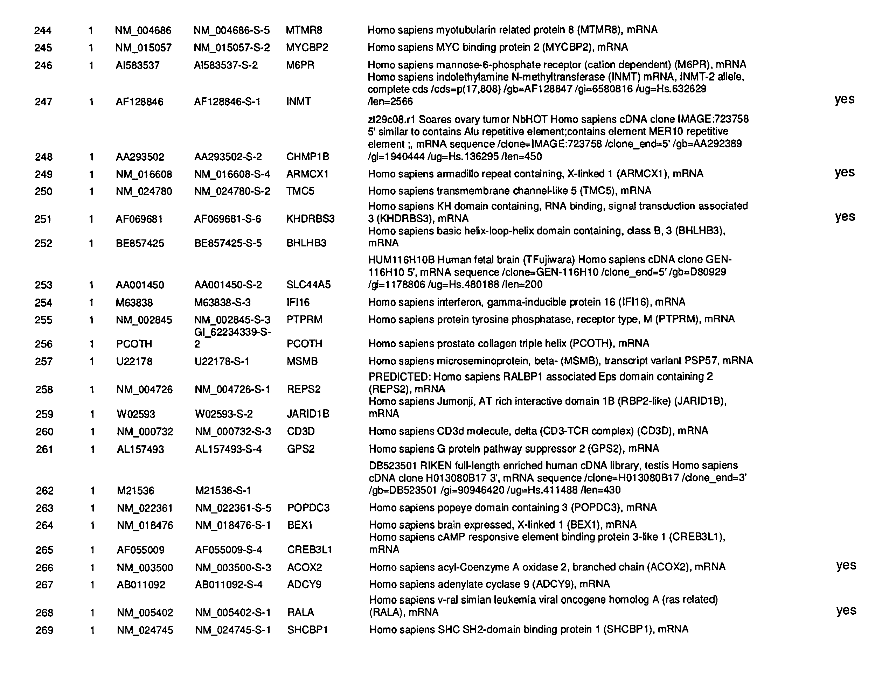

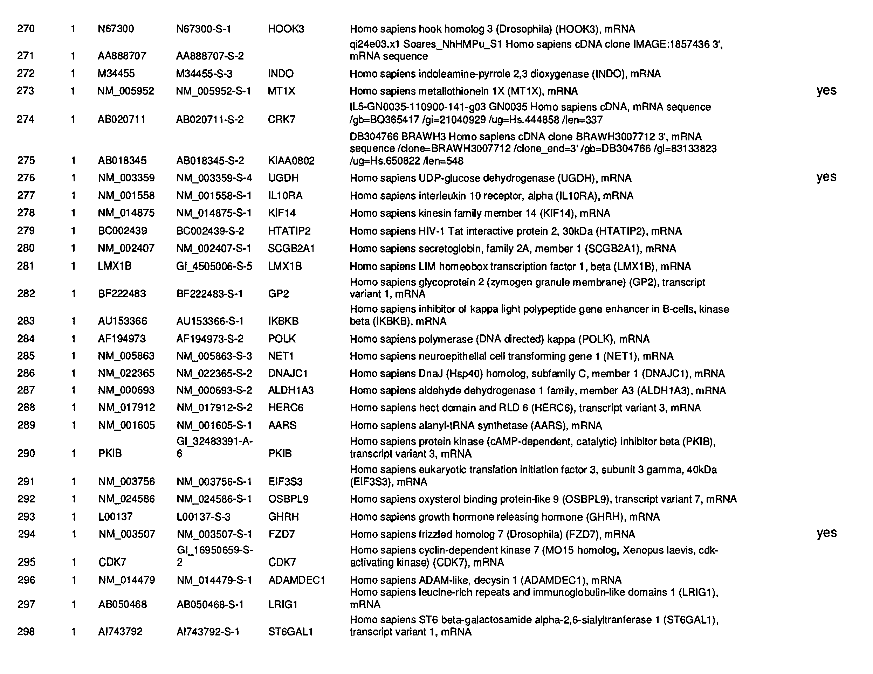

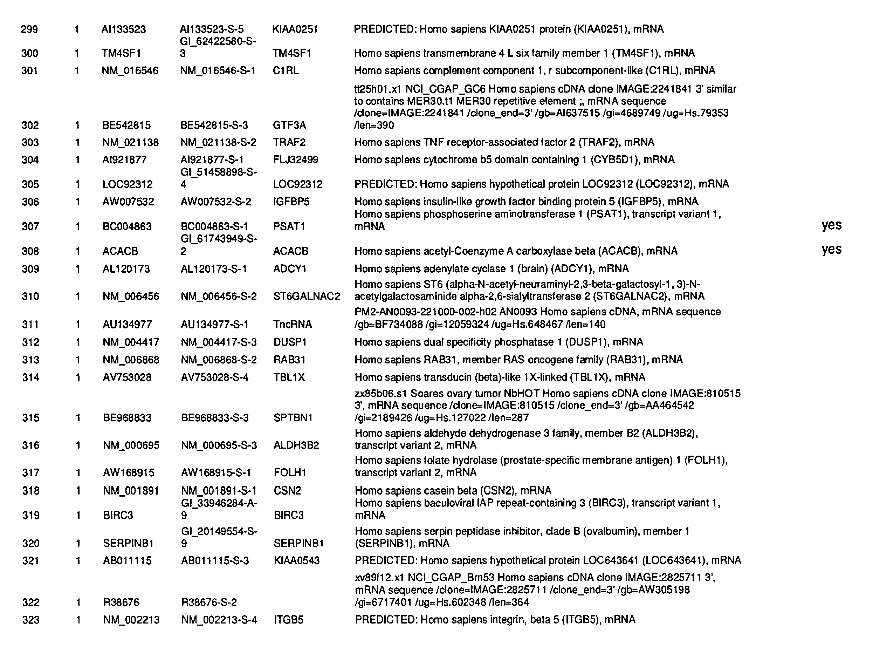

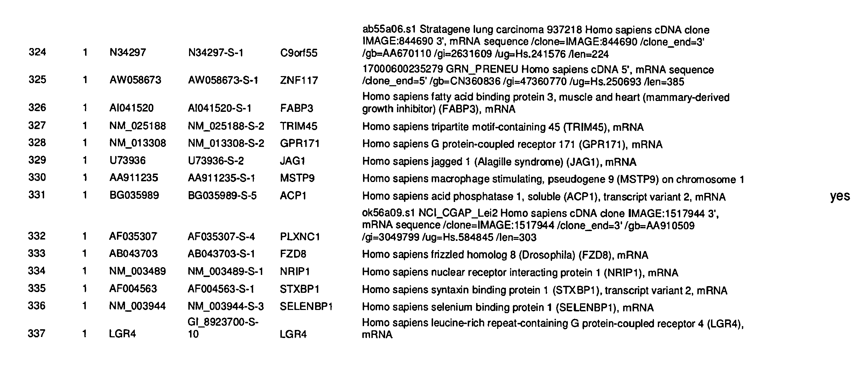

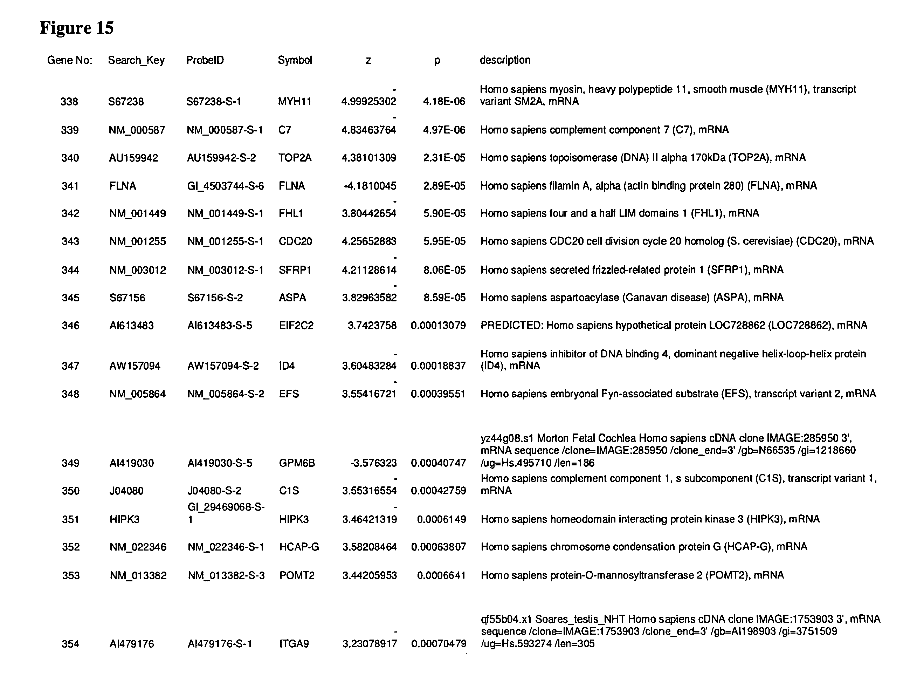

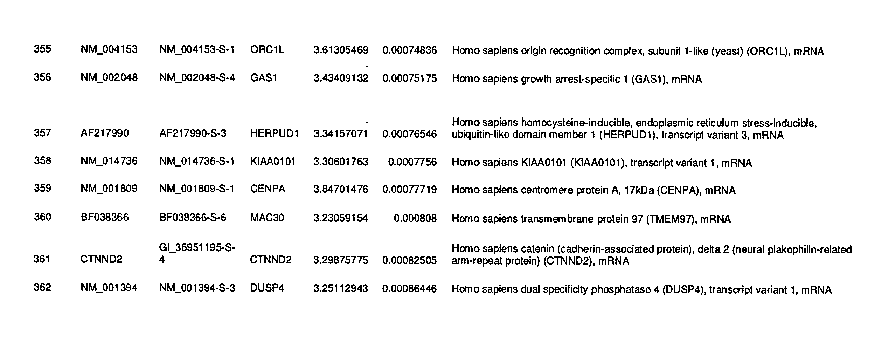

In another aspect, the disclosure includes a method to determine the risk of prostate cancer recurrence and/or metastasis in a subject. The method includes assaying a sample comprising prostate cancer cells from said subject for the expression levels of two or more genes selected from Gene Nos: 1 to 362 in Figures 14 and 15 herein, and comparing the expression level of each gene individually, or the aggregated expression levels *in toto,* with the mean or median expression level(s) thereof in a population of prostate cancer cells. This assessment may then be used to determine the risk of prostate cancer recurrence and/or metastasis in the subject from which the sample was obtained. The expression levels of the disclosed genes (or expressed sequences) are correlated with a low risk of cancer recurrence and/or metastasis, or a high risk of cancer recurrence and/or metastasis, based upon comparison to the mean or median level of expression in a population of prostate cancer cells. Put differently, the individual genes (or expressed sequences) disclosed herein are expressed at higher, or lower, levels (in comparison to the mean or median level of expression in a prostate cancer cell population) such that the deviation from the mean or median is correlated with an higher or lower risk as described herein. Each aggregation of deviations in two or more genes (or expressed sequences) is an expression pattern or profile of the disclosure.

In some embodiments, the assaying for expression levels may be performed by use of two or more nucleic acid probes selected from SEQ ID NOs: 1-362 as disclosed herein. These probes hybridize, under appropriate conditions, and detect expressed sequences as disclosed herein. In other embodiments, other probes which hybridize to the same expressed sequences as SEQ ID NOs: 1-362 may be used. These additional probes may be prepared or selected by analysis of the expressed sequences detected by SEQ ID NOs: 1-362. In other cases, additional probe sequence may be all or part of the sequences identified as Gene Nos: 1-362. In many embodiments, a probe may be used to detect a region of sequence amplified from an expressed sequence (or gene) as described herein.

The detection or determination of expression levels of two or more (gene) sequences may be referred to as detecting or determining an expression profile or signature. Expression patterns of the disclosure comprising two or more of the disclosed sequences are identified and used as described herein. For their identification, a large sampling of the gene expression levels in a sample containing prostate cancer cells is obtained through quantifying the expression levels of mRNA. In one embodiment, the disclosure includes detecting gene (or sequence) expression levels by analyzing global, or near global, gene expression from single cells or homogenous cell populations which have been dissected away from, or otherwise isolated or purified from, contaminating cells beyond that possible by a simple biopsy. Because the expression of numerous genes fluctuate between cells from different patients as well as between cells from the same patient sample, multiple data from expression of individual gene sequences are used as reference data to generate models which in turn permit the identification of individual genes and sequences, the expression of which are correlated with particular prostate cancer, or post-prostatectomy, outcomes.

The expression levels of various genes in these models are then analyzed to identify nucleic acid sequences, the expressions of which are positively, or negatively, correlated, with a prostate cancer, or post-prostatectomy, outcome. This disclosure includes nucleic acid sequences, which are a subset of expressed sequences in a cell, identified as correlating to outcomes as described herein. An expression pattern or profile of the disclosure includes a combination of these identified sequences (or genes). The use of multiple samples for identification of expressed sequences increases the confidence with which a gene is considered to correlate with a prostate cancer recurrence, metastasis, and/or survival outcome. Without sufficient confidence, it remains unpredictable whether a particular gene is actually correlated with an outcome and also unpredictable whether expression of a gene may be successfully used to identify the outcome for a prostate cancer, or post-prostatectomy, subject or patient. Once identified, two or more nucleic acid sequences corresponding to the disclosed genes (or expressed sequences) may be selected for assessment as an expression profile to be detected or assessed for its predictive properties.

A profile of expression levels that is highly correlated with one outcome relative to another may be used to assay a prostate cancer cell containing sample from a subject or patient to predict the outcome of the subject from whom the sample was obtained. Such an assay may be used as part of a method to determine the therapeutic treatment for said subject based upon the outcome identified.

The correlated genes may be used in pairs (with significant accuracy) or in or in higher numbers to increase the ability to accurately correlate a molecular expression phenotype with a prostate cancer, or post-prostatectomy, outcome. This correlation provides a molecular determination of prostate cancer recurrence, cancer metastasis, and/or survival outcomes as disclosed herein. Without being bound by theory, and offered to improve understanding of the instant disclosure, the disclosed molecular determination is more powerful than other molecular indicators related to prostate cancer, such as the determination of prostate serum antigen (PSA) levels. Additional uses of the correlated expression patterns and profiles are in the classification of cells and tissues; determination of diagnosis and/or prognosis; and determination and/or alteration of therapy.

The ability to discriminate is conferred by the identification of expression of the individual gene sequences as relevant and not by the form of the assay used to determine the actual level of expression. An assay may utilize any identifying feature of an identified individual gene as disclosed herein as long as the assay reflects, quantitatively or qualitatively, expression of the gene sequence in the "transcriptome" (the transcribed fraction of genes in a genome) or the "proteome" (the translated fraction of expressed genes in a genome). Identifying features include, but are not limited to, unique nucleic acid sequences used to encode (DNA), or express (RNA), said gene or epitopes specific to, or activities of, a protein encoded by said gene. All that is required is the identity of the gene sequence(s) necessary to discriminate between prostate cancer, or post-prostatectomy, outcomes and an appropriate cell containing sample for use in an expression assay.

Similarly, the nature of the cell containing sample is not limiting, as fresh tissue, freshly frozen tissue, and fixed tissue, such as formalin-fixed paraffin-embedded (FFPE) tissues, may be used in the disclosed methods. In some embodiments, the sample may be that of a needle (core) biopsy or other biopsy.

For detecting an identified expression pattern or profile, the disclosure includes detecting gene (or sequence) expression patterns by gathering global, or near global, gene expression from single cells or homogenous cell populations which have been dissected away from, or otherwise isolated or purified from, contaminating cells beyond that possible by a simple biopsy. The expression levels of the genes (sequences) in the pattern or profile are then detected or otherwise measured. In other embodiments, a method may only detect or measure the expression levels of the genes (sequences) in the profile without assessment or other determination of the expression of other genes or sequences.

In an additional aspect, the analysis of expression levels may be performed in combination with, or in place of, other assessments or indicators of prostate cancer. In some embodiments, the analysis is made in combination with a method of determining the grade of prostate cancer in a sample comprising prostate cancer cells from a subject. In other embodiments, the combination is with a method of determining the stage of prostate cancer in the sample. A third possibility is combination with detecting or determining PSA levels in the subject, optionally before a procedure used to isolate the prostate cancer cells. Of course a combination with any one, two, or all three of these representative examples is possible. Whenever more than one type of assessment is used, the result is a multivariate analysis. The disclosure expressly includes all possible combinations of assessments described herein as multivariate embodiments.

Generally, any accepted method of assessing prostate cancer grade and/or stage as known to the skilled person may be used. In some cases, the method of determining prostate cancer grade comprises determination of a Gleason Score (or Gleason Grade). In other cases, the method of determining prostate cancer stage comprises a determination according to the American Joint Committee on Cancer (AJCC) tumor staging system for assessing prostate cancer stage. And as described herein, the analysis of gene (sequence) expression levels may be performed in place of either the Gleason Score or the AJCC tumor stage determination.

In cases of PSA levels, its assessment may be conducted before a prostatectomy which is used to provide a sample comprising prostate cancer cells for use in any method described herein..

In a further aspect, the disclosure includes physical and methodological means for detecting the expression of genes (or sequences) disclosed herein. These means may be directed to assaying one or more aspect of the DNA template(s) underlying the expression of the gene (or sequence), of the RNA used as an intermediate to express the gene (or sequence), or of the proteinaceous product expressed by the gene (or sequence).

One advantage provided by the disclosure is that contaminating, non-prostate cancer cells (such as infiltrating lymphocytes or other immune system cells) may be removed to reduce their effect on measurements of the expression patterns or profiles disclosed herein to predict the cancer recurrence and/or survival outcomes of patients. Such contamination is present where a biopsy containing many cell types is used to generate gene expression profiles.

While the present disclosure is described mainly in the context of human prostate cancer, it may be practiced in the context of prostate cancer of any animal known to be potentially afflicted by prostate cancer. Non-limiting examples of animals for the application of the present disclosure are mammals, particularly those important to agricultural applications (such as, but not limited to, cattle, sheep, horses, and other "farm animals"), animal models of prostate cancer, and animals for human companionship (such as, but not limited to, dogs and cats).

### BRIEF DESCRIPTION OF THE FIGURES

Figure 1 illustrates a representative data analysis scheme, based on human patient samples as described in Example 1 and Table 1 herein.
Figure 2 shows the result of 500 gene selection iterations of the Random Forests™ algorithm as described herein.
Figure 3A shows a representative identification of prostate cancer on a slide containing a radical prostatectomy specimen (FFPE tissue) with an area of cancerous tissue identified. Figure 3B shows the excised (macro-dissected) prostate cancer material after removal of non-cancerous tissue.
Figure 4 shows correlation between the gene risk signature and Gleason Grade or score (<=6, 7 or >=8) of samples from the training and test sets.
Figure 5 shows correlation between the gene risk signature and AJCC pathologic tumor stage (II or III) of samples from the training and test sets.
Figure 6 shows correlation between the gene risk signature and pre-operative PSA (prostate serum antigen) value of samples from the training and test sets.
Figure 7 shows results from Kaplan-Meier analysis for freedom from PSA failure (increase of PSA values) after surgical intervention to treat prostate cancer. The results show segregation into a "low" risk subpopulation with better cancer recurrence and probability of survival outcomes (lower incidence of recurrence and death due to metastasis) and a "high" risk subpopulation with poorer outcomes (higher incidence of recurrence and metastasis).

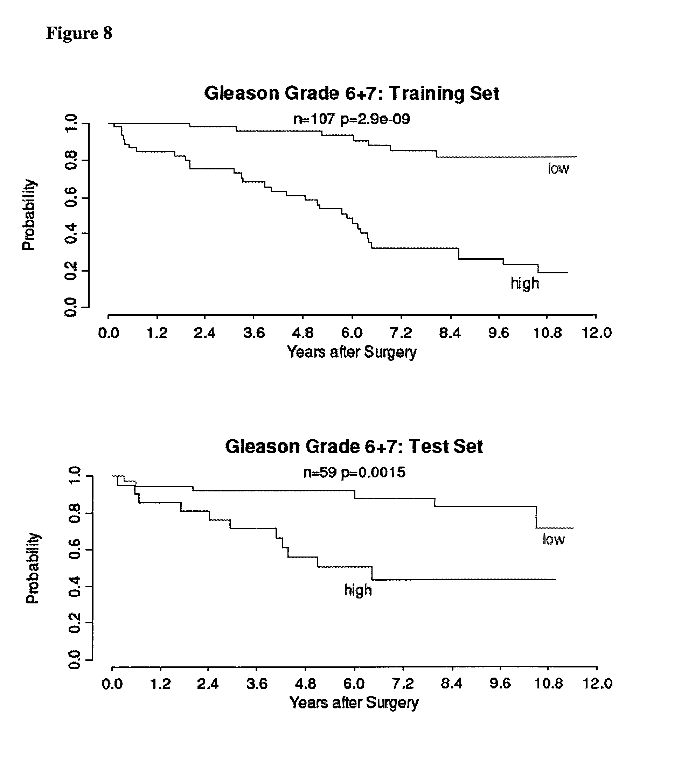

Figure 8 shows results from Kaplan-Meier analysis for freedom from PSA failure after surgical intervention to treat prostate cancer in cells samples with Gleason Grade or scores of <=6 and 7. The results show segregation into a "low" risk subpopulation with better cancer recurrence and probability of survival outcomes (lower incidence of recurrence and death due to metastasis) and a "high" risk subpopulation with poorer cancer recurrence outcomes (higher incidence of recurrence and metastasis). Similar results are observed if the samples are separated into those with Gleason Grade or score of <=6, score of 7, or score of >=8.

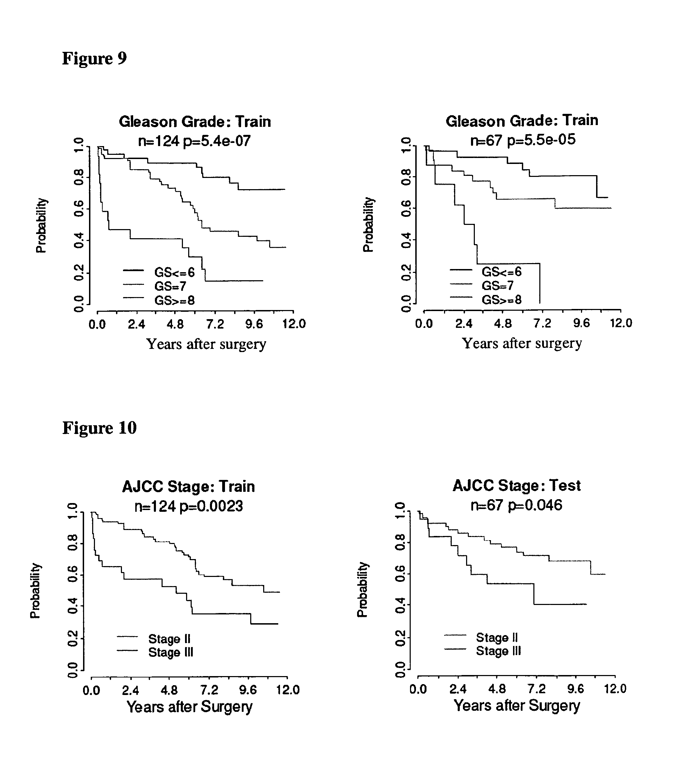

Figure 9 shows survival curves based on Gleason Grade alone.
Figure 10 shows survival curves based on AJCC Stage alone.

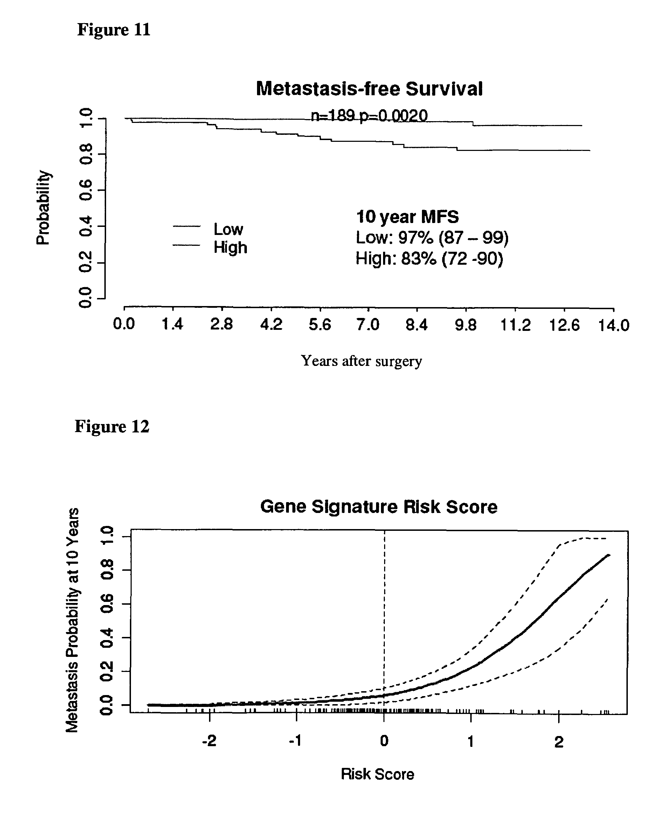

Figure 11 shows the results of using a disclosed Risk Score (based on 62 representative genes) to segregate 189 patients into a "low" risk subpopulation with better probability of survival outcomes (lower incidence of death due to metastasis) and a "high" risk subpopulation with poorer outcomes (higher incidence of metastasis) over 10 years.
Figure 12 shows a plot of the Figure 11 Risk Score versus the probability of metastasis within 10 years. As indicated by the graph, a Score over 0 in prostate cancer cells of a subject indicates an increasing risk of metastasis occurring within 10 years following surgical intervention.

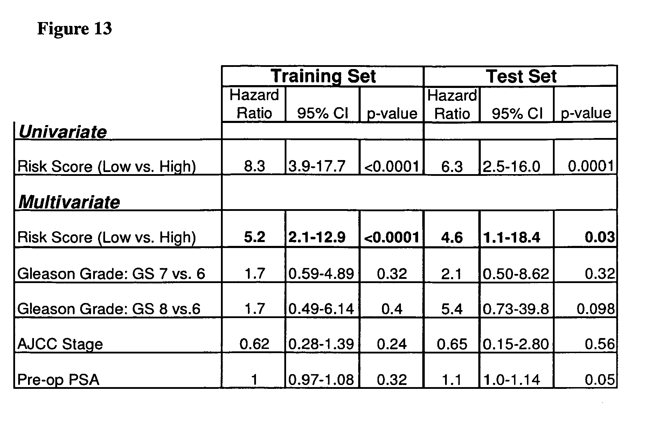

Figure 13 summarizes and compares the use of univariate and multivariate analysis for PSA failure. The indicated AJCC Stage is II versus III. CI refers to confidence interval.
Figure 14 lists the annotations and identifying information for 337 genes for use as described herein. The Gene No. may be used to cross reference each gene with the corresponding SEQ ID NO of the probe and its performance information in Table 1 below. The frequency with which the identified molecules occur among 500 sets generated (as described in Example 1) is indicated in the "freq" column. The remaining columns contain identifying information for each gene, such as gene accession number ("Search Key" column), accession number for the corresponding probe sequence in Table 1 ("ProbeID" column), gene name symbol ("Symbol" column), and a brief description (last column).
Figure 15 lists the annotations and identifying information for an additional 25 genes for use as described herein. The Gene No. and performance information (z and p values) may be used to cross reference each gene with the corresponding SEQ ID NO of the probe and its performance information in Table 3 below. Identifying information for each gene includes gene accession number ("Search Key" column), accession number for the corresponding probe sequence in Table 3 ("ProbeID" column), gene name symbol ("Symbol" column), and a brief description (last column).

### DETAILED DESCRIPTION OF MODES OF PRACTICING THE DISCLOSURE

### Definitions of terms

A gene expression "pattern" or "profile" or "signature" refers to the relative expression of two or more genes (expressed sequences) between two or more prostate cancer, or post-prostatectomy, cancer recurrence, metastasis, and/or survival outcomes which expression is correlated with being able to distinguish between the outcomes.

A "gene" or "expressed sequence" is a polynucleotide that encodes, and expresses in a detectable manner, a discrete product, whether RNA or proteinaceous in nature. It is appreciated that more than one polynucleotide may be capable of encoding a discrete product. The term includes alleles and polymorphisms of a gene or expressed sequence that encodes the same product, or a functionally associated (including gain, loss, or modulation of function) analog thereof, based upon chromosomal location and ability to recombine during normal mitosis.

The terms "correlate" or "correlation" or equivalents thereof refer to an association between expression of two or more genes and a physiologic state of a prostate cell to the exclusion of one or more other states as identified by use of the methods as described herein. A gene may be expressed at higher or lower levels and still be correlated with one or more prostate cancer, or post-prostatectomy, state or outcome. One way to express correlation is with a z value as disclosed herein for the expression of various genes (or expressed sequences). The z value may be viewed as indicating the strength, or weight, of the association between the expression level of a gene (or expressed sequence) and a particular outcome, such as cancer recurrence, cancer metastasis, and/or survival over time. The value may have a positive or negative (+/-) sign which arbitrarily, but consistently, indicates over or under expression, respectively. Because the signs are arbitrary, their assignment can be readily (but consistently) reversed without deleterious effect.

In some embodiments, the strength (or weight) is multiplied by the expression level of a given gene (or expressed sequence), after normalization thereof (such as relative to expression of a reference gene that is expressed at relatively constant levels) to provide a value or score for the expression of that gene (or sequence). In many cases, the expression data is normalized, median-centered, and log-transformed as known to the skilled person prior to further use, such as in clustering and discriminant analysis. Where more than one expression level is used (as in the case of a gene expression profile or pattern), the values or scores may be summed and then analyzed as an aggregate value for assessing the correlation or conducting classification based on the correlation.

A "polynucleotide" is a polymeric form of nucleotides of any length, either ribonucleotides or deoxyribonucleotides. This term refers only to the primary structure of the molecule. Thus, this term includes double- and single-stranded DNA and RNA. It also includes known types of modifications including labels known in the art, methylation, "caps", substitution of one or more of the naturally occurring nucleotides with an analog, and internucleotide modifications such as uncharged linkages (e.g., phosphorothioates, phosphorodithioates, etc.), as well as unmodified forms of the polynucleotide.

The term "amplify" is used in the broad sense to mean creating an amplification product can be made enzymatically with DNA or RNA polymerases. "Amplification," as used herein, generally refers to the process of producing multiple copies of a desired sequence, particularly those of a sample. "Multiple copies" mean at least 2 copies. A "copy" does not necessarily mean perfect sequence complementarity or identity to the template sequence.

The term "corresponding" can refers to, where appropriate, a nucleic acid molecule as sharing a substantial amount of sequence identity with another nucleic acid molecule. Substantial amount means at least 95%, usually at least 98% and more usually at least 99%, and sequence identity is determined using the BLAST algorithm, as described in Altschul et al. (1990), J. Mol. Biol. 215:403-410 (using the published default setting, i.e. parameters w=4, t=17). Methods for amplifying mRNA are generally known in the art, and include reverse transcription PCR (RT-PCR) and those described in U.S. Patent Application 10/062,857 (filed on October 25, 2001), as well as U.S. Provisional Patent Applications 60/298,847 (filed June 15, 2001) and 60/257,801 (filed December 22, 2000), all of which are hereby incorporated by reference in their entireties as if fully set forth. Another method which may be used is quantitative PCR (or Q-PCR). Alternatively, RNA may be directly labeled as the corresponding cDNA by methods known in the art.

A "microarray" is a linear or two-dimensional array of discrete regions, each having a defined area, formed on the surface of a solid support such as, but not limited to, glass, plastic, or synthetic membrane. The density of the discrete regions on a microarray is determined by the total numbers of immobilized polynucleotides to be detected on the surface of a single solid phase support, such as at least about 50/cm², at least about 100/cm², at least about 500/cm², but below about 1,000/cm² in some embodiments. The arrays may contain less than about 500, about 1000, about 1500, about 2000, about 2500, or about 3000 immobilized polynucleotides in total. As used herein, a DNA microarray is an array of oligonucleotides or polynucleotides placed on a chip or other surfaces used to hybridize to amplified or cloned polynucleotides from a sample. Because the position of each particular group of polynucleotides in the array is known, the identities of a sample polynucleotides can be determined based on their binding to a particular position in the microarray.

Because the disclosure relies upon the identification of genes (or expressed sequences) that are over- or under-expressed, one embodiment of the disclosure involves determining expression by hybridization of mRNA, or an amplified or cloned version thereof (such as DNA or cDNA), of a sample cell to a polynucleotide that is unique to a particular gene sequence. Polynucleotides of this type may contain at least about 20, at least about 22, at least about 24, at least about 26, at least about 28, at least about 30, or at least about 32 consecutive basepairs of a gene sequence that is not found in other gene sequences. The term "about" as used in the previous sentence refers to an increase or decrease of 1 from the stated numerical value. Other embodiments may use polynucleotides of at least or about 50, at least or about 100, at least about or 150, at least or about 200, at least or about 250, at least or about 300, at least or about 350, or at least or about 400 basepairs of a gene sequence that is not found in other gene sequences. The term "about" as used in the preceding sentence refers to an increase or decrease of 10% from the stated numerical value. Such polynucleotides may also be referred to as polynucleotide probes that are capable of hybridizing to sequences of the genes, or unique portions thereof, described herein. In many cases, the hybridization conditions are stringent conditions of about 30% v/v to about 50% formamide and from about 0.01M to about 0.15M salt for hybridization and from about 0.01M to about 0.15M salt for wash conditions at about 55 to about 65°C or higher, or conditions equivalent thereto.

In other embodiments, other probes which hybridize to the same expressed sequences as SEQ ID NOs: 1-362 may be used. These additional probes may be prepared or selected by analysis of the expressed sequences detected by SEQ ID NOs: 1-362. Such additional polynucleotide probes for use in the disclosure may have about or 95%, about or 96%, about or 97%, about or 98%, or about or 99% identity with the gene sequences to be used. Identity is determined using the BLAST algorithm, as described above. These additional probes may also be described on the basis of the ability to hybridize to expressed genes and sequences of the disclosure under stringent conditions as described above or conditions equivalent thereto.

In many cases, the sequences are those of mRNA encoded by the genes, the corresponding cDNA to such mRNAs, and/or amplified versions of such sequences. In some embodiments of the disclosure, the polynucleotide probes are immobilized on an array, other devices, or in individual spots that localize the probes. In many embodiments, the probes are directed to a region of a gene (or expressed sequence) that is, or contains, a part of a 3' untranslated region. In some cases, the region is within about 100, 200, 300, 400, 500, 600, 700, 800, or 900 or more nucleotides of the polyadenylation signal or polyadenylated tail in an mRNA.

Alternatively, and in another embodiment of the disclosure, gene expression may be determined by analysis of expressed protein in a cell sample of interest by use of one or more antibodies specific for one or more epitopes of individual gene products (proteins) in said cell sample. Such antibodies are may be labeled to permit their easy detection after binding to the gene product.

The term "label" refers to a composition capable of producing a detectable signal indicative of the presence of the labeled molecule. Suitable labels include radioisotopes, nucleotide chromophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical or chemical means.

The term "support" refers to conventional supports such as beads, particles, dipsticks, fibers, filters, membranes and silane or silicate supports such as glass slides.

As used herein, a "prostate tissue sample" or "prostate cancer cell sample" refers to a sample of prostate tissue isolated from an individual, such as one afflicted with prostate cancer. The sample may be from material removed via a prostatectomy, such as a radical prostatectomy. Alternatively, they are obtained by other means, such as needle (core) biopsy or other biopsy techniques, like laterally directed biopsies, the conventional sextant biopsy approach, different combinations of sextant and lateral biopsies as extended techniques, transrectal ultrasound guided prostate biopsy, and others as known to the skilled person. Such samples are primary isolates (in contrast to cultured cells) and may be collected by any suitable means recognized in the art. In some embodiments, the "sample" may be collected by an invasive method, including, but not limited to, surgical biopsy. A sample may contain prostate tumor cells which are isolated by known methods or other appropriate methods as deemed desirable by the skilled practitioner. Isolation methods include, but are not limited to, microdissection, laser capture microdissection (LCM), or laser microdissection (LMD) before use in accordance with the disclosure.

"Expression" and "gene expression" include transcription and/or translation of nucleic acid material.

As used herein, the term "comprising" and its cognates are used in their inclusive sense; that is, equivalent to the term "including" and its corresponding cognates.

Conditions that "allow" an event to occur or conditions that are "suitable" for an event to occur, such as hybridization, strand extension, and the like, or "suitable" conditions are conditions that do not prevent such events from occurring. Thus, these conditions permit, enhance, facilitate, and/or are conducive to the event. Such conditions, known in the art and described herein, depend upon, for example, the nature of the nucleotide sequence, temperature, and buffer conditions. These conditions also depend on what event is desired, such as hybridization, cleavage, strand extension or transcription.

Sequence "mutation," as used herein, refers to any sequence alteration in the sequence of a gene disclosed herein interest in comparison to a reference sequence. A sequence mutation includes single nucleotide changes, or alterations of more than one nucleotide in a sequence, due to mechanisms such as substitution, deletion or insertion. Single nucleotide polymorphism (SNP) is also a sequence mutation as used herein. Because the present disclosure is based on the level of gene (or sequence) expression, mutations in non-coding, but regulatory, regions of genes as disclosed herein may also be assayed in the practice of the disclosure.

"Detection" includes any means of detecting, including direct and indirect detection of gene expression and changes therein. For example, "detectably less" products may be observed directly or indirectly, and the term indicates any reduction (including the absence of detectable signal). Similarly, "detectably more" product means any increase, whether observed directly or indirectly.

Prostatectomy refers to the removal of prostate tissue by a skilled clinician, such as a surgeon. Non-limiting examples include radical prostatectomy; open (traditional) prostatectomy (involving an incision through the perineum); laparoscopic prostatectomy; and robotic (nerve sparing) prostatectomy.

Gleason score refers to the grading of a sample of prostate cancer by a trained pathologist according to the Gleason system, which assigns a Gleason score using numbers from 1 to 5 based upon similarities in the cells of a sample of prostate tissue to cancerous tissue or normal prostate tissue. Tissue that looks much like normal prostate tissue is given a score or grade of 1 while a tissue that lacks normal features and the cells seem to be spread haphazardly through the prostate is given a score or grade of 5. Scores, or grades of 2 through 4, inclusive, have features in between these possibilities. But because prostate cancers may have areas with different scores or grades, separate scores or grades are given to the two areas that make up most of the tissue. The two scores or grades are added to yield a Gleason score (or Gleason sum) between 2 and 10.

Unless defined otherwise all technical and scientific terms used herein have the same meaning as commonly understood to one of ordinary skill in the art to which this disclosure belongs.

### General

The disclosure is based in part upon the discovery of gene (or sequence) expression-based predictors of prostate serum antigen (PSA) failure by using separate training and test sets of prostate cancer samples from archived FFPE tissues from 1993-1995. The samples had related long-term follow up clinical data from the patients, including those with no evidence of disease (NED) for at least 10 years post-surgery and those that developed a subsequent recurrence (such as distant metastasis), from whom the samples were obtained. 62, 92, 337 and 362 genes (or expressed sequences), from a starting set of about 1536 genes with a high degree of dynamic expression (or variation in expression levels) within different cancer types, were identified via RNA-based expression profiling of the samples. The expression levels of these genes correlate with PSA failure and clinical outcomes as described herein and therefore may be used as predictors as described herein. The identified genes have been reported to participate in cellular functions including cell cycle, blood coagulation and wound healing, transcription regulation, and apoptosis.

The predictive capability of the disclosed gene expression pattern (profile) may be correlated to Gleason score and AJCC tumor stage. It is also consistent with pre-operative PSA levels. The profile also has the ability to stratify samples with Gleason scores of <=6 and 7 into low and high risk of PSA failure. In multivariate analysis with known prognostic factors, the profile was the only predictor that remains statistically significant (p < 0.05). Therefore, detection of the profile at biopsy, or in tissue removed during prostatectomy, allows the distinguishing of indolent from aggressive cancers.

The disclosure includes the identification and use of gene expression patterns (or profiles or "signatures") which discriminate between (or are correlated with) prostate cancer survival and recurrence outcomes in a subject. Such patterns may be determined by the methods of the disclosure by use of a number of reference cell or tissue samples, such as those reviewed by a pathologist of ordinary skill in the pathology of prostate cancer, which reflect prostate cancer cells as opposed to normal or other non-cancerous cells. The outcomes experienced by the subjects from whom the samples may be correlated with expression data to identify patterns that correlate with the outcomes. Because the overall gene expression profile differs from person to person, cancer to cancer, and cancer cell to cancer cell, correlations between certain cells and genes expressed or underexpressed may be made as disclosed herein to identify genes that are capable of discriminating between prostate cancer outcomes.

### Identification and description of genes and sequences

The disclosure may be practiced with any two or more of the disclosed genes (expressed sequences) found to be differentially expressed with respect to prostate cancer, or post-prostatectomy, outcomes, such as post-surgical PSA rise of > 0.2 ng ("PSA failure"). The identification was made by using expression profiles of various homogenous prostate cancer cell populations. The expression level of each gene of the expression profile may be correlated with a particular outcome. Alternatively, the expression levels of two or more genes (expressed sequences) may be clustered (combined) and used based on correlations with particular outcomes. Non-limiting examples of outcomes include but not limited to, distant metastasis, such as to a lymph node or bone, or need for follow-up chemotherapy or radiation therapy or cryotherapy. In additional embodiments, the expression profile may be used to stratify subjects or patients into a differential prognosis of "watchful waiting" versus "need of further treatment" where the latter may include neoadjuvant or adjuvant therapy beyond surgery to remove the prostate cancer/tumor. The disclosed methods may thus be used as part of prostate cancer management.

Genes with significant correlations to prostate cancer (or post-prostatectomy) survival, metastasis, or recurrence outcomes are used to discriminate between outcomes. Alternatively, genes with significant correlations may be used in combination with genes with lower correlations to form an expression pattern or profile of the disclosure without significant loss of ability to discriminate between outcomes. Such combinations (or expression patterns) may be selected and tested by any appropriate means recognized in the art, including, but not limited to, cluster analysis, supported vector machines, neural networks or other algorithm known in the art. The patterns or profiles are capable of predicting the classification of an unknown sample based upon the expression of the genes used for discrimination in the models. "Leave one out" cross-validation may be used to test the performance of various combinations and to help identify weights (expressed genes or sequences) that are less informative or detrimental to the predictive ability of a combination. Cross-validation may also be used to identify genes or sequences, the expression of which enhance the predictive ability of the models.

The disclosed genes (sequences) expressed in correlation with particular prostate cancer, or post-prostatectomy, outcomes provide the ability to focus gene expression analysis to only those genes that contribute to the ability to stratify a subject among different outcomes. The expression of other genes in a prostate cancer cell would be relatively unable to provide information concerning, and thus assist in the differential prognosis (or discrimination) of, a prostate cancer outcome.

As will be appreciated by those skilled in the art, the combinations (expression patterns or profiles) are highly useful even when based on a small set of reference gene expression data and can become increasingly accurate with the inclusion of more reference data although the incremental increase in accuracy will likely diminish with each additional datum. The preparation of additional reference gene expression data using genes identified and disclosed herein for discriminating between different outcomes in prostate cancer, or post-prostatectomy, is routine and may be readily performed by the skilled artisan to permit the generation of models as described above to predict the status of an unknown sample based upon the expression levels of those genes.

The disclosure includes the expressed sequences in the following Table 1 and expressed genes in Figure 14.

**Table 1**

| SEQ ID NO: | freq | Probe_Sequence | z | P | set of 62 | Cox92 |
|---|---|---|---|---|---|---|
| 1 | 500 | GGCTCCTAGAAGCCCCATTCAATATCACTACTCTTTAACGAGTGCC | -3.00978 | 0.004298 | yes | |
| 2 | 488 | TGACTGGATGGACACATTGCTGTGGGTAGTCCCTCCTACTAGGA | 3.250421 | 0.000$78 | yes | yes |
| 3 | 475 | CCAACTATGAAAGGCCATAGAAACGTTTTAATTTTCAATGAAGTCACTGA | 1.295466 | 0.199714 | yes | |
| 4 | 386 | ATATTTTGGTGTTTGCGAGGCATGCAGTCAATATTTTGTACAGTTAGT | 2.775622 | 0.00728 | yes | |
| 5 | 349 | TGCATGGTTGGTCTGAAAATAGAGTTGGGCTTAATGTTGACTTCTATTAC | 5.04529 | 6.51 E-06 | yes | yes |
| 6 | 338 | TCACATCAGTCTTCCGGAATCAAGATCAACATATCAGGTGGTCATT | 3.405667 | 0.000631 | yes | yes |
| 7 | 334 | CCCCACCCCTATCGTGGTTATTGTGIIIIIGGACTGAATTTACTTG | 4.583428 | 2.21 E-07 | yes | yes |
| 8 | 297 | CCGGATGGTGCTTCTAATTTTCTGCTAACCTGTACTGTGGTGTGTGTA | 3.80189 | 0.000379 | yes | yes |
| 9 | 291 | CCTCCGAGCTGCTAGCTGACAAATACAATTCTGAAGGAATCCAAA | 3.710157 | 0.00019 | yes | yes |
| 10 | 287 | GCCTGTATCCCGGTGGGAGTACTATGAGTCAGTGTACACAGAACG | -3.6926 | 0.000507 | yes | yes |
| 11 | 267 | AAACCATCAGCCGGCCTTTTATATGGGTCTTCACTCTGACTAGAATT | 3.57085 | 0.0014 | yes | |
| 12 | 267 | GACTGATGCCAGGACAACCTTTCTCCCAGATGTAAACAGAGAGACATG | -4.19554 | 2.68E-05 | yes | yes |
| 13 | 259 | CACTGGACACCCTTCGAGTGTGGGTTTTAACATCCCTGTGAGATT | 2.634469 | 0.006429 | yes | |
| 14 | 254 | GATCTGGGGATCACGCCTTGCCCAAGTGTGAGATTACCTTTCT | 2.052031 | 0.040119 | yes | |
| 15 | 244 | TTCAGGCTTAATGCTGCACCTAGATATAAATGCTAATGATACTTGGGTT | 5.729346 | 2.33E-07 | yes | yes |
| 16 | 232 | GAGTCAGTGGATGGACAGGTGGTTTCTTCCCACAAGAGAGAAAT | -3.406 | 0.001156 | yes | |
| 17 | 227 | TGGTAGGAGATACTAATTGGATTCGGAGATATTAATTGGATTTGGCCA | -1.61758 | 0.110916 | yes | |
| 18 | 211 | CCTAAGGTGGTTGTGCTCGGAGGGTTTCTTGTTTCTTTTCCATTTT | 1.001552 | 0.315659 | yes | |
| 19 | 194 | GCTCAATATTCCCAGAATAGTTTTCAATGTATTAATGAAGTGATTAATTGGCT | 4.402902 | 4.56E-06 | yes | yes |
| 20 | 189 | TTGCTTTTTCTTCCTTTGGGATGTTGGAAGCTACAGAAATATTTATAAA | 3.113451 | 0.002649 | yes | |
| 21 | 181 | AGGCCTCATCCTCCACTGAAGAGTATGGATTGAAGGATTGTGAAC | 4.029959 | 5.79E-05 | yes | yes |
| 22 | 181 | GCTGAAGGACCCTGAGGAGCTTCGCAACTACATGGAGAGGATC | 2.98415 | 0.004216 | yes | |
| 23 | 178 | TGTTTCAAAACCACTTGCCATCCTGTTAGATTGCCAGTTCCTGG | 1.89886 | 0.057651 | yes | |
| 24 | 164 | ATCAAGCAAGTTCCTGCTGCTGAAGGATAAGACACAGATGACCTG | -0.84556 | 0.373466 | yes | |
| 25 | 142 | TAAGTCGGGTGGCAATTGTCAGGGTGTGGGAATTTCTTTTCCTAC | 3.39272 | 0.000659 | yes | yes |
| 26 | 140 | AGTTCTGACCCAACCACAGAGGATGCTGACATCATTTGTATTATGTTC | -2.26722 | 0.020347 | yes | |
| 27 | 135 | CCATGGCAGTGGGAAAAATGTAGGAGACTGTTTGGAAATTGATTTT | -1.91339 | 0.060183 | yes | |
| 28 | 118 | AGGACCTGAAGGGTGACATCCAGGAGGGGCCTCTGAAATTTC | 1.467298 | 0.152499 | yes | |
| 29 | 117 | GGACTCATCTTTCCCTCCTTGGTGATTCCGCAGTGAGAGAGT | 2.349832 | 0.026905 | yes | |
| 30 | 117 | CTCCCTGAAAAACCATTCCTGCTGAAACTGCTGTAGAAATTGTGAAG | 3.856822 | 3.49E-05 | yes | yes |
| 31 | 114 | CCCCTGAAAAGTGAGCAGCAACGTAAAAACGTATGTGAAGCCTCT | 2.97408 | 0.002585 | | |
| 32 | 109 | CACCTGCTCTAGGGACGATTCGTTTGAAAGAGAGTAAGATGCATTAA | -3.92943 | 0.000275 | yes | yes |
| 33 | 103 | CCACCTGTTCTCAATTTGCAAGAATTAGAGGCGTATAGAGACAAATTG | 1.583424 | 0.10573 | yes | |
| 34 | 102 | TGTTTGGTCGTAATGTCTGCATGATATTTGTGCACATTTATTAAGTATCG | -3.38591 | 0.000419 | yes | yes |
| 35 | 102 | GGCTGGGTGTTTTCAAATGTCAGCTTAAAATTGGTAATTGAATGGAA | -2.34381 | 0.021355 | yes | |
| 36 | 99 | GCAAGCATAAGGGAAAATGTCACGTAAACTAGATCAGGGAACAAAATC | 1.603483 | 0.122455 | yes | |
| 37 | 92 | GCCAAGACCACCCAGGAAACCATCGACAAGACTGCTAACCAG | -2.61622 | 0.013024 | yes | |
| 38 | 81 | TTTGCAAAGAATCCAGGACAAAAAGGATTAGATCTAGAAATGGCCATT | -0.1063 | 0.915443 | | |
| 39 | 77 | GGTGACTTTTGCAATTCAGGGAAGATTTGGGCATATTAAATGAAAGA | -2.49834 | 0.012822 | yes | |
| 40 | 74 | ATTATGCCACCTTGGATGGAGCCAAGGATATCGAAGGCTTGCT | -0.69455 | 0.484731 | | |
| 41 | 71 | TTGATTTGGGACTTGGGAGACCTCTCTTCTGTAAGCAACTCAATAAA | 3.539216 | 0.00045 | yes | yes |
| 42 | 67 | AATCCTGTGGTGAATGGTGGTGTACTTTAAAGCTGTCACCATGTTA | 1.171006 | 0.231962 | yes | |
| 43 | 64 | CTTGAGTCCCACCCAAAACCTCTAGTAGGGTTTTAATAACGCTCAC | 3.521289 | 0.000326 | | yes |
| 44 | 64 | TCTTTCAGAAGTGAAGAGGGGGCTAGAAGGACTCTGAGAAGTTGGTA | 3.320872 | 0.001015 | yes | |
| 45 | 60 | GCAGAGAGTGCCGATCTTACTCAAGTACCTAGACTCAGATACAGAGAAGG | 2.934405 | 0.002862 | yes | |
| 46 | 60 | CGGAACTCTTGTGCGTAAGGAAAAGTAAGGAAAACGATTCCTTCTAA | 2.370805 | 0.019885 | yes | |
| 47 | 59 | AATGTGGGAAGGTGGGGGTTATGGAGGAGATAACTCAAAACTTCT | 3.829441 | 0.00022 | yes | yes |
| 48 | 59 | CCCCATCTTGTGGTAACTTGCTGCTTCTGCACTTCATATCCATATTT | 1.175431 | 0.263596 | yes | |
| 49 | 54 | TTATCGATTCGTTGTGGACCCACAGATTGCATCT^{T}TAAATTCATAAT | 1.210782 | 0.239402 | | |
| 50 | 52 | AGGTCCTCAGATGGGAATTGCACAGTAGGATGTGGAACCTGTTT | -1.45819 | 0.141876 | yes | |
| 51 | 51 | TGTAACATTCCTGAAGCTGTTCCCACTCCCAGATGGTTTTATCAATA | 1.852187 | 0.06511 | yes | |
| 52 | 48 | TTTTAACTTCTATATGGGACCCGAATTAGACACTGCTGAATCCTGTAC | 3.449622 | 0.000296 | yes | yes |
| 53 | 46 | AGAAGAGCACAAAGCAAGGCCATTGCAACAGGCATTTAAAAATTATT | -4.08976 | 7.22E-05 | | yes |
| 54 | 46 | CTCAATGCATCCATCTTGGGCTGATCATGCCACAGATCTCATTC | 3.285094 | 0.001303 | yes | |
| 55 | 45 | AATTCCTCGGGAAAGGTGAACCTGAACAACCCAAGTCTCTCTCT | -4.17274 | 3.78E-05 | | yes |
| 56 | 45 | GCTCTGTTACAGCTCTGACCACGAAAAACTGAAGCCTCAGTACTTG | -3.86847 | 0.000157 | yes | yes |
| 57 | 44 | CCACTTGACAGTGGAGCAGAGGGGTTACCCAGATTTCAACCTCAT | 1.389785 | 0.187738 | | |
| 58 | 44 | TTCTGGGATTTCTCTAGAGGCTGGCAAGAACCAGTTGTTTTGTCTTG | 0.716713 | 0.475575 | | |
| 59 | 42 | TGAGCACCTTTTAAACCTGCTGCACAATAATTGAGGAAATAGACTCTTT | 1.253049 | 0.204046 | | |
| 60 | 42 | CGTGTCAACAATGGTAAAGGGGATGTATGGCATTGAGAATGAAGTC | -4.22412 | 0.000197 | yes | yes |
| 61 | 41 | TCAAAGTTCCCCAAGAAGAACTGGAAAATGCCAGCCTAGTGTTTAC | -1.17228 | 0.26041 | yes | |
| 62 | 37 | ATGTTCACCTGGCAATCAGCTGAGTTGAGACTTTGGAATAAGACACT | 3.369271 | 0.000783 | | yes |
| 63 | 37 | TTTAATTATGGTGAGGGTTTGCGTTTGGGTACAAGGAATATGAGAGAT | -1.57416 | 0.125115 | | |
| 64 | 34 | TGAGAGCATGCCAAAATTTGCTAAGTCTTACAAAGATCAAGGGCT | 3.791155 | 0.000213 | yes | yes |
| 65 | 33 | TCCCTACCAAGTGAAAATTGATGTGTGTTAAGAGGGTACAGAATTATCAAC | 2.285228 | 0.014636 | | |
| 66 | 33 | GGTGATTTGCTGCTGGCTTTCTATCATTTTTATGTTTTAATGCAAAG | -3.31128 | 0.001117 | yes | |
| 67 | 33 | GGCCAAGAATATTGCAAAATACATGAAGCTTCATGCACTTAAAGAAGTA | -0.5838 | 0.561233 | | |
| 68 | 32 | CTCCTCAGGACCCTCTGGGTCACACATCTTTAGGGTCAGTGAAC | -3.24137 | 0.000608 | yes | yes |
| 69 | 32 | GGCAACAGGAAACAGGTTTTGCAAGTTCAAGGTTCACTCCCTATAT | 3.728802 | 4.82E-05 | | yes |
| 70 | 30 | TGAAAAAGTTATCTCTGGGTATTGCATAAAAGGCTTCATCTTATAAAGTGA | -1.39464 | 0.192516 | | |
| 71 | 29 | CCACGAGGATGGCCCACAAGCAGATCTACTACTCGGACAAGT | 0.902775 | 0.379221 | | |
| 72 | 28 | TCTGCTCTCCATCCAGAGCCTTCTAGGAGAACCGAACATTGATAGT | 4.556078 | 1.33E-05 | | yes |
| 73 | 27 | GTGAGGAGCGAAGAGCCCTCTGCTCTAGGATTTGGGTTGAAAAA | 1.185538 | 0.24293 | | |
| 74 | 27 | TGGCACTTTGTTTGTGTTGTTGGAAAAAGTCACATTGCCATTAAAC | 0.806045 | 0.423301 | yes | |
| 75 | 26 | GGCTGGATCAAGGGCAAAAACTGGTCATTAAGTCATCTGACATTAA | 2.219451 | 0.029617 | | |
| 76 | 26 | GAGAGAAATTTTAGGTGGTTGAAATGATTAAATGGAAAGAGATTTATTTTCA | -2.55709 | 0.010361 | | |
| 77 | 26 | TCTTTGAGAAACAGCGTGGATTTTACTTATCTGTGTATTCACAGAGCTT | 1.067981 | 0.294969 | yes | |
| 78 | 25 | ATAGAGCACCCAGCCCCAGCCCTGTAAATGGAATTTACCAGATG | 1.605946 | 0.10608 | yes | |
| 79 | 24 | TCACAGGATCCTGAGCTGCACTTACCTGTGAGAGTCTTCAAACTTT | 0.054807 | 0.956306 | | |
| 80 | 24 | GCTGAAGTGTTCATAAGATAACAATAGGCTTGAATCTCCAATTCAAATGAAT | 0.213151 | 0.831225 | | |
| 81 | 24 | TTGTGACATTGTGACAAGCTCCATGTCCTTTAAAATCAGTCACTCTG | -5.23137 | 3.27E-07 | yes | yes |
| 82 | 24 | TGGAAACTCTTGGACCAAGATTAGGATTAATTTGTTTTTGAAGTTTTTTG | 0.949064 | 0.344716 | | |
| 83 | 22 | CATTCAATCCGGACCATTTTCTGGAGAATGGACAGTTTAAGAAAAGG | 0.784157 | 0.439473 | | |
| 84 | 22 | TGCTGAACTCACAGTTAGACAATCCATGGTTTAATGCACATGAAATTACC | -0.14282 | 0.887169 | | |
| 85 | 22 | ACGATAACCTGGCAGTGGAAGGAAAGAAGCATGGTCTACTTTAGGT | -2.47268 | 0.010003 | | |
| 86 | 21 | CTGCTTGATTTTTGCCTCTTCCAGTCTTCCTGACACTTTAATTACCA | -4.14257 | 2.03E-05 | | yes |
| 87 | 21 | TCAACATCACAATGGCAAAGAAGAAATATACTGTACAAAACTGCAGGAA | -1.72036 | 0.093687 | | |
| 88 | 21 | TTTGCATGTCCAAATTGCTTCCTTCTTTTTAGCAGAAAGGAGGAGT | -1.91298 | 0.083553 | yes | |
| 89 | 21 | TCCAGGCATTTTAGAACTATGCAATTGTGATTTAAAATGCAACTTTGT | -2.15046 | 0.037021 | | |
| 90 | 21 | GAGACAGGGAAACACAAGGGGAGTAGAAGGCTTCAGTAGAAGATTTC | 1.665338 | 0.12772 | | |
| 91 | 20 | GTGGTCATCATCAAGGCATGCCAGGATACCTTCCTGGTGCTAT | -1.18886 | 0.2414 | | |
| 92 | 20 | CCTGGTAAGTATGCAGCACATTGCTTATATCCTGGGTATGCATTATTTT | 3.422692 | 0.000874 | | yes |
| 93 | 20 | TCAATGAGTAACAGGAAAATTTTAAAAATACAGATAGATATATGCTCTGCATG | 3.192274 | 0.001222 | | |
| 94 | 20 | TGCCCAGTTTGTTCAAGAAGCCACTTACAAGGAAGTAAGCAAAATG | -0.54687 | 0.573053 | | |
| 95 | 19 | GAAACGGGGCCATATAGTTTGGTTATGACATCAATATTTTACCTAGGTG | 4.220137 | 3.53E-05 | | yes |
| 96 | 18 | ATTTCCATGCCGTCTACAGGGATGACCTGAAGAAATTGCTAGAGAC | -1.16115 | 0.230302 | | |
| 97 | 18 | TGCATAATTCATTGTTGCCAAGGAATAAAGTGAAGAAACAGCACCTT | -1.41926 | 0.138831 | | |
| 98 | 18 | CTGCTCTTGTGCCCTTCTGAGCCCACAATAAAGGCTGAGCTCTTA | 2.079621 | 0.016021 | | |
| 99 | 18 | CCTCCGGAAGCTGTCGACTTCATGACAAGCATTTTGTGAACTAG | 1.81169 | 0.070381 | yes | |
| 100 | 18 | CATAGCTCTTTGGGTCGTGAACCTAATTGTAAACTTTCAGGTATTTTTG | 1.766145 | 0.074049 | | |
| 101 | 17 | CCAGTTATGCAGCACCTGGCTAAGAATGTAGTCATGGTAAATCAAGG | 3.991931 | 0.000108 | | yes |
| 102 | 17 | GGCAGGCACTTTAATACCAAACTGTAACATGTGTCAACTGTATACAACTCA | 1.692111 | 0.097387 | yes | |
| 103 | 16 | GCCACACTGAAAAGGAAAATGGGAATTTATAACCCAGTGAGTTCAGC | 0.852486 | 0.40553 | | |
| 104 | 16 | CTGGGCATACAACCCTCTGCTTTCACATCTCTGAGCTATATCCTCA | -3.7346 | 0.000251 | | yes |
| 105 | 16 | AGCAGACAAAAAAGGCACTTTTCAGAACATCAAATTCCTAATGAAGAAG | -3.23615 | 0.002278 | yes | |
| 106 | 15 | TGCTTCATTGTGCCCTTTTTCTTATTGGTTTAGAACTCTTGATTTTG | 3.311718 | 0.001031 | | |
| 107 | 15 | GATGTGGCAGAATCCACACCAGCTTATCAACCAACACAGCTAATTT | 1.389693 | 0.166098 | | |
| 108 | 15 | GGGGGAGTAAAAAATTGAATTTTAACAAAAGATCTTAGGGGAATGTGATT | -3.0524 | 0.002691 | | |
| 109 | 14 | CCTTGATGCTGTCTGTACAGGGTTCATATTTTGTAGCGAAAGTCGTTT | -2.22662 | 0.020629 | | |
| 110 | 14 | CATCCAGGACACTGGGAGCACATAGAGATTCACCCATGTTTGTT | 1.067035 | 0.287699 | | |
| 111 | 14 | CGGAAGAACTGAGCACTCTGTTCTCCAAACCTATCAGAAATTTGTGG | 3.742475 | 0.000285 | | yes |
| 112 | 13 | ACCAGTAGGGGCTTATAATAAAGGACTGTAATCTTATTTAGGAAGTTGACTT | -4.16547 | 2.91E-05 | | yes |
| 113 | 13 | CACTTTCAGATAAGAGGTGTTTGCTGGGATGGAAGAACTACCTGGC | 1.85552 | 0.076369 | | |
| 114 | 13 | GAAAGCCTTCCTCGGGTTCAAAGCTGGATTTTGAACTGAAGAAGAT | 3.344591 | 0.000724 | | yes |
| 115 | 12 | TGAAAATTGGTAGATCAGAGTTGAGCTGATTGGAGGACCAAATTAAAA | -2.61178 | 0.007099 | | |
| 116 | 12 | TTCCATTGTAATTGCTATCGCCATCACAGCTGAACTTGTTGAGAT | 3.126618 | 0.001574 | | |
| 117 | 12 | GACACAGATGACTCTTTGGTGTTGGTCTTTTTGTCTGCAGTGAATGTT | 1.891374 | 0.056055 | | |
| 118 | 11 | TCAAGAAAGGGATTCCGAGGCCAATAAGCCCTCCTTTCTCTTG | -1.24114 | 0.198279 | | |
| 119 | 10 | TCCAGATAACTTTCAGGCACTGCTGGAGTGTCGGATAAATTCTGGT | 1.813774 | 0.066896 | | |
| 120 | 10 | AATCCTCACATCGTGCCAAACTTAGTCTGGTTACTAAGCCTAAAAACA | 2.403548 | 0.016851 | | |
| 121 | 10 | ATTGAGGATTTGTGGGCAGCCAGAGGGAGTCTGACTGAAGTTTAC | -2.29581 | 0.02535 | | |
| 122 | 9 | TGCCAGAATCTAGTGGGATGGAAGTTTTTGCTACATGTTATCCACC | -0.34425 | 0.731248 | | |
| 123 | 9 | TTCCTTCATGTAACTTCCCTGAAAAATCTAAGTGTTTCATAAATTTGAGAG | 2.695661 | 0.00798 | | |
| 124 | 9 | CTCTGGTGTTTTCGCCAGACAATAAACTTACACTGGAAGCTTTGAT | -3.03684 | 0.002403 | | |
| 125 | 9 | TTGAACACAGGCTTTGTCTGAATGATGTTGTTTTATCTCTTGAACACAA | -3.59572 | 0.000682 | | yes |
| 126 | 9 | GAAGCCAAAGTACCCGCACTGCGAGGAGAAGATGGTTATCATCAC | 1.937332 | 0.052778 | | |
| 127 | 9 | AGCTGGCGCCAGCTTCTTCTCCTGGATCCAGTAAGAGTTTCG | -0.59083 | 0.544181 | | |
| 128 | 9 | CCTGAAGGAAACCACTGGCTTGATATTTCTGTGACTCGTGTTGC | -0.58376 | 0.559447 | | |
| 129 | 8 | CCACCTTTCCTCCCAGCAAGCATCTGGCCAATCCTATTCTTC | -2.29915 | 0.014561 | | |
| 130 | 8 | GTGAGGTACAGGCGGAAGTTGGAATCAGGTTTTAGGATTCTGTCTC | 4.941946 | 1.60E-05 | | yes |
| 131 | 8 | CTTGGCCTGAAGAGGTGCAGAAAATACAGACCAAAGTTGACCAG | -1.962 | 0.049061 | | |
| 132 | 8 | ACATAGTGACATGCACACGGGAAAGCCTTAAAAATATCCTTGATGTAC | -3.61201 | 0.000318 | | yes |
| 133 | 8 | TAATGCAAGCCCTGACTGGGTGGAAGCTGAAGTCTTGCTGTTTTA | 2.396608 | 0.012653 | | |
| 134 | 8 | TCCTTTTTTGGGGAAATCTGAGGCTAGGTCAGAAAAACATAAAGCAC | -3.97531 | 0.00059 | | yes |
| 135 | 7 | GGCCCTTCCTGATGATCATTGTCCCTACAGACACCCAGAACATCTT | -2.36278 | 0.018244 | | |
| 136 | 7 | GGAGCTGGGGAGCTGTGTTAAGTCAAAGTAGAAACCCTCCAGTGTT | -4.42673 | 3.66E-06 | | yes |
| 137 | 7 | GCAGAAAAGAAGACGAGAATGCAACCATACCTAGATGGACTTTTCCAC | 2.728168 | 0.006056 | | |
| 138 | 7 | AACAAGTGGGATTTTCTGGGCCAGCAAGTCTTCCAAACTGTATATG | -0.21615 | 0.829698 | | |
| 139 | 7 | GCTGTGTGGGTCACACAAGGTCTACATTACAAAAGACAGAATTCAGG | -2.47087 | 0.013094 | | |
| 140 | 7 | TGCACAGATCTGCTTGATCAATTCCCTTGAATAGGGAAGTAACATTTG | 3.051955 | 0.001684 | | |
| 141 | 7 | CCCTATGAGTGGAAGGGTCCATTTTGAAGTCAGTGGAGTAAGCTTTA | -1.23786 | 0.21761 | | |
| 142 | 7 | AGAGCTTCTGAGGCGCTGCTTTGTCAAAAGGAAGTCTCTAGGTTC | 1.688918 | 0.097307 | | |
| 143 | 6 | TGTGAAAACAAGCTTCAAAGCCATATGGACACTGTGACAATGACTA | 3.369288 | 0.000926 | | yes |
| 144 | 6 | GCATCTCCCTGACCTTCTCCAGGGACAGAAGCAGGAGTAAGTTTC | -4.1766 | 1.77E-05 | | yes |
| 145 | 6 | CGCCAGCTACAATGCCATGGTGCTCATTCAAAAGACCGACAC | -1.32939 | 0.195817 | | |
| 146 | 6 | GATCCGGGATGGGAGACCCCACTTIAGAAAGGGTCGTCACTC | 2.803856 | 0.002763 | | |
| 147 | 6 | TGGATTGAGAAAACCTATATCCATTCTTTATATCAATGTATAGTTTTAGTCTCCT | 2.448797 | 0.019091 | | |
| 148 | 6 | GGTGAATGCCCTCAACTTCTCAGTGAATTACAGTGAAGACTTTGTTGA | 1.886404 | 0.063745 | | |
| 149 | 6 | ATACGGCGAGGTAGAGTTGGCCATATTTCAGAGACTTAGATTGACGT | 4.337458 | 1.70E-05 | | yes |
| 150 | 6 | GCTCGTTTGGTGCACTCTCGTGGGAGACAATCAGAGAACAACATA | 1.225008 | 0.19426 | | |
| 151 | 6 | GCCTTTCCATCTGGCATTTCCCGCTCATTTATATGACTTGCTGAG | 2.968053 | 0.002255 | | |
| 152 | 6 | TCCGGTGACCAGGTGTCTACAAGGACAAGGTTCAGAAATTGTACAG | 2.304752 | 0.025941 | | |
| 153 | 5 | GCAGAGGTTCTTTTAAAGGGGCAGAAAAACTCTGGGAAATAAGAGAG | -3.24369 | 0.000993 | | yes |
| 154 | 5 | TTTTCCCCACCCGAGATGAAGGATACGCTGTATTTTTTGCCTAAT | 2.24033 | 0.011993 | | |
| 155 | 5 | ACTGCAGGATACACTCCCCTCCTGCTACCTAGGCAGGCGTGAG | -0.88751 | 0.353721 | | |
| 156 | 5 | GGTTTAACCCGAGTCACCCAGCTGGTCTCATACATAGACAGCACTT | 2.232118 | 0.02553 | | |
| 157 | 5 | GACACTTGTTTAGACGATTGGCCATTCTAAAGTTGGTGAGTTTGTCAA | 1.647226 | 0.102927 | | |
| 158 | 5 | TCACCAAAAGCTGTATGACTGGATGTTCTGGTTACCTGGTTTACAAAAT | 2.938772 | 0.002383 | | |
| 159 | 5 | CATAAGCTGGTATCAGTGGTTCGGGGGAAATAGTTCCATTCTATGACTC | -3.01637 | 0.003841 | | |
| 160 | 5 | TTGGCGATCATTTCCCAAGATTGGTTTCCCTTGAGTTTTTGTTAAA | -4.22349 | 5.18E-05 | | yes |
| 161 | 5 | CCTAGTTTGATGCCCTTATGTCCCGGAGGGGTTCACAAAGTGCT | -0.55135 | 0.58089 | | |
| 162 | 5 | TTTTCGAAGGATAATTTTGGAGGCNAGAAAAAATGGACGGGG | -2.37921 | 0.016098 | | |
| 163 | 5 | TGTTCGCGACTAGTTGGCTCTGAGATACTAATAGGTGTGTGAGGCTC | -2.17442 | 0.018122 | | |
| 164 | 5 | CACCTGGACCCCTGCATTGGAACTGGAGGCAGGGAACAT | -2.2306 | 0.022824 | | |
| 165 | 5 | TGGAAGGATGGAAGAAACGCCTGGAGAATATTTGGGATGAGACAC | -1.79624 | 0.085727 | | |
| 166 | 5 | GCATTACTTTGAATTTAATGTTGCGCTTGTGCACTGTGTTAATATTGTTT | -2.46291 | 0.016207 | | |
| 167 | 5 | GGTGATGGGGACCGTCTTTCTTTTACTGACACATGACCAATCATA | -2.30099 | 0.020722 | | |
| 168 | 5 | TGGGCAAAACCATTGAATTTCACATGGGTGGTAATATGGAGTTAAA | -2.99545 | 0.002532 | | |
| 169 | 5 | AACCAGCCTTCAGAGCGTTCTCTGTCCTGCTTCTAACGTCACTT | 0.01601 | 0.987237 | | |
| 170 | 5 | TGCCACATTTGACTGAATTGAGCTGTCATTTGTACATTTAAAGCAGC | -0.67643 | 0.497085 | | |
| 171 | 4 | TATATGGTTTCCAAAGGGTGCCCCTATGATCCATTGTCCCCACT | -1.76935 | 0.075774 | | |
| 172 | 4 | TTTAAGGACTGATCATTGGCTCTGAGGACACTTCAACTAGTTAGCCTTCT | 0.11679 | 0.907115 | | |
| 173 | 4 | CCCTCATCAAAGTCCTCGGTGTTTTTTAAATTATCAGAACTGCCC | -2.8669 | 0.003674 | | |
| 174 | 4 | CAAATGGTTACCTTGTTATTTAACCCATTTGTCTCTACTTTTCCCTGTACTT | 3.411476 | 0.000689 | | yes |
| 175 | 4 | GCCAGCTGCATGCAGGAGCGTGCCATCCAGACAGACTTC | -2.24724 | 0.024396 | | |
| 176 | 4 | CACTCCAGGCAGGTCTTGGGGCTCCTATGTAAGCTGTGTTAAGC | -1.38308 | 0.168369 | | |
| 177 | 4 | GGCTGGCAACTTAGAGGTGGGGAGCAGAGAATTCTCTTATCCAAC | -2.0969 | 0.052047 | | |
| 178 | 4 | GATTCATCCAGCCTTCCAGCTCTGTTATTTAAAGCAAGAACACTTCTG | 4.423002 | 2.51E-05 | | yes |
| 179 | 4 | CCTAAAGCAAGCCTGAATTGGCTATGCAGTACATTGTATTCTGTTTG | -4.28624 | 2.42E-05 | | yes |
| 180 | 4 | CGGGCTTTTAGCAGCATGTACCCAAAGTGTTCTGATTCCTTCAACT | -0.69941 | 0.485384 | | |
| 181 | 4 | CTAAGGGATGGGGCAGTCTCTGCCCAAACATAAAGAGAACTCTGG | 0.451498 | 0.654537 | | |
| 182 | 4 | AAATCCAAACTCTCAATTACGCCATGGTAATTCAGTCACTAAAATATGT | -3.42088 | 0.000689 | | yes |
| 183 | 4 | GCAGGAACCGCGAGATGGTCTAGAGTCAGCTTACATCCCTGA | -2.37983 | 0.026005 | | |
| 184 | 4 | TGTTCCACTGAGCTCCTGTTGCTTACCATCAAGTCAACAGTTATCA | 0.232032 | 0.818608 | | |
| 185 | 4 | CTCAATGTAACCTCAGGGGCCAGTTTTAGCATTTGAAATGGTTCT | -1.76115 | 0.095531 | | |
| 186 | 4 | TCTGACCAGTTACAGCCCCAAAGATGCAGTGATAACTGTGATGTATG | -1.37322 | 0.133757 | | |
| 187 | 4 | GCATCCAGAACAGCCTGCTTGGACACAGCTCGGTGGAAGAT | -2.12389 | 0.039832 | | |
| 188 | 3 | TTACAAATGACTCAGCCCACGTGCCACTCAATACAAATGTTCTGCTAT | 2.395553 | 0.016443 | | |
| 189 | 3 | ACAGCCCTGCTCCCAAGTACAAATAGAGTGACCCGTAAAATCTAGG | -3.50224 | 0.000637 | | yes |
| 190 | 3 | CCAGATACTACTCGGCGCTGCGACACTACATCAACCTCATCACC | -2.27536 | 0.031464 | | |
| 191 | 3 | AATAAGCAGGATGTTGGCCACCAGGTGCCTTTCAAATTTAGAAA | -1.68039 | 0.069608 | | |
| 192 | 3 | GGAATTTGATTCTTCCAGAATGACCTTCTTATTTATGTAACTGGCTTTCA | 3.817877 | 0.000172 | | yes |
| 193 | 3 | CAAGGTGTAGCAAGTGTACCCACACAGATAGCATTCAACAAAAGCTG | -2.7616 | 0.00587 | | |
| 194 | 3 | CACAGAGTCTGAAAAGCGGGTCTCCGTCTACCAGAAGGTGACCTCC | -0.46636 | 0.642653 | | |
| 195 | 3 | TGAGGACTCAGAAGTTCAAGCTAAATATTGTTTACATTTTCTGGTACTCTG | 2.004388 | 0.050059 | | |
| 196 | 3 | TGGAATGGTGAAAGAGAGATGCCGTGTTTTGAAAGTAAGATGATGAAA | 1.286332 | 0.218274 | | |
| 197 | 3 | CAGGGGTTGAGAGCTTTCTGCCTTAGCCTACCATGTGAAACTCTA | 0.396152 | 0.693055 | | |
| 198 | 3 | CACAGATGAGAACCACGCCTAGCCAAAATCACTTTTCCTGTTTGC | 0.932001 | 0.350214 | | |
| 199 | 3 | AGGCATGGGAGTCATTGTCCACATCATCGAGAAGGACAAAATC | 1.048222 | 0.289167 | | |
| 200 | 3 | AACGGGATCCTCTGTGGTCGCGACACTGACCTAGACGGCTT | 1.930889 | 0.05573 | | |
| 201 | 3 | TGTGTGGCTTCCTGCAAGGTACCTTCATCTCTGAGTTACCTGACTC | -2.12276 | 0.038612 | | |
| 202 | 3 | GCTGTCCTCAAAGCATCCAGTGAACACTGGAAGAGGCTTCTAGAA | 2.647942 | 0.012248 | | |
| 203 | 3 | CCCTTTCTTTGATGGTGCTTGCAGGTTTTCTAGGTAGAAATTATTTCA | 1.507454 | 0.122396 | | |
| 204 | 3 | AAATGTACCAATCAGCATGCTGTGTCTAGCTCAAGAACTCAAGCTCC | -0.57666 | 0.565438 | | |
| 205 | 3 | CGTCACCCGAAAAAGTTCCCTCCATATCCCTTTGCAGTCAGTTC | 0.760999 | 0.450353 | | |
| 206 | 3 | CATGACCGCATGGTATACCTGGGCCTCTCAGACTACTTCTTCAACA | -2.48108 | 0.008418 | | |
| 207 | 3 | GCATTAGTATGACAGTAGGGGGGCTGTTAGAATTGCTGCTATACTGGT | -1.3436 | 0.203689 | | |
| 208 | 3 | CCAGGATAAAGCTTCCGGGAAAACAGCTATTATATCAGCTTTTCTGA | 2.988968 | 0.005851 | | |
| 209 | 3 | TTCCTGCTACACATGCCCTGAATGAATTGCTAAATTTCAAAGGAAAT | 0.810494 | 0.410805 | | |
| 210 | 2 | TGGCCTGTGGTTATCTTGGAAATTGGTGATTTATGCTAGAAAGCTTT | -2.73832 | 0.007474 | | |
| 211 | 2 | GGGAGAAAGCTAATGTTTTCCACAAGACTGAACAACGTGTATTTACACG | -3.19477 | 0.000546 | | yes |
| 212 | 2 | GTGCTGGGTGCATATCATCCAGGATAATATTCTGCCCAACTCCAT | -1.23963 | 0.215301 | | |
| 213 | 2 | CCCAGGATTATGTTTGTTGACCCATCTCTGACAGTTAGAGCCGATAT | -1.35681 | 0.189959 | | |
| 214 | 2 | GCTGGACAGGAGCACTTTATCTGAAGACAAACTCATTTAATCATCTTTG | 1.270148 | 0.203802 | | |
| 215 | 2 | AGTTGCAAAAATGGCTCCATCGGTAACTCAAGCTTCAGAATGTTATG | 2.416151 | 0.015977 | | |
| 216 | 2 | TCATCGGAGTAGATTCCGGGTGCCTTTACTCCACTGTGACCTCATA | -1.28409 | 0.192946 | | |
| 217 | 2 | GTGTGCTCAGGCAATTATTTTGCTAAGAATGTGAATTCAAGTGCAG | 2.642687 | 0.006795 | | |
| 218 | 2 | TGGGAAAGTATCAGGAGTGCCATGATTCCAATGTTTTCCTTCTTTTA | 2.157816 | 0.025861 | | |
| 219 | 2 | AGACGGCGCAGACATGTCAGAACAAAGTAAGGATCTGAGCGAC | -3.49114 | 0.000292 | | yes |
| 220 | 2 | CCAGCATTAAGTACTGTATATCGCCCTGTACTTGGATAGGCTGGCTAAC | 0.144643 | 0.885072 | | |
| 221 | 2 | AGGCAAGGAGGAGGGGAATTTTAAAACCATCTTATTTGAAGTGAGAG | 1.851673 | 0.064779 | | |
| 222 | 2 | TGGTGCTCTATGCTCAATGATGGTCTTACACATTCCTCTAGGGAAAG | 3.451224 | 0.001721 | | |
| 223 | 2 | CCCTTTCTATTCTGAACAACTGTCTCCATTTTTCAAGTGTGAGAGATAAGG | 2.35354 | 0.015021 | | |
| 224 | 2 | CAGTCGAGACCCAGATCCACTGAACATCTGTGTCTTTATTTTGCTG | 1.792116 | 0.072731 | | |
| 225 | 2 | GCAGACATCCTGTGAAGCAGGACCTGCTGAAGAGGAGACTTTCTAT | 0.353515 | 0.72344 | | |
| 226 | 2 | TGAGAAAGCTCAAGATTCCAAGGCCTATTCAAAAATCACTGAAGGAAA | 2.674193 | 0.01623 | | |
| 227 | 2 | CAATCCGAGTTCCCGGATGAGGGAACATTCTGCAGTATAAAGGG | -1.59105 | 0.109934 | | |
| 228 | 2 | CAGCACCAAGTCTACGGGTGCCAGATCAGTAGGGCCTGTGATT | 2.448186 | 0.011472 | | |
| 229 | 2 | AGCAACAGCAAATCACGACCACTGATAGATGTCTATTCTTG7rGGA | 1.239036 | 0.208803 | | |
| 230 | 2 | CTTGCCCATCTAGCACTTTGGAAATCAGTATTTAAATGCCAAATAATC | 1.984819 | 0.047707 | | |
| 231 | 2 | TTTTCAGGTTTATTCTTTTAGCAGGTGTAGTTAAACGACCTCCACTGAAC | 0.755574 | 0.449369 | | |
| 232 | 2 | TGCCAAAAATTAAAGTGCAATATTGTATATTTTTAAGAACAAATTTAAAATAGAA | -0.31259 | 0.755488 | | |
| 233 | 2 | TTCTGAGGAGGAGAGAGTGAGGGTTTTGCTATTGACTGACTTGAAC | -0.56126 | 0.582467 | | |
| 234 | 2 | CTGGGGGCGCAACCACCCCTTCCTTAGGTTGATGTGCTT | -2.07743 | 0.039244 | | |
| 235 | 2 | ACGCTGTGCGTTTGTCAGAATGAAGTATACAAGTCAATGTTTTTCCC | -0.98359 | 0.325597 | | |
| 236 | 2 | TGTTGAATACTTGGCCCCATGAGCCATGCCTTTCTGTATAGTACAC | -1.61334 | 0.089292 | | |
| 237 | 2 | GCTATACCTCATTCACAGCTCCTTGTGAGTGTGTGCACAGGAAATAAG | 0.661879 | 0.50136 | | |
| 238 | 2 | ACACTGTTGGACCTGACCCACACTGAATGTAGTCTTTCAGTACGAGA | 1.423365 | 0.138012 | | |
| 239 | 1 | TCCTGCAAGTAAGAATGTTTTCACACTGAGCTATTGATTTAACCAAGC | 0.61193 | 0.55105 | | |
| 240 | 1 | GGGAGGTCAGACACGCTTCATTATATCTCCGTCTCTTTTATGGTTT | 0.118933 | 0.905287 | | |
| 241 | 1 | TGGTTGTGCTTGCTTTCCTTTTTTAGAAAGTTCTAGAAAATAGGAAAACG | -0.29803 | 0.763403 | | |
| 242 | 1 | GCTCCGGCAGCACCTTTATCTATGGTTATGTGGATGCAGCATATAAG | 0.36714 | 0.713965 | | |
| 243 | 1 | AACCTCCCAGAGTTAGCCAGCCTTTCAGAGTTGAAGTCACAGCT | 3.427416 | 0.000767 | | yes |
| 244 | 1 | ATGTGCACCTTTGAGGCTACGGGCTTCTCCAAAGACTTAGGAATCT | -2.69533 | 0.003868 | | |
| 245 | 1 | ATGTGGCCATTACCGTCATTGGCCTGTGAAGCATTGGACATTTATA | 1.572459 | 0.10227 | | |
| 246 | 1 | GGCCTGTGAAAACAGAGGCTTTTGCATTGTCTCTTGACATCAGAAGT | -1.55078 | 0.126022 | | |
| 247 | 1 | AGCCAAGGCAGGGTGGACAGTGTGAGAGAGCTAGTGTAAGCTGT | -4.58277 | 3.08E-05 | | yes |
| 248 | 1 | ACATGGTTGTGCAGGGCCATGTGTGAAGACAGCATGAGTCTTA | -1.37266 | 0.163501 | | |
| 249 | 1 | TGATGTTGGTTGTAATGGTTGGGTTTAGGATGAACCATTTTAAGGAT | -3.34973 | 0.000692 | | yes |
| 250 | 1 | GTCCCAAAGGTGGAATACAACCAGAGGTCTCATCTCTGAACTTTGTT | -0.7171 | 0.473628 | | |
| 251 | 1 | GGCGTCTACAGAGACCAGCCATATGGCAGATACTGATTGTACTGTCT | 3.48851 | 0.000532 | | yes |
| 252 | 1 | TGTTTGCCTCAAACGCTGTGTTTAAACAACGTTAAACTCTTAGCCT | 0.57799 | 0.564212 | | |
| 253 | 1 | TGCCAAAAATAAACTCACATGAGCACATGACAGTCTGAGCTCTATAATCA | 0.417675 | 0.679323 | | |
| 254 | 1 | GTGCATGGACGACTGAACACAATCAACTGTGAGGAAGGAGATAAACT | -0.49188 | 0.625569 | | |
| 255 | 1 | CCTTGCCACGGTTCTAGAGCAGCGTAGACAGCTGGTAAACTGAAGA | -1.4853 | 0.140016 | | |
| 256 | 1 | CATCGAGAGCGCACACAAGACGCCACTGTAAAAGGATCACAGAT | -2.23853 | 0.021028 | | |
| 257 | 1 | GGAGTTCCAGGAGATTCAACCAGGATGTTTCTACACCTGTGGGTTA | -2.6129 | 0.009463 | | |
| 258 | 1 | CAGCCACCATCAAAGCCCATTCGTAGGAAATTCAGACCAGAAAAC | -2.13238 | 0.039635 | | |
| 259 | 1 | TTTGGAAGGCATTGAAGCTTGCACCTTTTCATGTACAGCATTAAAA | 2.984702 | 0.00337 | | |
| 260 | 1 | GACCAGGTCTATCAGCCCCTCCGAGATCGAGATGATGCTCAGTAC | 0.012992 | 0.989634 | | |
| 261 | 1 | ATCCAACACAGCCAGAACCCGCGATTCTACCACAAGTGACCATC | -2.02092 | 0.048641 | | |
| 262 | 1 | TTGCCTGGAGAAAGAGAAGAAAATATTTTTTAAAAAGCTAGTTTATTTAGC | -0.31184 | 0.755184 | | |
| 263 | 1 | TCACCCCTGACACAACATTTTCAGAATTCCAGACGATACTGTGATAA | 0.561867 | 0.577848 | | |
| 264 | 1 | ACGGAAACAGACCCCTGCTTTCGAATTTACATGTTCATGATGTGC | -3.07043 | 0.001314 | | |
| 265 | 1 | CTTCGCCCCTCCCTTGTTTTATATTTTATGAAGTTAGTGCGGG | -0.71287 | 0.476918 | | |
| 266 | 1 | ACCAACCAATACTCAGGAGAACCCTGCCTATGAGGAATATATAAGACCA | -3.58227 | 0.000386 | | yes |
| 267 | 1 | TGTTTGGAAAATCACATCATGCCTAGAATCTGAAATTGAATTAGCAA | 1.822415 | 0.059401 | | |
| 268 | 1 | GCCAAGAGAATCAGAGAAAGATGCTGCATTTTATAATCAAAGCCCAAAC | 3.532588 | 0.000225 | | yes |
| 269 | 1 | CGTTACAGTATTCTGATTATATTACTGACACAGTCAAAATGATTAACTGTACAA | 2.897694 | 0.007766 | | |
| 270 | 1 | TTGTGCCTGTGTGTTACCATGCTAAGAATGTCTTTGTTTAAAGGGAA | -1.30273 | 0.21043 | | |
| 271 | 1 | GCAACAGCAAGCTGTAGAGCGGGCCAATGATAAATCACATTGAATC | 2.236428 | 0.023294 | | |
| 272 | 1 | AAGCCAAAGGAACTGGAGGCACTGATTTAATGAATTTCCTGAAGA | 0.742825 | 0.459843 | | |
| 273 | 1 | TTTCCTCTTGATCGGGAACTCCTGCTTCTCCTTGCCTCGAAAT | -3.54509 | 0.000263 | | yes |
| 274 | 1 | CCAGGTACAATGGCAGAGCCTTTCCATACCTGTACTCACAACTAGC | -0.50817 | 0.617396 | | |
| 275 | 1 | TGGTCTTCTTGCATCGATGATCCAACAGCAACACCATTTTTAAATTA - ' | 2.61129 | 0.008828 | | |
| 276 | 1 | TGTCCCTTTGATACTTGTGCTCTGCTGAGAATGTACAGTTTGCATTAA | 4.051892 | 5.82E-05 | | yes |
| 277 | 1 | GAGAGGCAGCATTGCACAGTGAAAGAATTCTGGATATCTCAGGAG | 0.118343 | 0.905829 | | |
| 278 | 1 | TTGGATGAGATTAGGAGATCAGAGGCTGGACCTTCTCTTGATAATGC | 3.212168 | 0.003088 | | |
| 279 | 1 | AATTGTTGACATTCATGTCTCTGAGTTACAAAAGTGCTAATTCACTACATGT | 1.92036 | 0.050161 | | |
| 280 | 1 | GGGGAAATTCAAGCAGTGTTTCCTCAACCAGTCACATAGAACTCTG | -0.78853 | 0.427972 | | |
| 281 | 1 | CTGTCCAGCCGCATGGAGGGCATGATGGCTTCCTACAC | 2.265809 | 0.038306 | | |
| 282 | 1 | TGTCAATCTGTCCTCGGCTGCCCTTCTCATTTGTTGATGGGAC | 2.656332 | 0.009243 | | |
| 283 | 1 | GGAGACTTTCACAAGTGGTTTCCATGGAGATAGAATGAAGCATTCTGT | 0.674533 | 0.493893 | | |
| 284 | 1 | AGTAGTTTCTCCAAGTACTTTTGTGCTATCAATGAGTTCTTCTCAAAAAAT | -2.07479 | 0.048611 | | |
| 285 | 1 | TTTCTTTGCTAAGCCTTGCATGCAAAATTTGAAATTTTAACATTGGC | 0.55078 | 0.582445 | | |
| 286 | 1 | ACAAAACGAGTCCAGCGACGAGGAGAGCCTGAGAAAAGAGAGAG | -1.49962 | 0.131713 | | |
| 287 | 1 | AAATGAGGGCCCGTAACAGAACCAGTGTGTGTATAACGAAAACCAT | -1.4828 | 0.140027 | | |
| 288 | 1 | TCACCTCAGTCTCTAATTGGCTGTGAGTCAGTCTTTCATTTACATAGGGT | 1.359493 | 0.181639 | | |
| 289 | 1 | CGTCCAGCCAAGAGCTCTTCATCTGCTACAAGAACATTTGAATCTT | -0.00765 | 0.993894 | | |
| 290 | 1 | GATTGCAATGATGATGTCCAAGGTAAGCTATTAAAAGGCAGGTTACT | 2.290805 | 0.021577 | | |
| 291 | 1 | CTTGCCAGCAGCAATCATTTGGGGAAGAATCTACAGTTGCTGAT | 1.790334 | 0.066752 | | |
| 292 | 1 | TTCCTTTGGGAGAAACCTGTTCATTCCAATCTTCTAATTACAGTGGTT | -2.87514 | 0.002884 | | |
| 293 | 1 | CAGGACATCATGAGCAGGCAGCAGGGAGAGAGCAACCAAGAG | 2.201609 | 0.048986 | | |
| 294 | 1 | ACTCGGATTCTTTTGCATGATGGGGTAAAGCTTAGCAGAGAATCATG | -4.84228 | 2.84 E-06 | | yes |
| 295 | 1 | GACGTGAAGTCTCGGGCAAAGCGTTATGAGAAGCTGGACTTCCT | 1.175155 | 0.244078 | | |
| 296 | 1 | CCTCTGTGTTCACTTCGCCTTGCTCTTGAAAGTGCAGTATTTTTCT | 1.150612 | 0.26183 | | |
| 297 | 1 | TGAGGTGTCATCACTGTTGAAAAGGTAAGCACATTTAGAATTTTGTTCTT | 0.963905 | 0.33534 | | |
| 298 | 1 | CCCTTACCCCTCTCTGGGCCCATGAATTCCTGGCTTGGTTTA | -3.09019 | 0.003466 | | |
| 299 | 1 | GGGAAAACATCCATGCTGGACTCCTGAAGAAGTTAAATGAACTGGA | 0.235534 | 0.813451 | | |
| 300 | 1 | CTCCACCAGAAGGGCACACTTTCATCTAATTTGGGGTATCACTGAG | -1.26117 | 0.216194 | | |
| 301 | 1 | TTCTCTCAAAATACTAAACAGAGGTGGTT7FATTGATAAGATTTTGGCTGT | -0.63027 | 0.53117 | | |
| 302 | 1 | GCATGCTGTTGTACATGATCCTGACAAGAAGAAAATGAAGCTCAAAGT | 2.105255 | 0.032858 | | |
| 303 | 1 | GTTCTCCCCTCTGGCCCCTGGAGAGAAGGGAGCATTCCTA | 0.808842 | 0.416399 | | |
| 304 | 1 | TCTTGGTGGGTCAAGACTTTCTGATAAATCAGTTAGCACCATGCAT | -0.87476 | 0.375354 | | |
| 305 | 1 | CTGGAATAATGGAAAGAAATGGGGGCTTTGGAGAACTAGGATGTTTC | 3.031192 | 0.002176 | | |
| 306 | 1 | GCCCACATGGATAGCACAGTTGTCAGACAAGATTCCTTCAGATTC | -2.85804 | 0.004514 | | |
| 307 | 1 | CGCCTTCCTCTTTTTAAGCTGTTTTATGAAAAAGACCTAGAAGTTCTTG | 3.505339 | 0.000639 | | yes |
| 308 | 1 | CTCCAAGCCGATCACCAAGAGTAAATCAGAAGCAAACCTCATC | -3.8295 | 0.000169 | | yes |
| 309 | 1 | AAAGTTGTGTAAGCGCGTGCGTTCTTCTGGGTTTGGCTAGATAG | -0.09465 | 0.924545 | | |
| 310 | 1 | ATGGACTGCTTTGCTGGATTGGCACTGAGCAACTTTAGGAAATGTC | -1.62044 | 0.109697 | | |
| 311 | 1 | TGATGAAATAACTTGGGGCGTTGAAGAGCTGTTTAATTTTAAATGCC | 1.824359 | 0.047857 | | |
| 312 | 1 | GCTCGCCCCTGTTTTTTGTAGAATCTCTTCATGCTTGACATACCTAC | 1.700431 | 0.04944 | | |
| 313 | 1 | GATAGGTGCCATCGTGGTTGAGACAAGTGCAAAAAATGGTATTAAT | -0.74552 | 0.463916 | | |
| 314 | 1 | TGTCTGTGTCAGACGTACAGCCAGACATGTTCTCTATTGGCATTTTT | -1.1303 | 0.263264 | | |
| 315 | 1 | CAGGCCTGGTGCTCAGTCGTACGAGCTGTACCTCTCAACTTTTG | 0.790296 | 0.40455 | | |
| 316 | 1 | AACTCCTGCGATCAGCTTGTGACTTACAAACCTTGTTTAAAAGCTG | 2.767204 | 0.006542 | | |
| 317 | 1 | TCAGTGAGAGACTCCAGGACTTTGACAAAAGCAAGCATGTCATCTATG | 2.720559 | 0.00547 | | |
| 318 | 1 | AGCCACTTGCCCCAGTTCATAACCCCATTAGTGTCTAAGAAGATTTC | -0.77976 | 0.423543 | | |
| 319 | 1 | CACCAGGGACACATTTCTCTGTCTTTTTTGATCAGTGTCCTATACATC | 0.388028 | 0.695317 | | |
| 320 | 1 | GTCAGCTTGCCCAGGTTCAAACTGGAAGAGAGTTACACTCTCAACTC | -3.24347 | 0.001134 | | |
| 321 | 1 | TGATACCCACCGGGTCTGACATTCCAAGTAACCAGTATGTAACTGG | 2.310635 | 0.02143 | | |
| 322 | 1 | TTGTGCAGATAGTATTTCTGATTGATGTCATCTATCAAGAATTTCAAGAGATT | -0.97212 | 0.33353 | | |
| 323 | 1 | AAGACTGTCAGGAAGGGTCGGAGTCTGTAAAACCAGCATACAGTTT | 2.012969 | 0.039001 | | |
| 324 | 1 | AATGGGCATAAAGCTTCACACTAGTAACAAAAATGGCTTAACTTTATTACA | -0.43582 | 0.664998 | | |
| 325 | 1 | TCACATCAGGGCAAATGAAATATCCATCAACTCCAGCATTTATCATT | 2.686802 | 0.005053 | | |
| 326 | 1 | TGCCTTTTTGCCTTTGGTAACATAACTCTGGGAGTCTTGGTTTAT | -2.86852 | 0.003159 | | |
| 327 | 1 | CCACTGGTCATGCTGTGGAAAATTTAATGAGAAATCTGAATGCACAT | -2.15718 | 0.038994 | | |
| 328 | 1 | CATTCCGCTCAAAGGTCACTGAGACTTTTGCCTCACCTAAAGAGA | 1.771767 | 0.077694 | | |
| 329 | 1 | TGAGGAAATCAAAGTGCTATTACGAAGTTCAAGATCAAAAAGGCTTATAA | 1.823273 | 0.066945 | | |
| 330 | 1 | GCTTTGGGGAGGACAAAACTTGTAAGTACAGTCAAGGACAAGACTTG | 3.022171 | 0.002277 | | |
| 331 | 1 | ACGTTCCAGGGCCCAAAGCCCAGCTCTTTGTTCAGTTGACTTA | 3.641037 | 0.000299 | | yes |
| 332 | 1 | TGGCCAAATTAGATGTGTGCTGAAGACAATCAGTCACTGGGTCTATA | 0.779861 | 0.438386 | | |
| 333 | 1 | TGGCTTGTCATTCTGTACACTGACCTTAGGCATGGAGAAAATTACTTG | 1.77954 | 0.072713 | | |
| 334 | 1 | TCATGAATTTTTTAATCCCATTGCAAACATTATTCCAAGAGTATCCCAG | 0.918826 | 0.36453 | | |
| 335 | 1 | CCCCGTCTCCCTCCCAACTTATACGACCTGATTTCCTTAGGA | -2.18861 | 0.026936 | | |
| 336 | 1 | CCACACAGCCAAGCTGAGACTGTGGCAATGTGTTGAGTCATATACAI'T | 1.12796 | 0.260712 | | |
| 337 | 1 | ATTACCTCAGTCCCCGAGGACAGTTTTGAAGGACTTGTTCAGTTAC | 1.485189 | 0.158865 | | |

### Assay methods

To determine the (increased or decreased) expression levels of genes (expressed sequences) in the practice of the disclosure, any method known in the art may be utilized. In some embodiments, expression based on detection of RNA which hybridizes to the genes (or probe sequences) identified and disclosed herein is used. This is readily performed by any RNA detection or amplification+detection method known or recognized as equivalent in the art such as, but not limited to, reverse transcription-PCR (RT-PCR), real-time PCR, real-time RT-PCR, the methods disclosed in U.S. Patent Application 10/062,857 (filed on October 25, 2001) as well as U.S. Provisional Patent Applications 60/298,847 (filed June 15, 2001) and 60/257,801 (filed December 22, 2000), and methods to detect the presence, or absence, of RNA stabilizing or destabilizing sequences.

Alternatively, expression based on detection of DNA status may be used. Detection of the DNA of an identified gene as methylated or deleted may be used for genes that have decreased expression in correlation with a particular prostate cancer, or post-prostatectomy, outcome. This may be readily performed by PCR based methods known in the art, including, but not limited to, Q-PCR. Conversely, detection of the DNA of an identified gene as amplified may be used for genes that have increased expression in correlation with a particular prostate cancer, or post-prostatectomy, outcome. This may be readily performed by PCR based, fluorescent in situ hybridization (FISH) and chromosome in situ hybridization (CISH) methods known in the art.

Expression based on detection of a presence, increase, or decrease in protein levels or activity may also be used. Detection may be performed by any immunohistochemistry (IHC) based, blood based (especially for secreted proteins), antibody (including autoantibodies against the protein) based, exfoliate cell (from the cancer) based, mass spectroscopy based, and image (including used of labeled ligand) based method known in the art and recognized as appropriate for the detection of the protein. Antibody and image based methods are additionally useful for the localization of tumors after determination of cancer by use of cells obtained by a non-invasive procedure (such as lavage or needle aspiration), where the source of the cancerous cells is not known. A labeled antibody or ligand may be used to localize the carcinoma(s) within a patient.

One embodiment using a nucleic acid based assay to determine expression is by immobilization of one or more sequences of the genes identified herein as a polynucleotide on a solid support, including, but not limited to, a solid substrate as an array or to beads or bead based technology as known in the art. In some embodiments, the assay is DASL (cDNA-mediated Annealing, Selection, extension and Ligation) assay available from Illumina. Alternatively, solution based expression assays known in the art may also be used.

The immobilized polynucleotide probes may be unique or otherwise specific to the disclosed genes (or expressed sequences) such that the polynucleotides are capable of hybridizing to a DNA or RNA corresponding to the genes (or expressed sequences). These polynucleotides may be the full length of the genes (or expressed sequences) or be probes of shorter length (up to one nucleotide shorter than the full length sequence known in the art by deletion from the 5' or 3' end of the sequence) that are optionally minimally interrupted (such as by mismatches or inserted non-complementary basepairs) so that their hybridization with cognate DNA or RNA corresponding to the genes (or expressed seuqences) is not affected. In many embodiments, a polynucleotide probe contains sequence from the 3' end of a disclosed gene or expressed sequence. Polynucleotide probes containing mutations relative to the sequences of the disclosed genes may also be used so long as the presence of the mutations still allows hybridization to produce a detectable signal.

The immobilized polynucleotides may be used to determine the state of nucleic acid samples prepared from sample prostate cell(s) for which the outcome of the sample's subject (e.g. patient from whom the sample is obtained) is not known or for confirmation of an outcome that is already assigned to the sample's subject. Without limiting the disclosure, such a cell may be from a patient with prostate cancer, such as material removed by prostatectomy. The immobilized polynucleotide(s) need only be sufficient to specifically hybridize to the corresponding nucleic acid molecules derived from the sample under suitable conditions. While expression of even a single correlated gene may to able to provide adequate accuracy in discriminating between two prostate cancer outcomes, the disclosure includes use of expression levels from more than one gene or expressed sequence.

Therefore, the disclosure includes use of two or more, three or more, four or more, five or more, six or more, seven or more, eight or more, nine or more, ten or more, or eleven or more genes or expressed sequences disclosed herein to discriminate among outcomes. Additionally, expression levels of 12 or more, 14 or more, 16 or more, 18 or more, 20 or more, 22 or more, 24 or more, 26 or more, 28 or more, 30 or more, 32 or more, 34 or more, 36 or more, 38 or more, 40 or more, 45 or more, 50 or more, 55 or more, 60 or more, 62 or more, 65 or more, 70 or more, 75 or more, 80 or more, 85 or more, 90 or more, or 92 or more genes or expressed sequences may be assayed and used in a disclosed method. Of course additional embodiments include using 100 or more, 150 or more, 200 or more, 250 or more, 300 or more, up to the 337 in each of Table 1 and Figure 14, or the total 362 genes (or expressed sequences) as disclosed herein.

In some embodiments, the genes (expressed sequences) of the set of 62, or the set of 92, are used. In other embodiments, the genes (or expressed sequences) used are from the set of 337 in Table 1 and Figure 14, or are a combination of genes from there and those in Table 3 and Figure 15. In many cases, the combination or set of genes (or expressed sequences) used includes Gene No: 1 (FEV) or an expressed sequence which hybridizes to SEQ ID NO: 1. In further embodiments, a combination includes one or more genes or expressed sequences expressed with a correlation p value of <0.0001.

Alternatively, a combination includes genes or expressed sequences with a high frequency of occurrence as disclosed herein. Non-limiting examples of such genes or expressed sequences with a frequency of more than 400, more than 350, more than 300, more than 250, more than 200, more than 150, or more than 100 as described herein. But of course combinations of one or more genes (or sequences) with a higher frequency with one or more genes (or sequences) with a lower frequency may also be used.

In embodiments where only two or a few genes are to be analyzed, the nucleic acid derived from the sample prostate cancer cell(s) may be preferentially amplified by use of appropriate primers (such as used in PCR) such that only the genes to be analyzed are amplified to reduce contaminating background signals from other expressed genes or sequences in the sample. The size of a PCR amplicon of the disclosure may be of any size, including at least or about 50, at least or about 100, at least about or 150, at least or about 200, at least or about 250, at least or about 300, at least or about 350, or at least or about 400 consecutive nucleotides, all with inclusion of the portion complementary to the PCR primers used. Of course the PCR may optionally be reverse-transcription coupled PCR (or RT-PCR in the case of RNA starting material) or quantitative PCR, such as real-time PCR, or combinations thereof. Of course RNA from the samples described herein may be prepared and used by means readily known to the skilled person.

Alternatively, and where multiple genes are to be analyzed or where very few cells (or one cell) is used, the nucleic acid from the sample may be globally amplified before hybridization to immobilized polynucleotide probes, such as on an array or microarray. Of course RNA, or the cDNA counterpart thereof may be directly labeled and used, without amplification, by methods known in the art.

The disclosure provides a more objective set of criteria, in the form of gene expression profiles of a discrete set of genes, to discriminate (or delineate) between prostate cancer, or post-prostatectomy, outcomes. In some embodiments, the assays are used to discriminate between better and poorer outcomes within 10, or about 10, years after surgical intervention to remove prostate cancer tumors. Comparisons that discriminate between outcomes after about 10, about 20, about 30, about 40, about 50, about 60, about 70, about 80, about 90, or about 100 months may also be performed.

While better and poorer cancer recurrence, metastasis and/or survival outcomes may be defined relatively in comparison to each other, a "better" outcome may be viewed as one that is better than a 50% chance of cancer recurrence and/or 50% chance of survival after about 60 months post surgical intervention to remove prostate cancer tumor(s). A "better" outcome may also be a better than about 60%, about 70%, about 80% or about 90% cancer recurrence and/or chance of survival about 60 months post surgical intervention. A "poorer" outcome may be viewed as a 50% or more chance of cancer recurrence and/or less than 50% chance of survival after about 60 months post surgical intervention to remove prostate cancer tumor(s).

The disclosed methods may also be used with solid tissue material. For example, a solid biopsy may be collected and prepared for visualization followed by determination of expression of two or more genes or expressed sequences identified herein to determine the prostate cancer, or post-prostatectomy, outcome. One means is by use of *in situ* hybridization with polynucleotide or protein identifying probe(s) for assaying expression of said gene(s).

In some embodiments, the detection of gene expression from the samples may be by use of a single microarray able to assay gene expression from some or all genes disclosed herein for convenience and accuracy.

### Additional embodiments

Other uses of the disclosure include providing the ability to identify prostate cancer cell samples as correlated with particular prostate cancer survival or recurrence outcomes for further research or study. This provides a particular advantage in many contexts requiring the identification of cells based on objective genetic or molecular criteria.

The materials for use in the methods of the present disclosure are ideally suited for preparation of kits produced in accordance with well known procedures. The disclosure thus provides kits comprising agents for the detection of expression of the disclosed genes and sequences for identifying prostate cancer, or post-prostatectomy, outcomes. Such kits optionally comprise the agent with an identifying description or label or instructions relating to their use in the methods of the present disclosure, is provided. Such a kit may comprise containers, each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more primer complexes of the present disclosure (e.g., appropriate length poly(T) or random primers linked to a promoter reactive with the RNA polymerase). A set of instructions will also typically be included.

The methods provided by the disclosure may also be automated in whole or in part. All aspects of the disclosure may also be practiced such that they consist essentially of a subset of the disclosed genes to the exclusion of material irrelevant to the identification of prostate cancer recurrence, metastasis, and/or survival outcomes via a cell containing sample.

Having now generally provided the disclosure, the same will be more readily understood through reference to the following examples which are provided by way of illustration, and are not intended to be limiting of the disclosure, unless specified.

### EXAMPLES

### Example I: Clinical specimen collection and clinicopathological parameters.

Prostate samples with PSA and other patient outcome data from radical prostatectomies in 1993-1995 were used to discover sets of genes (expressed sequences), the expression levels of which correlate with clinical prostate cancer, or post-prostatectomy, outcome. Other samples were used to test, or verify the predictive or prognostic ability of the identified gene sets.

The characteristics of the patient profiles corresponding to the samples are shown in Table 2.

**Table 2**

| **Factor Description** | | **Train (n=124)** | **Test (n=67)** | **All (n=191)** |
|---|---|---|---|---|
| **Age** | Mean | 61.8 | 62.7 | 6.2.1 |
| | Range | 45 - 77 | 50 - 78 | 45 - 78 |
| **Gleason Score** | <=6 | 42 (34%) | 27 (40%) | 69 (36%) |
| | 7 | 65 (52%) | 32 (48%) | 97 (51%) |
| | >=8 | 17 (14%) | 8 (12%) | 25 (13%) |
| **AJCC Stage** | II | 95 (77%) | 49 (73%) | 144 (75%) |
| | III | 29 (23%) | 18 (27%) | 47 (25%) |
| **Surgical Margin** | positive | 51 (41%) | 26 (39%) | 77 (40%) |
| | negative | 73 (59%) | 41 (61%) | 114 (60%) |
| **BCF Follow-up (years)** | Mean | 6.9 | 7.7 | 7.2 |
| | Range | 0.2 - 11.5 | 1.6 - 11.4 | 0.2 - 11.5 |
| **BCF Event** | No | 71 (57%) | 43 (64%) | 114 (60%) |
| | Yes | 53 (43%) | 24 (36%) | 77 (40%) |
| **MFS Follow-up (years)** | Mean | 9 | 8.7 | 8.9 |
| | Range | 0.2 - 13.3 | 0.3 - 12.7 | 0.2 - 13.3 |
| | Unknown | 1 | 1 | 2 |
| **Metastasis Event** | No | 112 (91%) | 62 (94%) | 174 (92%) |
| | Yes | 11 (9%.) | 4 (6%) | 15 (8%) |
| | Unknown | 1 | 1 | 2 |
| **Pre-opPSA** | Mean | 8.4 | 8.9 | 8.6 |
| | Range | 1.1 - 37.2 | 1.7 - 31.8 | 1.1 - 37.2 |
| | Unknown | 49 | 20 | 69 |

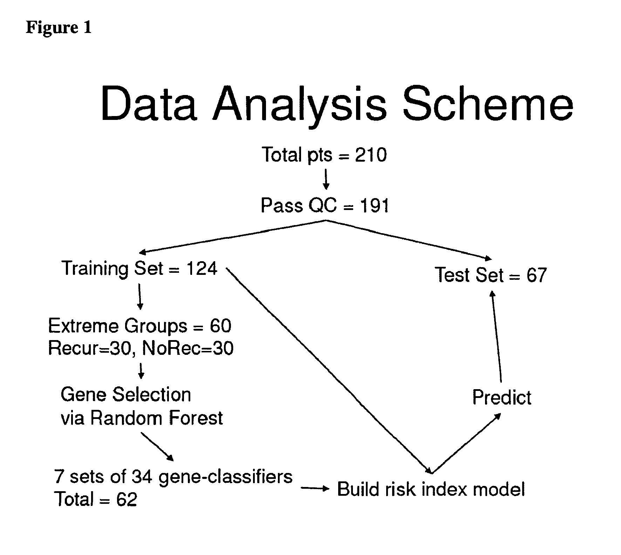

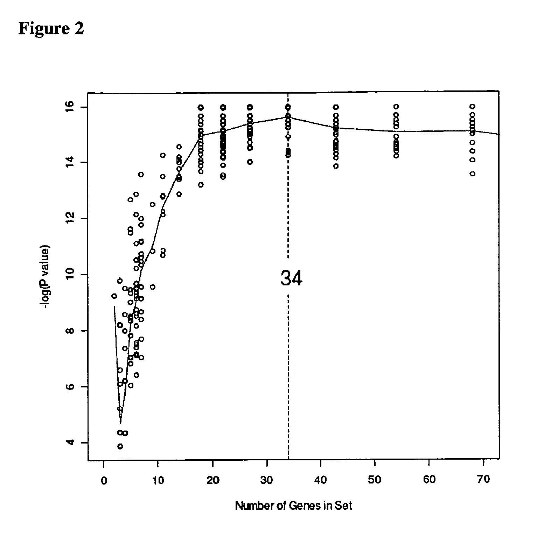

The methodology for using the patient samples for this identification is schematically illustrated in Figure 1. Briefly, 191 patient samples were selected from an initial set of 210. The 191 were divided into a training set (124 samples) and a test set (67 samples), where the former was used to identify gene sets via the Random Forests™ algorithm. Representative results with 500 independent runs (gene sets) obtained by use of the algorithm are shown in Figure 2 as a plot of the number of genes (expressed sequences) in a set versus the log of unadjusted P values (equivalent to log rank test). Figure 2 shows the performance of these 500 sets, containing various numbers of genes (expressed sequences) and their ability to accurately classify samples (and so patients) as high or low risk of cancer recurrence and/or metastasis with significance as indicated by the observed unadjusted P values. The genes (expressed sequences) in the 500 sets reflect 337 unique (non-duplicate) nucleic acid molecules that are summarized in Table 1 above and Figure 14 herein.

Seven sets of 34 genes generated by the algorithm were reviewed and found to reflect 62 non-duplicate molecules which are identified in Table 1 above (see "set of 62" column therein, which may be cross referenced with Figure 14 via Gene No. (in comparison to SEQ ID NO) and frequency of occurrence among the 337 entries. The expression levels of these 62 genes (expressed sequences) are discussed herein as a non-limiting, and representative, example of gene sets, including the additional sets shown in Figure 2 and as disclosed herein. So expression levels of the 62 genes in prostate cancer cell samples were used as a risk index (predictor) of outcome in the test set as illustrated in Figure 1.

### Example II: Identification of subtypes with different patient outcomes

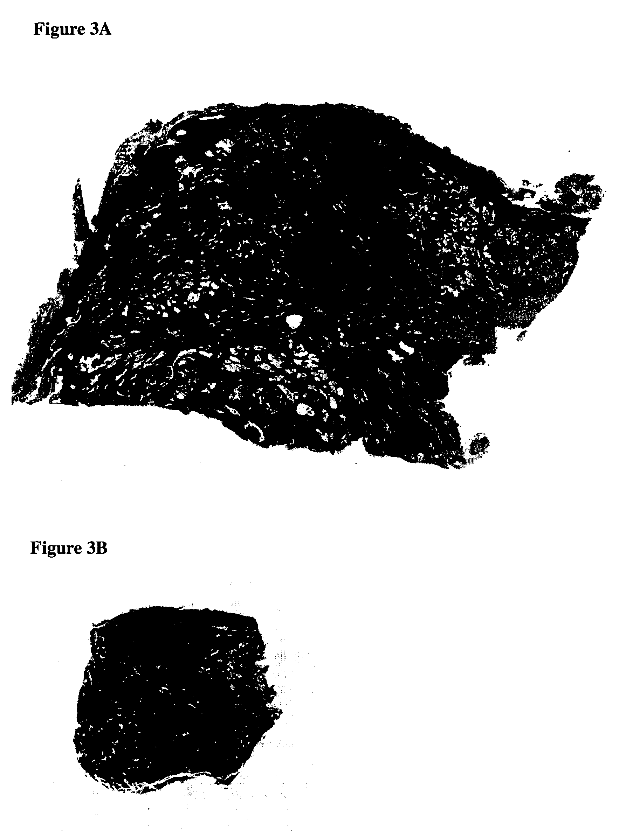

Gene selection was used with a training set of 124 samples to identify genes that predicted outcomes. See Figure 1 and Table 2. The identified genes were then used to predict outcomes in a test set of 67 samples processed by isolation of cancerous cells (see Figures 3A and 3B). The predicted outcomes were compared to the documented outcomes for the patients from whom the samples were obtained.

As a representative example, gene-specific oligonucleotide probes are immobilized, such as on a microarray and then hybridized to Cy5-labeled sample RNA and Cy3-labeled reference RNA in a co-hybridization reaction at 65°C in 1x hybridization buffer. The immobilized label is washed at 37°C with 0.1x SSC/0.005% Triton X-102. Image analysis is performed using image analysis software. Raw Cy5/Cy3 ratios is normalized using intensity-dependent non-linear regression.

Normalized Cy5/Cy3 ratios from all samples are median (or mean) centered for each gene. Identification and permutation testing for significance of differential gene expression is performed using appropriate software, such as Cox regression analysis and Random Forests™. Disease free survival is calculated from the date of diagnosis. Events are scored as the first recurrence or distant metastasis. Survival curves were calculated by the Kaplan-Meier estimates and compared by *log-*rank tests.

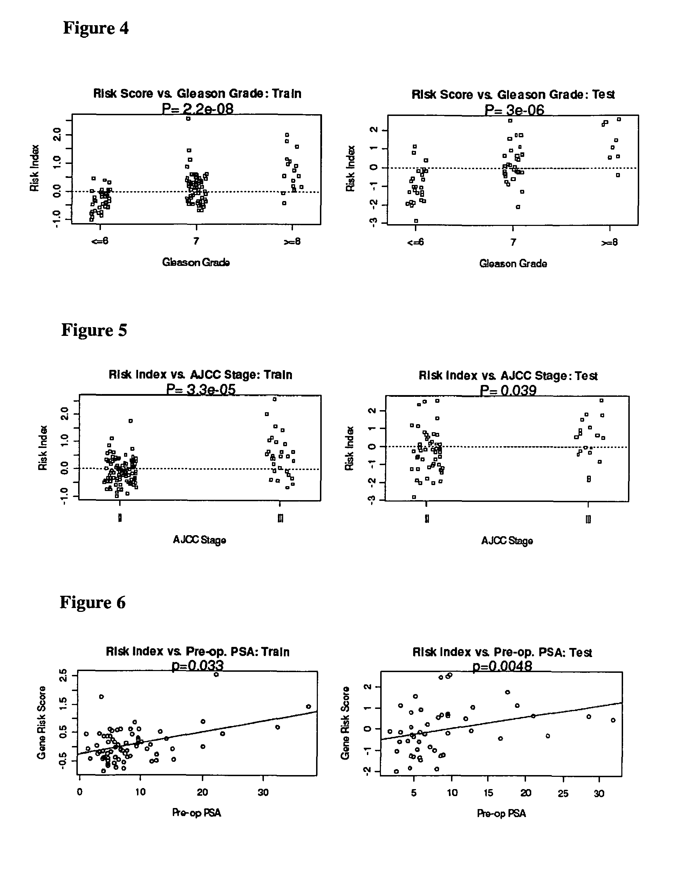

Figures 4-6 show the performance of a representative Risk Index (or Risk Score) using the disclosed 62 gene set as a combination in comparison to known factors used for assessing prostate cancer samples and patients. The factors are Gleason Grade (or Gleason Score), AJCC staging system for prostate cancer, and pre-operation (pre-surgical intervention) PSA (prostate serum antigen) levels. These comparisons show that a gene expression profile of the disclosure correlates with these other factors and so may be used in combination with any one or more of these factors or used alone to recapitulate information provided by each of these factors.

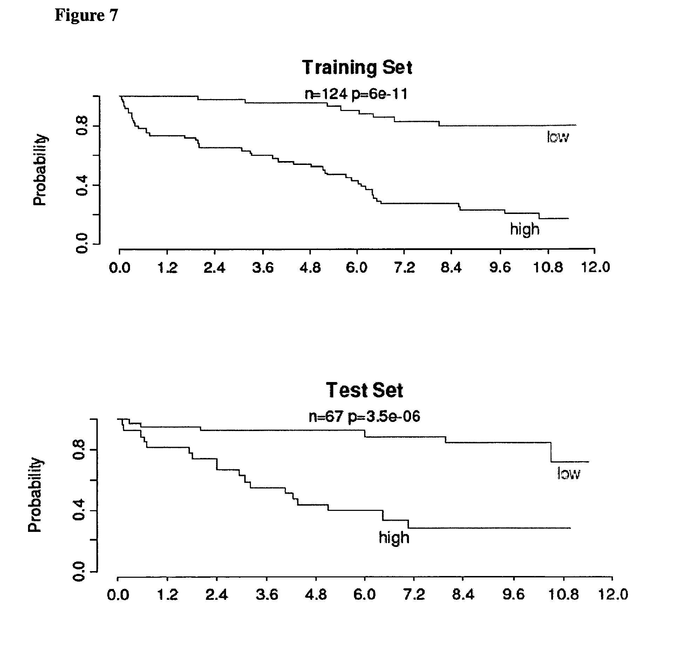

Expression levels of the 62 genes (expressed sequences) were also found to be able to segregate the 124 samples of the training set and the 67 samples of the test set into "high" and "low" risk classifications (see Figure 7).

### Example III: Identification of subtypes within different prostate cancer grades

In combination with Figure 7, Figure 4 demonstrates that a Risk Score (or Risk Index) in the "high" and "low" subclasses, based on expression levels of the representative 62 gene set, may be used to classify samples (and so patients) in a manner consistent with Gleason Grade, where the higher the grade, the greater the likelihood of a Risk Score above a value of zero, and so a higher risk of a poor outcome. The zero value represents the mean and/or the median value for the Score across all prostate cancer samples in the set. The "high risk" classification is based on a Risk Score above zero, and the "low risk" classification is based on values below zero. As a result, the Risk Score (or Risk Index) may be used to classify samples, and so patients, with a Gleason Grade of ≤6 or 7 (or even ≥8) into "high" and "low" risk groups as described herein. See Figure 8.

This demonstrates that the Risk Score (or Risk Index) may be used to classify samples, and so patients, independent from use of the Gleason Grade. Alternatively, the two analyses may be used together, optionally in series, such as determination of the Gleason Grade followed by determination of the Risk Score in samples (and so patients) with a Grade of ≤6 or 7 (or even ≥8). The combined use is particularly advantageous to "rule in" subjects at high risk for appropriate or further clinical treatment, such as by chemotherapy or radiation or cryotherapy as known to the skilled clinician and to "rule out" subjects at low risk so that they are not unnecessarily subject to treatments with undesirable side effects.

### Example IV: Identification of subtypes within different prostate cancer stages

In combination with Figure 7, Figure 5 demonstrates that a Risk Score (or Risk Index) in the "high" and "low" subclasses, based on expression levels of the representative 62 gene set, may be used to classify samples (and so patients) in a manner consistent with AJCC prostate cancer stage, where stage III is more likely to correlate with a Risk Score above a value of zero, and so higher risk of a poor outcome outcome is present. The zero value again represents the mean and/or the median value for the Score across all prostate cancer samples in the set. The "high risk" group is again based on a Risk Score above zero, and the "low risk" group is based on values below zero. As a result, the Risk Score (or Risk Index) may be used to classify samples, and so patients, with an AJCC stage II or III classification into "high" and "low" risk groups as described herein.

This demonstrates that the Risk Score (or Risk Index) may be used to classify samples, and so patients, independent from use of the AJCC stage assessment. Alternatively, the two analyses may be used together, optionally in series, such as determination of the AJCC stage followed by determination of the Risk Score in samples (and so patients) with stage II or III prostate cancer. The combined use is particularly advantageous to "rule in" subjects at high risk for appropriate clinical treatment, as known to the skilled clinician, and to "rule out" subjects at low risk so that they may reasonably avoid treatments with undesirable side effects. Combined used may also alter the classification of a patient or subject as suited for "watchful waiting."

### Example V: Combination with pre-operative PSA analysis

While the Gleason Grade and AJCC stage analysis both require use of prostate cancer cell samples, the consistencies in Figure 6 between Risk Score (or Risk Index) in the "high" and "low" subclasses, based on expression levels of the representative 62 gene set, indicates that a pre-operative PSA value should be considered in combination with a Risk Score as described herein. The combination may be in series, such as determination of the pre-operative PSA value followed by determination of the Risk Score in samples (and so patients) with PSA values of 30 or more, 25 or more, 20 or more, 15 or more, 10 or more, or 5 or more.

### Example VI: Identification of genes by Cox regression analysis

Cox regression analysis was used to identify 92 genes (or expressed sequences) the expression levels of which are capable of classifying prostate cancer cell containing samples as described above. Sixty-seven (67) of these 92 genes (or expressed sequences) are common to the 337 genes (or expressed sequences) in Figure 14 and Table 1 (see "Cox92" column in Figure 14 or Table 1, which can be cross referenced to each other by Gene No. (versus SEQ ID NO) and frequency. The remaining twenty-five (25) genes (or expressed sequences) out of the 92 are listed in Figure 15 and Table 3 below, which may be cross referenced to each other by Gene No. (in comparison to SEQ ID NO) as well as z and p values.

**Table 3**

| SEQ ID NO: | Probe_Sequence | Z | p |
|---|---|---|---|
| 338 | GATGCACCAGGCGAGGAAACGAGACCTCTTTCGTTCCTTCTAG | -4.999253 | 4.18E-06 |
| 339 | ATGCCTGAGGCATGAGTGACTGTGCATTTGAGATAGTTTTCCCTAT | -4.834638 | 4.97E-06 |
| 340 | TGAAAAAGAACAAGAGCTGGACACATTAAAAAGAAAGAGTCCATCAGATT | 4.3810131 | 2.31 E-05 |
| 341 | CAGAAGGACCTGGGGGATGGCGTGTATGGCTTCGAGTATTACC | -4.181005 | 2.89E-05 |
| 342 | AGGGCTGTCATCAACATGGATATGACATTTCACAACAGTGACTAG | -3.804427 | 5.90E-05 |
| 343 | TGGCTTTGCACAGAACCAGCTAGTTATTTGGAAGTACCCAACC | 4.2565288 | 5.95E-05 |
| 344 | TGTGGGTTAGCATCAAGTTCTCCCCAGGGTAGAATTCAATCAGAGCT | -4.211286 | 8.06E-05 |
| 345 | CAATGCAAAAAGTATTCGCTGCTGTTTACATTAGAAATCACTTCCAGC | -3.829636 | 8.59E-05 |
| 346 | TGTACAGTGGAACCATGTGACCATGTCTTGTGCTTGCAATATAGAAA | 3.7423758 | 0.0001308 |
| 347 | GGCAAATCCTTCAAGCAGGGATAAAAGTCGATCTTCAAACATTAACTT | -3.604833 | 0.0001884 |
| 348 | GGGTTTGGGGGAGATTTTACTCCTTTCTTCAACAACTATTCACTGGA | -3.554167 | 0.0003955 |
| 349 | TTTTAGGCCTTTGGGGGAATTGATTTTTATCCACAGGTAGAAAATG | -3.576323 | 0.0004075 |
| 350 | CTATGCAGGAAAATAGCACCCCCCGTGAGGACTAATCCAGATACATC | -3.553166 | 0.0004276 |
| 351 | AGGTATGGCCTCACAAGTCTTGGTCTACCCACCATATGTTTATCAAA | -3.464213 | 0.0006149 |
| 352 | AGGAGGTGTCAGACTGCTGAAGCCGACTCTGAAAGTGATCATGAAGT | 3.5820846 | 0.0006381 |
| 353 | GAGGGCATGTTGTCCATATCCCTGTGGAATACAGACCGTGTAACT | 3.4420595 | 0.0006641 |
| 354 | TCTAATGTGCACGGTGTGACTGGCAGAGTGAGTTTAAAAGCTTTACGA | -3.230789 | 0.0007048 |
| 355 | TGTTGCCAAGCTAGTCTACAAAGCATCTGATTTTGGAAGTACATGGAAT | 3.6130547 | 0.0007484 |
| 356 | TTACACCTTTCCCCCCTGAAATGTATAGAATCCATTTGTCATCAGG | -3.434091 | 0.0007518 |
| 357 | AATTGGTGAACAAAAAATGCCCAAGGCTTCTCATGTCTTTATTCTG | -3.341571 | 0.0007655 |
| 358 | GGAGAATTCTTTAGGTTGTCCCCTAAAGATTCTGAAAAAGAGAATCAGA | 3.3060176 | 0.0007756 |
| 359 | GTAGCCTCACCATTAGTGGCAGCATCATGTAACTGAGTGGACTGTG | 3.8470148 | 0.0007772 |
| 360 | TTTGCTCACAAGCCATATTGGCCCGATTAGTGGTACTGTCTGACTC | 3.2305915 | 0.000808 |
| 361 | TGAGCTTACAACAGGTCTCGAGCTGGTGGACTCCTGTATTAGGTCACT | 3.2987578 | 0.000825 |
| 362 | GCATGCAGATGTCAAGGCAGTTAGGAAGTAAATGGTGTCTTGTAGA | 3.2511294 | 0.0008645 |

All references cited herein, including patents, patent applications, and publications, are hereby incorporated by reference in their entireties, whether previously specifically incorporated or not.

Having now fully described the invention, it will be appreciated by those skilled in the art that the same can be performed within a wide range of equivalent parameters, concentrations, and conditions without departing from the spirit and scope of the disclosure and without undue experimentation.

While this disclosure has been described in connection with specific embodiments thereof, it will be understood that it is capable of further modifications. This application is intended to cover any variations, uses, or adaptations of the disclosure following, in general, the principles of the disclosure and including such departures from the present disclosure as come within known or customary practice within the art to which the disclosure pertains and as may be applied to the essential features hereinbefore set forth.

We set out below exemplary embodiments of the invention:
1. A method to determine the cancer recurrence and/or survival outcome, or prognosis, of a prostate cancer afflicted subject, said method comprising assaying a sample comprising prostate cancer cells of said subject for the expression levels of genes, wherein the expression levels are correlated with
   a low risk of cancer recurrence and/or metastasis,
   a high risk of cancer recurrence and/or metastasis, or
   elevated PSA levels after about one year of prostatectomy.
2. A method to determine the risk of prostate cancer recurrence and/or metastasis in a subject, said method comprising
   assaying a sample comprising prostate cancer cells from said subject for the expression levels of two or more genes selected from Gene Nos: 1 to 362 in Figures 14 and 15,
   comparing the expression level of each gene, or the aggregated expression level, with the mean or median expression values in prostate cancer cells, and
   determining the risk of prostate cancer recurrence and/or metastasis in said subject
   wherein the expression levels are correlated with
   a low risk of cancer recurrence and/or metastasis, or
   a high risk of cancer recurrence and/or metastasis.
3. The method of item 2 wherein said method further comprises selecting a treatment for a subject with the determined cancer recurrence and/or survival outcome.
4. The method of item 1 or 2 or 3 wherein said assaying comprises preparing RNA from said sample.
5. The method of item 4 wherein said RNA is used for PCR.
6. The method of item 4 wherein said assaying comprises using an array.
7. The method of item 1 or 2 or 3 wherein said sample is dissected from tissue removed during prostatectomy.
8. The method of item 5 wherein said PCR is RT-PCR, optionally real time RT-PCR.
9. The method of item 1 or 2 or 3 or 4 or 5 or 6 or 7 or 8 wherein said expression levels are correlation is with a p value of <0.0001.
10. The method of item 1 or 2 or 3 or 4 or 5 or 6 or 7 or 8 wherein said sample comprises isolated prostate cancer cells.
11. A method to determine therapeutic treatment for a prostate cancer patient after prostatectomy, said method comprising
   determining a cancer recurrence and/or survival outcome for said patient by assaying a sample of prostate cancer cells from said patient for the expression levels of genes, wherein the expression levels are correlated with
   a low risk of cancer recurrence or metastasis,
   a high risk of cancer recurrence or metastasis, or
   elevated PSA levels after about one year of prostatectomy; and
   selecting a treatment for a patient with such a cancer recurrence and/or survival outcome.
12. The method of item 3 or 11 wherein said treatment comprises chemotherapy.
13. The method of item 3 or 11 wherein said treatment comprises radiation therapy.
14. The method of item 1 or 2 further comprising
   i) determining the grade of prostate cancer in said sample,
   ii) determining the stage of prostate cancer in said sample, or
   iii) both;
      wherein i), ii) or both i) and ii) are optionally performed before determining the risk of prostate cancer recurrence and/or metastasis in said subject.
15. The method of item 14 wherein determining prostate cancer grade comprises determination of a Gleason Score.
16. The method of item 14 wherein determining prostate cancer stage comprises determination of the AJCC stage.
17. The method of item 14 wherein the method comprises determining prostate cancer grade by a Gleason Score and determining prostate cancer stage according to AJCC stage to produce a multivariate analysis for determining the risk of prostate cancer recurrence and/or metastasis in said subject.
18. The method of item 2 further comprising determining prostate serum antigen (PSA) levels in said subject, optionally before a prostatectomy which is used to provide said sample comprising prostate cancer cells.
19. The method of item 2 wherein expression levels of 4 or more, such as 6 or more, 8 or more, 10 or more, 12 or more, 14 or more, 16 or more, 18 or more, 20 or more, 22 or more, 24 or more, 26 or more, 28 or more, 30 or more, 32 or more, 34 or more, 36 or more, 38 or more,40 or more, 45 or more, 50 or more, 55 or more, 60 or more, 65 or more, 70 or more, or 92 or more genes are assayed.
20. The method of item 2 wherein said assaying comprises determining the expression level of Gene No. 1, optionally via use of SEQ ID NO: 1 as a probe.

## Claims

1. A method to determine the cancer recurrence and/or survival outcome, or prognosis, of a prostate cancer afflicted subject, said method comprising assaying a sample comprising prostate cancer cells of said subject for the expression levels of two or more genes and determining an aggregated expression level of the genes, wherein the aggregated expression level is correlated with
a low risk of cancer recurrence and/or metastasis,
a high risk of cancer recurrence and/or metastasis, or
elevated PSA levels after about one year of prostatectomy.

2. The method of claim 1 wherein said method further comprises selecting a treatment for a subject with the determined cancer recurrence and/or survival outcome.

3. The method of claim 1 or 2 wherein said assaying comprises preparing RNA from said sample.

4. The method of claim 3 wherein said RNA is used for PCR.

5. The method of claim 3 wherein said assaying comprises using an array.

6. The method of claim 1 or 2 wherein said sample is dissected from tissue removed during prostatectomy.

7. The method of claim 4 wherein said PCR is RT-PCR, optionally real time RT-PCR.

8. The method of any preceding claim wherein said expression levels are correlated with a p value of <0.0001.

9. The method of any preceding claim wherein said sample comprises isolated prostate cancer cells.

10. A method to determine therapeutic treatment for a prostate cancer patient after prostatectomy, said method comprising
determining a cancer recurrence and/or survival outcome for said patient by assaying a sample of prostate cancer cells from said patient for the expression levels of two or more genes and determining an aggregated expression level of the genes, wherein the aggregated expression level is correlated with
a low risk of cancer recurrence or metastasis,
a high risk of cancer recurrence or metastasis, or
elevated PSA levels after about one year of prostatectomy; and
selecting a treatment for a patient with such a cancer recurrence and/or survival outcome.

11. The method of claim 3 or 10 wherein said treatment comprises chemotherapy or radiation therapy.

12. The method of claim 1 further comprising
i) determining the grade of prostate cancer in said sample,
ii) determining the stage of prostate cancer in said sample, or
iii) both;
wherein i), ii) or both i) and ii) are optionally performed before determining the risk of prostate cancer recurrence and/or metastasis in said subject.

13. The method of claim 12 wherein determining prostate cancer grade comprises determination of a Gleason Score or the AJCC stage or
wherein the method comprises determining prostate cancer grade by a Gleason Score and determining prostate cancer stage according to AJCC stage to produce a multivariate analysis for determining the risk of prostate cancer recurrence and/or metastasis in said subject.

14. The method of claim 1 further comprising determining prostate serum antigen (PSA) levels in said subject, optionally before a prostatectomy which is used to provide said sample comprising prostate cancer cells.

15. The method of claim 1 wherein expression levels of 4 or more, such as 6 or more, 8 or more, 10 or more, 12 or more, 14 or more, 16 or more, 18 or more, 20 or more, 22 or more, 24 or more, 26 or more, 28 or more, 30 or more, 32 or more, 34 or more, 36 or more, 38 or more,40 or more, 45 or more, 50 or more, 55 or more, 60 or more, 65 or more, 70 or more, or 92 or more genes are assayed.


SEQUENCE LISTING

<110>  AVIARADX, INC.
       GENERAL HOSPITAL CORPORATION

<120>  PROSTATE CANCER SURVIVAL AND RECURRENCE

<130>  N.109024A

<150>  US 60/891,477
<151>  2007-02-23

<160>  362

<170>  PatentIn version 3.5

<210>  1
<211>  46
<212>  DNA
<213>  Artificial Sequence

<220>
<223>  Probe Sequence

<400>  1
ggctcctaga agccccattc aatatcacta ctctttaacg agtgcc                    46


<210>  2
<211>  44
<212>  DNA
<213>  Artificial Sequence

<220>
<223>  Probe Sequence

<400>  2
tgactggatg gacacattgc tgtgggtagt ccctcctact agga                      44


<210>  3
<211>  50
<212>  DNA
<213>  Artificial Sequence

<220>
<223>  Probe Sequence

<400>  3
ccaactatga aaggccatag aaacgtttta attttcaatg aagtcactga                50


<210>  4
<211>  48
<212>  DNA
<213>  Artificial Sequence

<220>
<223>  Probe Sequence

<400>  4
atattttggt gtttgcgagg catgcagtca atattttgta cagttagt                  48


<210>  5

<211>  50
<212>  DNA
<213>  Artificial Sequence

<220>
<223>  Probe Sequence

<400>  5
tgcatggttg gtctgaaaat agagttgggc ttaatgttga cttctattac                50


<210>  6
<211>  46
<212>  DNA
<213>  Artificial Sequence

<220>
<223>  Probe Sequence

<400>  6
tcacatcagt cttccggaat caagatcaac atatcaggtg gtcatt                    46


<210>  7
<211>  46
<212>  DNA
<213>  Artificial Sequence

<220>
<223>  Probe Sequence

<400>  7
ccccacccct atcgtggtta ttgtgttttt ggactgaatt tacttg                    46


<210>  8
<211>  48
<212>  DNA
<213>  Artificial Sequence

<220>
<223>  Probe Sequence

<400>  8
ccggatggtg cttctaattt tctgctaacc tgtactgtgg tgtgtgta                  48


<210>  9
<211>  45
<212>  DNA
<213>  Artificial Sequence

<220>
<223>  Probe Sequence

<400>  9
cctccgagct gctagctgac aaatacaatt ctgaaggaat ccaaa                     45


<210>  10
<211>  45
<212>  DNA
<213>  Artificial Sequence

<220>

<223>  Probe Sequence

<400>  10
gcctgtatcc cggtgggagt actatgagtc agtgtacaca gaacg                    45


<210>  11
<211>  47
<212>  DNA
<213>  Artificial Sequence

<220>
<223>  Probe Sequence

<400>  11
aaaccatcag ccggcctttt atatgggtct tcactctgac tagaatt                  47


<210>  12
<211>  48
<212>  DNA
<213>  Artificial Sequence

<220>
<223>  Probe Sequence

<400>  12
gactgatgcc aggacaacct ttctcccaga tgtaaacaga gagacatg                 48


<210>  13
<211>  45
<212>  DNA
<213>  Artificial Sequence

<220>
<223>  Probe Sequence

<400>  13
cactggacac ccttcgagtg tgggttttaa catccctgtg agatt                    45


<210>  14
<211>  43
<212>  DNA
<213>  Artificial Sequence

<220>
<223>  Probe Sequence

<400>  14
gatctgggga tcacgccttg cccaagtgtg agattacctt tct                      43


<210>  15
<211>  49
<212>  DNA
<213>  Artificial Sequence

<220>
<223>  Probe Sequence

<400>  15
ttcaggctta atgctgcacc tagatataaa tgctaatgat acttgggtt                49

<210>  16
<211>  44
<212>  DNA
<213>  Artificial Sequence

<220>
<223>  Probe Sequence

<400>  16
gagtcagtgg atggacaggt ggtttcttcc cacaagagag aaat                        44


<210>  17
<211>  48
<212>  DNA
<213>  Artificial Sequence

<220>
<223>  Probe Sequence

<400>  17
tggtaggaga tactaattgg attcggagat attaattgga tttggcca                    48


<210>  18
<211>  46
<212>  DNA
<213>  Artificial Sequence

<220>
<223>  Probe Sequence

<400>  18
cctaaggtgg ttgtgctcgg agggtttctt gtttcttttc catttt                      46


<210>  19
<211>  53
<212>  DNA
<213>  Artificial Sequence

<220>
<223>  Probe Sequence

<400>  19
gctcaatatt cccagaatag ttttcaatgt attaatgaag tgattaattg gct              53


<210>  20
<211>  49
<212>  DNA
<213>  Artificial Sequence

<220>
<223>  Probe Sequence

<400>  20
ttgctttttc ttcctttggg atgttggaag ctacagaaat atttataaa                   49


<210>  21
<211>  45
<212>  DNA
<213>  Artificial Sequence

<220>
<223>  Probe Sequence

<400>  21
aggcctcatc ctccactgaa gagtatggat tgaaggattg tgaac                    45


<210>  22
<211>  43
<212>  DNA
<213>  Artificial Sequence

<220>
<223>  Probe Sequence

<400>  22
gctgaaggac cctgaggagc ttcgcaacta catggagagg atc                      43


<210>  23
<211>  44
<212>  DNA
<213>  Artificial Sequence

<220>
<223>  Probe Sequence

<400>  23
tgtttcaaaa ccacttgcca tcctgttaga ttgccagttc ctgg                     44


<210>  24
<211>  45
<212>  DNA
<213>  Artificial Sequence

<220>
<223>  Probe Sequence

<400>  24
atcaagcaag ttcctgctgc tgaaggataa gacacagatg acctg                    45


<210>  25
<211>  45
<212>  DNA
<213>  Artificial Sequence

<220>
<223>  Probe Sequence

<400>  25
taagtcgggt ggcaattgtc agggtgtggg aatttctttt cctac                    45


<210>  26
<211>  48
<212>  DNA
<213>  Artificial Sequence

<220>
<223>  Probe Sequence

<400>  26

agttctgacc caaccacaga ggatgctgac atcatttgta ttatgttc 48

<210> 27
<211> 46
<212> DNA
<213> Artificial Sequence

<220>
<223> Probe Sequence

<400> 27
ccatggcagt gggaaaaatg taggagactg tttggaaatt gatttt 46

<210> 28
<211> 42
<212> DNA
<213> Artificial Sequence

<220>
<223> Probe Sequence

<400> 28
aggacctgaa gggtgacatc caggaggggc ctctgaaatt tc 42

<210> 29
<211> 42
<212> DNA
<213> Artificial Sequence

<220>
<223> Probe Sequence

<400> 29
ggactcatct ttccctcctt ggtgattccg cagtgagaga gt 42

<210> 30
<211> 47
<212> DNA
<213> Artificial Sequence

<220>
<223> Probe Sequence

<400> 30
ctccctgaaa aaccattcct gctgaaactg ctgtagaaat tgtgaag 47

<210> 31
<211> 45
<212> DNA
<213> Artificial Sequence

<220>
<223> Probe Sequence

<400> 31
cccctgaaaa gtgagcagca acgtaaaaac gtatgtgaag cctct 45

<210> 32
<211> 47

<212>  DNA
<213>  Artificial Sequence

<220>
<223>  Probe Sequence

<400>  32
cacctgctct agggacgatt cgtttgaaag agagtaagat gcattaa                    47


<210>  33
<211>  48
<212>  DNA
<213>  Artificial Sequence

<220>
<223>  Probe Sequence

<400>  33
ccacctgttc tcaatttgca agaattagag gcgtatagag acaaattg                   48


<210>  34
<211>  50
<212>  DNA
<213>  Artificial Sequence

<220>
<223>  Probe Sequence

<400>  34
tgtttggtcg taatgtctgc atgatatttg tgcacattta ttaagtatcg                 50


<210>  35
<211>  47
<212>  DNA
<213>  Artificial Sequence

<220>
<223>  Probe Sequence

<400>  35
ggctgggtgt tttcaaatgt cagcttaaaa ttggtaattg aatggaa                    47


<210>  36
<211>  48
<212>  DNA
<213>  Artificial Sequence

<220>
<223>  Probe Sequence

<400>  36
gcaagcataa gggaaaatgt cacgtaaact agatcaggga acaaaatc                   48


<210>  37
<211>  42
<212>  DNA
<213>  Artificial Sequence

<220>
<223>  Probe Sequence

<400>  37
gccaagacca cccaggaaac catcgacaag actgctaacc ag                          42


<210>  38
<211>  48
<212>  DNA
<213>  Artificial Sequence

<220>
<223>  Probe Sequence

<400>  38
tttgcaaaga atccaggaca aaaaggatta gatctagaaa tggccatt                    48


<210>  39
<211>  47
<212>  DNA
<213>  Artificial Sequence

<220>
<223>  Probe Sequence

<400>  39
ggtgactttt gcaattcagg gaagatttgg gcatattaaa tgaaaga                     47


<210>  40
<211>  43
<212>  DNA
<213>  Artificial Sequence

<220>
<223>  Probe Sequence

<400>  40
attatgccac cttggatgga gccaaggata tcgaaggctt gct                         43


<210>  41
<211>  47
<212>  DNA
<213>  Artificial Sequence

<220>
<223>  Probe Sequence

<400>  41
ttgatttggg acttgggaga cctctcttct gtaagcaact caataaa                     47


<210>  42
<211>  46
<212>  DNA
<213>  Artificial Sequence

<220>
<223>  Probe Sequence

<400>  42
aatcctgtgg tgaatggtgg tgtactttaa agctgtcacc atgtta                      46

<210>  43
<211>  46
<212>  DNA
<213>  Artificial Sequence

<220>
<223>  Probe Sequence

<400>  43
cttgagtccc acccaaaacc tctagtaggg ttttaataac gctcac                    46


<210>  44
<211>  47
<212>  DNA
<213>  Artificial Sequence

<220>
<223>  Probe Sequence

<400>  44
tctttcagaa gtgaagaggg ggctagaagg actctgagaa gttggta                   47


<210>  45
<211>  50
<212>  DNA
<213>  Artificial Sequence

<220>
<223>  Probe Sequence

<400>  45
gcagagagtg ccgatcttac tcaagtacct agactcagat acagagaagg                50


<210>  46
<211>  47
<212>  DNA
<213>  Artificial Sequence

<220>
<223>  Probe Sequence

<400>  46
cggaactctt gtgcgtaagg aaaagtaagg aaaacgattc cttctaa                   47


<210>  47
<211>  45
<212>  DNA
<213>  Artificial Sequence

<220>
<223>  Probe Sequence

<400>  47
aatgtgggaa ggtgggggtt atggaggaga taactcaaaa cttct                     45


<210>  48
<211>  47
<212>  DNA
<213>  Artificial Sequence

<220>
<223>  Probe Sequence

<400>  48
ccccatcttg tggtaacttg ctgcttctgc acttcatatc catattt                    47


<210>  49
<211>  47
<212>  DNA
<213>  Artificial Sequence

<220>
<223>  Probe Sequence

<400>  49
ttatccattc gttgtggacc cacagattgc atctttaaat tcataat                    47


<210>  50
<211>  44
<212>  DNA
<213>  Artificial Sequence

<220>
<223>  Probe Sequence

<400>  50
aggtcctcag atgggaattg cacagtagga tgtggaacct gttt                       44


<210>  51
<211>  47
<212>  DNA
<213>  Artificial Sequence

<220>
<223>  Probe Sequence

<400>  51
tgtaacattc ctgaagctgt tcccactccc agatggtttt atcaata                    47


<210>  52
<211>  48
<212>  DNA
<213>  Artificial Sequence

<220>
<223>  Probe Sequence

<400>  52
ttttaacttc tatatgggac ccgaattaga cactgctgaa tcctgtac                   48


<210>  53
<211>  47
<212>  DNA
<213>  Artificial Sequence

<220>
<223>  Probe Sequence

<400>  53
agaagagcac aaagcaaggc cattgcaaca ggcatttaaa aattatt                    47

<210>  54
<211>  44
<212>  DNA
<213>  Artificial Sequence

<220>
<223>  Probe Sequence

<400>  54
ctcaatgcat ccatcttggg ctgatcatgc cacagatctc attc                        44


<210>  55
<211>  44
<212>  DNA
<213>  Artificial Sequence

<220>
<223>  Probe Sequence

<400>  55
aattcctcgg gaaaggtgaa cctgaacaac ccaagtctct ctct                        44


<210>  56
<211>  46
<212>  DNA
<213>  Artificial Sequence

<220>
<223>  Probe Sequence

<400>  56
gctctgttac agctctgacc acgaaaaact gaagcctcag tacttg                      46


<210>  57
<211>  45
<212>  DNA
<213>  Artificial Sequence

<220>
<223>  Probe Sequence

<400>  57
ccacttgaca gtggagcaga ggggttaccc agatttcaac ctcat                       45


<210>  58
<211>  47
<212>  DNA
<213>  Artificial Sequence

<220>
<223>  Probe Sequence

<400>  58
ttctgggatt tctctagagg ctggcaagaa ccagttgttt tgtcttg                     47


<210>  59
<211>  49
<212>  DNA

<213>  Artificial Sequence

<220>
<223>  Probe Sequence

<400>  59
tgagcacctt ttaaacctgc tgcacaataa ttgaggaaat agactcttt                49


<210>  60
<211>  46
<212>  DNA
<213>  Artificial Sequence

<220>
<223>  Probe Sequence

<400>  60
cgtgtcaaca atggtaaagg ggatgtatgg cattgagaat gaagtc                46


<210>  61
<211>  46
<212>  DNA
<213>  Artificial Sequence

<220>
<223>  Probe Sequence

<400>  61
tcaaagttcc ccaagaagaa ctggaaaatg ccagcctagt gtttac                46


<210>  62
<211>  47
<212>  DNA
<213>  Artificial Sequence

<220>
<223>  Probe Sequence

<400>  62
atgttcacct ggcaatcagc tgagttgaga ctttggaata agacact                47


<210>  63
<211>  48
<212>  DNA
<213>  Artificial Sequence

<220>
<223>  Probe Sequence

<400>  63
tttaattatg gtgagcgttt ccgtttgggt acaaggaata tgagagat                48


<210>  64
<211>  45
<212>  DNA
<213>  Artificial Sequence

<220>
<223>  Probe Sequence

<400>  64
tgagagcatg ccaaaatttg ctaagtctta caaagatcaa gggct                        45


<210>  65
<211>  51
<212>  DNA
<213>  Artificial Sequence

<220>
<223>  Probe Sequence

<400>  65
tccctaccaa gtgaaaattg atgtgtgtta agagggtaca gaattatcaa c                 51


<210>  66
<211>  47
<212>  DNA
<213>  Artificial Sequence

<220>
<223>  Probe Sequence

<400>  66
ggtgatttgc tgctggcttt ctatcatttt tatgttttaa tgcaaag                      47


<210>  67
<211>  49
<212>  DNA
<213>  Artificial Sequence

<220>
<223>  Probe Sequence

<400>  67
ggccaagaat attgcaaaat acatgaagct tcatgcactt aaagaagta                    49


<210>  68
<211>  44
<212>  DNA
<213>  Artificial Sequence

<220>
<223>  Probe Sequence

<400>  68
ctcctcagga ccctctgggt cacacatctt tagggtcagt gaac                         44


<210>  69
<211>  46
<212>  DNA
<213>  Artificial Sequence

<220>
<223>  Probe Sequence

<400>  69
ggcaacagga aacaggtttt gcaagttcaa ggttcactcc ctatat                       46


<210>  70

<211>  51
<212>  DNA
<213>  Artificial Sequence

<220>
<223>  Probe Sequence

<400>  70
tgaaaaagtt atctctgggt attgcataaa aggcttcatc ttataaagtg a                51


<210>  71
<211>  42
<212>  DNA
<213>  Artificial Sequence

<220>
<223>  Probe Sequence

<400>  71
ccacgaggat ggcccacaag cagatctact actcggacaa gt                          42


<210>  72
<211>  46
<212>  DNA
<213>  Artificial Sequence

<220>
<223>  Probe Sequence

<400>  72
tctgctctcc atccagagcc ttctaggaga acccaacatt gatagt                      46


<210>  73
<211>  44
<212>  DNA
<213>  Artificial Sequence

<220>
<223>  Probe Sequence

<400>  73
gtgaggagcg aagagccctc tgctctagga tttgggttga aaaa                        44


<210>  74
<211>  46
<212>  DNA
<213>  Artificial Sequence

<220>
<223>  Probe Sequence

<400>  74
tggcactttg tttgtgttgt tggaaaaagt cacattgcca ttaaac                      46


<210>  75
<211>  46
<212>  DNA
<213>  Artificial Sequence

<220>

<223>  Probe Sequence

<400>  75
ggctggatca agggcaaaaa ctggtcatta agtcatctga cattaa                    46


<210>  76
<211>  52
<212>  DNA
<213>  Artificial Sequence

<220>
<223>  Probe Sequence

<400>  76
gagagaaatt ttaggtggtt gaaatgatta aatggaaaga gatttatttt ca             52


<210>  77
<211>  49
<212>  DNA
<213>  Artificial Sequence

<220>
<223>  Probe Sequence

<400>  77
tctttgagaa acagcgtgga ttttacttat ctgtgtattc acagagctt                 49


<210>  78
<211>  44
<212>  DNA
<213>  Artificial Sequence

<220>
<223>  Probe Sequence

<400>  78
atagagcacc cagccccacc cctgtaaatg gaatttacca gatg                      44


<210>  79
<211>  46
<212>  DNA
<213>  Artificial Sequence

<220>
<223>  Probe Sequence

<400>  79
tcacaggatc ctgagctgca cttacctgtg agagtcttca aacttt                    46


<210>  80
<211>  52
<212>  DNA
<213>  Artificial Sequence

<220>
<223>  Probe Sequence

<400>  80
gctgaagtgt tcataagata acaataggct tgaatctcca attcaaatga at             52

<210> 81
<211> 47
<212> DNA
<213> Artificial Sequence

<220>
<223> Probe Sequence

<400> 81
ttgtgacatt gtgacaagct ccatgtcctt taaaatcagt cactctg 47


<210> 82
<211> 50
<212> DNA
<213> Artificial Sequence

<220>
<223> Probe Sequence

<400> 82
tggaaactct tggaccaaga ttaggattaa tttgtttttg aagttttttg 50


<210> 83
<211> 47
<212> DNA
<213> Artificial Sequence

<220>
<223> Probe Sequence

<400> 83
cattcaatcc ggaccatttt ctggagaatg gacagtttaa gaaaagg 47


<210> 84
<211> 50
<212> DNA
<213> Artificial Sequence

<220>
<223> Probe Sequence

<400> 84
tgctgaactc acagttagac aatccatggt ttaatgcaca tgaaattacc 50


<210> 85
<211> 46
<212> DNA
<213> Artificial Sequence

<220>
<223> Probe Sequence

<400> 85
acgataacct ggcagtggaa ggaaagaagc atggtctact ttaggt 46


<210> 86
<211> 47
<212> DNA
<213> Artificial Sequence

<220>
<223>  Probe Sequence

<400>  86
ctgcttgatt tttgcctctt ccagtcttcc tgacacttta attacca                    47


<210>  87
<211>  49
<212>  DNA
<213>  Artificial Sequence

<220>
<223>  Probe Sequence

<400>  87
tcaacatcac aatggcaaag aagaaatata ctgtacaaaa ctgcaggaa                  49


<210>  88
<211>  46
<212>  DNA
<213>  Artificial Sequence

<220>
<223>  Probe Sequence

<400>  88
tttgcatgtc caaattgctt ccttcttttt agcagaaagg aggagt                     46


<210>  89
<211>  48
<212>  DNA
<213>  Artificial Sequence

<220>
<223>  Probe Sequence

<400>  89
tccaggcatt ttagaactat gcaattgtga tttaaaatgc aactttgt                   48


<210>  90
<211>  47
<212>  DNA
<213>  Artificial Sequence

<220>
<223>  Probe Sequence

<400>  90
gagacaggga aacacaaggg gagtagaagg cttcagtaga agatttc                    47


<210>  91
<211>  43
<212>  DNA
<213>  Artificial Sequence

<220>
<223>  Probe Sequence

<400>  91

gtggtcatca tcaaggcatg ccaggatacc ttcctggtgc tat                    43


<210>  92
<211>  49
<212>  DNA
<213>  Artificial Sequence

<220>
<223>  Probe Sequence

<400>  92
cctggtaagt atgcagcaca ttgcttatat cctgggtatg cattatttt              49


<210>  93
<211>  53
<212>  DNA
<213>  Artificial Sequence

<220>
<223>  Probe Sequence

<400>  93
tcaatgagta acaggaaaat tttaaaaata cagatagata tatgctctgc atg         53


<210>  94
<211>  46
<212>  DNA
<213>  Artificial Sequence

<220>
<223>  Probe Sequence

<400>  94
tgcccagttt gttcaagaag ccacttacaa ggaagtaagc aaaatg                 46


<210>  95
<211>  49
<212>  DNA
<213>  Artificial Sequence

<220>
<223>  Probe Sequence

<400>  95
gaaacggggc catatagttt ggttatgaca tcaatatttt acctaggtg              49


<210>  96
<211>  46
<212>  DNA
<213>  Artificial Sequence

<220>
<223>  Probe Sequence

<400>  96
atttccatgc cgtctacagg gatgacctga agaaattgct agagac                 46


<210>  97
<211>  47

<212>  DNA
<213>  Artificial Sequence

<220>
<223>  Probe Sequence

<400>  97
tgcataattc attgttgcca aggaataaag tgaagaaaca gcacctt                    47


<210>  98
<211>  45
<212>  DNA
<213>  Artificial Sequence

<220>
<223>  Probe Sequence

<400>  98
ctgctcttgt gcccttctga gcccacaata aaggctgagc tctta                      45


<210>  99
<211>  44
<212>  DNA
<213>  Artificial Sequence

<220>
<223>  Probe Sequence

<400>  99
cctccggaag ctgtcgactt catgacaagc attttgtgaa ctag                        44


<210>  100
<211>  49
<212>  DNA
<213>  Artificial Sequence

<220>
<223>  Probe Sequence

<400>  100
catagctctt tggctcgtga acctaattgt aaactttcag gtatttttg                  49


<210>  101
<211>  47
<212>  DNA
<213>  Artificial Sequence

<220>
<223>  Probe Sequence

<400>  101
ccagttatgc agcacctggc taagaatgta gtcatggtaa atcaagg                    47


<210>  102
<211>  51
<212>  DNA
<213>  Artificial Sequence

<220>
<223>  Probe Sequence

<400>  102
ggcaggcact ttaataccaa actgtaacat gtctcaactg tatacaactc a          51


<210>  103
<211>  47
<212>  DNA
<213>  Artificial Sequence

<220>
<223>  Probe Sequence

<400>  103
gccacactga aaaggaaaat gggaatttat aacccagtga gttcagc          47


<210>  104
<211>  46
<212>  DNA
<213>  Artificial Sequence

<220>
<223>  Probe Sequence

<400>  104
ctgggcatac aaccctctgc tttcacatct ctgagctata tcctca          46


<210>  105
<211>  49
<212>  DNA
<213>  Artificial Sequence

<220>
<223>  Probe Sequence

<400>  105
agcagacaaa aaaggcactt ttcagaacat caaattccta atgaagaag          49


<210>  106
<211>  47
<212>  DNA
<213>  Artificial Sequence

<220>
<223>  Probe Sequence

<400>  106
tgcttcattg tgcccttttt cttattggtt tagaactctt gattttg          47


<210>  107
<211>  46
<212>  DNA
<213>  Artificial Sequence

<220>
<223>  Probe Sequence

<400>  107
gatgtggcag aatccacacc agcttatcaa ccaacacagc taattt          46

<210>  108
<211>  50
<212>  DNA
<213>  Artificial Sequence

<220>
<223>  Probe Sequence

<400>  108
gggggagtaa aaaattgaat tttaacaaaa gatcttaggg gaatgtgatt                50


<210>  109
<211>  48
<212>  DNA
<213>  Artificial Sequence

<220>
<223>  Probe Sequence

<400>  109
ccttgatgct gtctgtacag ggttcatatt ttgtagcgaa agtcgttt                  48


<210>  110
<211>  44
<212>  DNA
<213>  Artificial Sequence

<220>
<223>  Probe Sequence

<400>  110
catccaggac actgggagca catagagatt cacccatgtt tgtt                      44


<210>  111
<211>  47
<212>  DNA
<213>  Artificial Sequence

<220>
<223>  Probe Sequence

<400>  111
cggaagaact gagcactctg ttctccaaac ctatcagaaa tttgtgg                   47


<210>  112
<211>  52
<212>  DNA
<213>  Artificial Sequence

<220>
<223>  Probe Sequence

<400>  112
accagtaggg gcttataata aaggactgta atcttattta ggaagttgac tt             52


<210>  113
<211>  46
<212>  DNA
<213>  Artificial Sequence

<220>
<223>  Probe Sequence

<400>  113
cactttcaga taagaggtgt ttgctgggat ggaagaacta cctggc                    46


<210>  114
<211>  46
<212>  DNA
<213>  Artificial Sequence

<220>
<223>  Probe Sequence

<400>  114
gaaagccttc ctcgggttca aagctggatt ttgaactgaa gaagat                    46


<210>  115
<211>  48
<212>  DNA
<213>  Artificial Sequence

<220>
<223>  Probe Sequence

<400>  115
tgaaaattgg tagatcagag ttgagctgat tggaggacca aattaaaa                  48


<210>  116
<211>  45
<212>  DNA
<213>  Artificial Sequence

<220>
<223>  Probe Sequence

<400>  116
ttccattgta attgctatcg ccatcacagc tgaacttgtt gagat                     45


<210>  117
<211>  48
<212>  DNA
<213>  Artificial Sequence

<220>
<223>  Probe Sequence

<400>  117
gacacagatg actctttggt gttggtcttt ttgtctgcag tgaatgtt                  48


<210>  118
<211>  43
<212>  DNA
<213>  Artificial Sequence

<220>
<223>  Probe Sequence

<400>  118
tcaagaaagg gattccgagg ccaataagcc ctcctttctc ttg                       43

<210>  119
<211>  46
<212>  DNA
<213>  Artificial Sequence

<220>
<223>  Probe Sequence

<400>  119
tccagataac tttcaggcac tgctggagtg tcggataaat tctggt                    46


<210>  120
<211>  48
<212>  DNA
<213>  Artificial Sequence

<220>
<223>  Probe Sequence

<400>  120
aatcctcaca tcgtgccaaa cttagtctgg ttactaagcc taaaaaca                  48


<210>  121
<211>  45
<212>  DNA
<213>  Artificial Sequence

<220>
<223>  Probe Sequence

<400>  121
attgaggatt tgtgggcagc cagagggagt ctgactgaag tttac                     45


<210>  122
<211>  46
<212>  DNA
<213>  Artificial Sequence

<220>
<223>  Probe Sequence

<400>  122
tgccagaatc tagtgggatg gaagtttttg ctacatgtta tccacc                    46


<210>  123
<211>  51
<212>  DNA
<213>  Artificial Sequence

<220>
<223>  Probe Sequence

<400>  123
ttccttcatg taacttccct gaaaaatcta agtgtttcat aaatttgaga g              51


<210>  124
<211>  46
<212>  DNA

<213>  Artificial Sequence

<220>
<223>  Probe Sequence

<400>  124
ctctggtgtt ttcgccagac aataaactta cactggaagc tttgat                    46


<210>  125
<211>  49
<212>  DNA
<213>  Artificial Sequence

<220>
<223>  Probe Sequence

<400>  125
ttgaacacag gctttgtctg aatgatgttc ttttatctct tgaacacaa                 49


<210>  126
<211>  45
<212>  DNA
<213>  Artificial Sequence

<220>
<223>  Probe Sequence

<400>  126
gaagccaaag tacccgcact gcgaggagaa gatggttatc atcac                     45


<210>  127
<211>  42
<212>  DNA
<213>  Artificial Sequence

<220>
<223>  Probe Sequence

<400>  127
agctggcgcc agcttcttct cctggatcca gtaagagttt cg                        42


<210>  128
<211>  44
<212>  DNA
<213>  Artificial Sequence

<220>
<223>  Probe Sequence

<400>  128
cctgaaggaa accactggct tgatatttct gtgactcgtg ttgc                      44


<210>  129
<211>  42
<212>  DNA
<213>  Artificial Sequence

<220>
<223>  Probe Sequence

<400>  129
ccacctttcc tcccagcaag catctggcca atcctattct tc                          42


<210>  130
<211>  46
<212>  DNA
<213>  Artificial Sequence

<220>
<223>  Probe Sequence

<400>  130
gtgaggtaca ggcggaagtt ggaatcaggt tttaggattc tgtctc                      46


<210>  131
<211>  44
<212>  DNA
<213>  Artificial Sequence

<220>
<223>  Probe Sequence

<400>  131
cttggcctga agaggtgcag aaaatacaga ccaaagttga ccag                        44


<210>  132
<211>  48
<212>  DNA
<213>  Artificial Sequence

<220>
<223>  Probe Sequence

<400>  132
acatagtgac atgcacacgg gaaagcctta aaaatatcct tgatgtac                    48


<210>  133
<211>  45
<212>  DNA
<213>  Artificial Sequence

<220>
<223>  Probe Sequence

<400>  133
taatgcaagc cctgactggg tggaagctga agtcttgctg tttta                       45


<210>  134
<211>  47
<212>  DNA
<213>  Artificial Sequence

<220>
<223>  Probe Sequence

<400>  134
tccttttttg gggaaatctg agcctagctc agaaaaacat aaagcac                      47


<210>  135

<211>  46
<212>  DNA
<213>  Artificial Sequence

<220>
<223>  Probe Sequence

<400>  135
ggcccttcct gatgatcatt gtccctacag acacccagaa catctt                    46


<210>  136
<211>  46
<212>  DNA
<213>  Artificial Sequence

<220>
<223>  Probe Sequence

<400>  136
ggagctgggg agctgtgtta agtcaaagta gaaaccctcc agtgtt                    46


<210>  137
<211>  48
<212>  DNA
<213>  Artificial Sequence

<220>
<223>  Probe Sequence

<400>  137
gcagaaaaga agacgagaat gcaaccatac ctagatggac ttttccac                  48


<210>  138
<211>  46
<212>  DNA
<213>  Artificial Sequence

<220>
<223>  Probe Sequence

<400>  138
aacaagtggg attttctggg ccagcaagtc ttccaaactg tatatg                    46


<210>  139
<211>  47
<212>  DNA
<213>  Artificial Sequence

<220>
<223>  Probe Sequence

<400>  139
gctgtgtggg tcacacaagg tctacattac aaaagacaga attcagg                   47


<210>  140
<211>  48
<212>  DNA
<213>  Artificial Sequence

<220>

<223>  Probe Sequence

<400>  140
tgcacagatc tgcttgatca attcccttga atagggaagt aacatttg                    48


<210>  141
<211>  47
<212>  DNA
<213>  Artificial Sequence

<220>
<223>  Probe Sequence

<400>  141
ccctatgagt ggaagggtcc attttgaagt cagtggagta agcttta                     47


<210>  142
<211>  45
<212>  DNA
<213>  Artificial Sequence

<220>
<223>  Probe Sequence

<400>  142
agagcttctg aggcgctgct ttgtcaaaag gaagtctcta ggttc                       45


<210>  143
<211>  46
<212>  DNA
<213>  Artificial Sequence

<220>
<223>  Probe Sequence

<400>  143
tgtgaaaaca agcttcaaag ccatatggac actgtgacaa tgacta                      46


<210>  144
<211>  45
<212>  DNA
<213>  Artificial Sequence

<220>
<223>  Probe Sequence

<400>  144
gcatctccct gaccttctcc agggacagaa gcaggagtaa gtttc                       45


<210>  145
<211>  42
<212>  DNA
<213>  Artificial Sequence

<220>
<223>  Probe Sequence

<400>  145
cgccagctac aatcccatgg tgctcattca aaagaccgac ac                          42

<210>  146
<211>  42
<212>  DNA
<213>  Artificial Sequence

<220>
<223>  Probe Sequence

<400>  146
gatccgggat gggagacccc actttagaaa gggtcgtcac tc                          42


<210>  147
<211>  55
<212>  DNA
<213>  Artificial Sequence

<220>
<223>  Probe Sequence

<400>  147
tggattgaga aaacctatat ccattcttta tatcaatgta tagttttagt ctcct            55


<210>  148
<211>  48
<212>  DNA
<213>  Artificial Sequence

<220>
<223>  Probe Sequence

<400>  148
ggtgaatgcc ctcaacttct cagtgaatta cagtgaagac tttgttga                    48


<210>  149
<211>  47
<212>  DNA
<213>  Artificial Sequence

<220>
<223>  Probe Sequence

<400>  149
atacggcgag gtagagttgg ccatatttca gagacttaga ttgacgt                     47


<210>  150
<211>  45
<212>  DNA
<213>  Artificial Sequence

<220>
<223>  Probe Sequence

<400>  150
gctcgtttgg tgcactctcg tgggagacaa tcagagaaca acata                       45


<210>  151
<211>  45
<212>  DNA
<213>  Artificial Sequence

<220>
<223>  Probe Sequence

<400>  151
gcctttccat ctggcatttc ccgctcattt atatgacttg ctgag                    45


<210>  152
<211>  46
<212>  DNA
<213>  Artificial Sequence

<220>
<223>  Probe Sequence

<400>  152
tccggtgacc aggtgtctac aaggacaagg ttcagaaatt gtacag                   46


<210>  153
<211>  47
<212>  DNA
<213>  Artificial Sequence

<220>
<223>  Probe Sequence

<400>  153
gcagaggttc ttttaaaggg gcagaaaaac tctgggaaat aagagag                  47


<210>  154
<211>  45
<212>  DNA
<213>  Artificial Sequence

<220>
<223>  Probe Sequence

<400>  154
ttttccccac ccgagatgaa ggatacgctg tattttttgc ctaat                     45


<210>  155
<211>  43
<212>  DNA
<213>  Artificial Sequence

<220>
<223>  Probe Sequence

<400>  155
actgcaggat acactcccct cctgctacct aggcaggcgt gag                      43


<210>  156
<211>  46
<212>  DNA
<213>  Artificial Sequence

<220>
<223>  Probe Sequence

<400>  156

ggtttaaccc gagtcaccca gctggtctca tacatagaca gcactt                    46

<210>  157
<211>  48
<212>  DNA
<213>  Artificial Sequence

<220>
<223>  Probe Sequence

<400>  157
gacacttgtt tagacgattg gccattctaa agttggtgag tttgtcaa                  48

<210>  158
<211>  49
<212>  DNA
<213>  Artificial Sequence

<220>
<223>  Probe Sequence

<400>  158
tcaccaaaag ctgtatgact ggatgttctg gttacctggt ttacaaaat                 49

<210>  159
<211>  49
<212>  DNA
<213>  Artificial Sequence

<220>
<223>  Probe Sequence

<400>  159
cataagctgg tatcagtggt tcgggggaaa tagttccatt ctatgactc                 49

<210>  160
<211>  46
<212>  DNA
<213>  Artificial Sequence

<220>
<223>  Probe Sequence

<400>  160
ttggcgatca tttcccaaga ttggtttccc ttgagttttt gttaaa                    46

<210>  161
<211>  44
<212>  DNA
<213>  Artificial Sequence

<220>
<223>  Probe Sequence

<400>  161
cctagtttga tgcccttatg tcccggaggg gttcacaaag tgct                      44

<210>  162
<211>  42

<212>  DNA
<213>  Artificial Sequence

<220>
<223>  Probe Sequence


<220>
<221>  misc_feature
<222>  (25)..(25)
<223>  n is a, c, g, or t

<400>  162
ttttcgaagg ataattttgg aggcnagaaa aaatggacgg gg                    42


<210>  163
<211>  47
<212>  DNA
<213>  Artificial Sequence

<220>
<223>  Probe Sequence

<400>  163
tgttcgcgac tagttggctc tgagatacta ataggtgtgt gaggctc               47


<210>  164
<211>  39
<212>  DNA
<213>  Artificial Sequence

<220>
<223>  Probe Sequence

<400>  164
cacctggacc cctgcattgg aactggaggc agggaacat                        39


<210>  165
<211>  45
<212>  DNA
<213>  Artificial Sequence

<220>
<223>  Probe Sequence

<400>  165
tggaaggatg gaagaaacgc ctggagaata tttgggatga gacac                 45


<210>  166
<211>  50
<212>  DNA
<213>  Artificial Sequence

<220>
<223>  Probe Sequence

<400>  166
gcattacttt gaatttaatg ttgcgcttgt gcactgtgtt aatattgttt            50


<210>  167

<211>  45
<212>  DNA
<213>  Artificial Sequence

<220>
<223>  Probe Sequence

<400>  167
ggtgatgggg accgtctttc ttttactgac acatgaccaa tcata                    45


<210>  168
<211>  46
<212>  DNA
<213>  Artificial Sequence

<220>
<223>  Probe Sequence

<400>  168
tgggcaaaac cattgaattt cacatgggtg gtaatatgga gttaaa                   46


<210>  169
<211>  44
<212>  DNA
<213>  Artificial Sequence

<220>
<223>  Probe Sequence

<400>  169
aaccagcctt cagagcgttc tctgtcctgc ttctaacgtc actt                     44


<210>  170
<211>  47
<212>  DNA
<213>  Artificial Sequence

<220>
<223>  Probe Sequence

<400>  170
tgccacattt gactgaattg agctgtcatt tgtacattta aagcagc                  47


<210>  171
<211>  44
<212>  DNA
<213>  Artificial Sequence

<220>
<223>  Probe Sequence

<400>  171
tatatggttt ccaaagggtg cccctatgat ccattgtccc cact                     44


<210>  172
<211>  50
<212>  DNA
<213>  artificial sequence

<220>

<223>  Probe Sequence

<400>  172
tttaaggact gatcattggc tctgaggaca cttcaactag ttagccttct                50


<210>  173
<211>  45
<212>  DNA
<213>  Artificial Sequence

<220>
<223>  Probe Sequence

<400>  173
ccctcatcaa agtcctcggt gttttttaaa ttatcagaac tgccc                      45


<210>  174
<211>  52
<212>  DNA
<213>  Artificial Sequence

<220>
<223>  Probe Sequence

<400>  174
caaatggtta ccttgttatt taacccattt gtctctactt ttccctgtac tt             52


<210>  175
<211>  39
<212>  DNA
<213>  Artificial Sequence

<220>
<223>  Probe Sequence

<400>  175
gccagctgca tgcaggagcg tgccatccag acagacttc                            39


<210>  176
<211>  44
<212>  DNA
<213>  Artificial Sequence

<220>
<223>  Probe Sequence

<400>  176
cactccaggc aggtcttggg gctcctatgt aagctgtgtt aagc                      44


<210>  177
<211>  45
<212>  DNA
<213>  Artificial Sequence

<220>
<223>  Probe Sequence

<400>  177
ggctggcaac ttagaggtgg ggagcagaga attctcttat ccaac                     45

<210>  178
<211>  48
<212>  DNA
<213>  Artificial Sequence

<220>
<223>  Probe Sequence

<400>  178
gattcatcca gccttccagc tctgttattt aaagcaagaa cacttctg                    48


<210>  179
<211>  47
<212>  DNA
<213>  Artificial Sequence

<220>
<223>  Probe Sequence

<400>  179
cctaaagcaa gcctgaattg gctatgcagt acattgtatt ctgtttg                     47


<210>  180
<211>  46
<212>  DNA
<213>  Artificial Sequence

<220>
<223>  Probe Sequence

<400>  180
cgggctttta gcagcatgta cccaaagtgt tctgattcct tcaact                      46


<210>  181
<211>  45
<212>  DNA
<213>  Artificial Sequence

<220>
<223>  Probe Sequence

<400>  181
ctaagggatg gggcagtctc tgcccaaaca taaagagaac tctgg                       45


<210>  182
<211>  49
<212>  DNA
<213>  Artificial Sequence

<220>
<223>  Probe Sequence

<400>  182
aaatccaaac tctcaattac gccatggtaa ttcagtcact aaaatatgt                   49


<210>  183
<211>  42
<212>  DNA
<213>  Artificial Sequence

<220>
<223>  Probe Sequence

<400>  183
gcaggaaccg cgagatggtc tagagtcagc ttacatccct ga                          42


<210>  184
<211>  46
<212>  DNA
<213>  Artificial Sequence

<220>
<223>  Probe Sequence

<400>  184
tgttccactg agctcctgtt gcttaccatc aagtcaacag ttatca                      46


<210>  185
<211>  45
<212>  DNA
<213>  Artificial Sequence

<220>
<223>  Probe Sequence

<400>  185
ctcaatgtaa cctcaggggc cagttttagc atttgaaatg gttct                       45


<210>  186
<211>  47
<212>  DNA
<213>  Artificial Sequence

<220>
<223>  Probe Sequence

<400>  186
tctgaccagt tacagcccca aagatgcagt gataactgtg atgtatg                     47


<210>  187
<211>  41
<212>  DNA
<213>  Artificial Sequence

<220>
<223>  Probe Sequence

<400>  187
gcatccagaa cagcctgctt ggacacagct cggtggaaga t                           41


<210>  188
<211>  48
<212>  DNA
<213>  Artificial Sequence

<220>
<223>  Probe Sequence

<400>  188

ttacaaatga ctcagcccac gtgccactca atacaaatgt tctgctat 48

<210> 189
<211> 46
<212> DNA
<213> Artificial Sequence

<220>
<223> Probe Sequence

<400> 189
acagccctgc tcccaagtac aaatagagtg acccgtaaaa tctagg 46

<210> 190
<211> 44
<212> DNA
<213> Artificial Sequence

<220>
<223> Probe Sequence

<400> 190
ccagatacta ctcggcgctg cgacactaca tcaacctcat cacc 44

<210> 191
<211> 44
<212> DNA
<213> Artificial Sequence

<220>
<223> Probe Sequence

<400> 191
aataagcagg atgttggcca ccaggtgcct ttcaaattta gaaa 44

<210> 192
<211> 50
<212> DNA
<213> Artificial Sequence

<220>
<223> Probe Sequence

<400> 192
ggaatttgat tcttccagaa tgaccttctt atttatgtaa ctggctttca 50

<210> 193
<211> 47
<212> DNA
<213> Artificial Sequence

<220>
<223> Probe Sequence

<400> 193
caaggtgtag caagtgtacc cacacagata gcattcaaca aaagctg 47

<210> 194
<211> 46

<212>  DNA
<213>  Artificial Sequence

<220>
<223>  Probe Sequence

<400>  194
cacagagtct gaaaagcggg tctccgtcta ccagaaggtg acctcc                    46


<210>  195
<211>  51
<212>  DNA
<213>  Artificial Sequence

<220>
<223>  Probe Sequence

<400>  195
tgaggactca gaagttcaag ctaaatattg tttacatttt ctggtactct g              51


<210>  196
<211>  48
<212>  DNA
<213>  Artificial Sequence

<220>
<223>  Probe Sequence

<400>  196
tggaatggtg aaagagagat gccgtgtttt gaaagtaaga tgatgaaa                  48


<210>  197
<211>  45
<212>  DNA
<213>  Artificial Sequence

<220>
<223>  Probe Sequence

<400>  197
caggggttga gagctttctg ccttagccta ccatgtgaaa ctcta                     45


<210>  198
<211>  45
<212>  DNA
<213>  Artificial Sequence

<220>
<223>  Probe Sequence

<400>  198
cacagatgag aaccacgcct agccaaaatc acttttcctg tttgc                     45


<210>  199
<211>  43
<212>  DNA
<213>  Artificial Sequence

<220>
<223>  Probe Sequence

<400>  199
aggcatggga gtcattgtcc acatcatcga gaaggacaaa atc                         43


<210>  200
<211>  41
<212>  DNA
<213>  Artificial Sequence

<220>
<223>  Probe Sequence

<400>  200
aacgggatcc tctgtggtcg cgacactgac ctagacggct t                           41


<210>  201
<211>  46
<212>  DNA
<213>  Artificial Sequence

<220>
<223>  Probe Sequence

<400>  201
tgtgtggctt cctgcaaggt accttcatct ctgagttacc tgactc                      46


<210>  202
<211>  45
<212>  DNA
<213>  Artificial Sequence

<220>
<223>  Probe Sequence

<400>  202
gctgtcctca aagcatccag tgaacactgg aagaggcttc tagaa                       45


<210>  203
<211>  48
<212>  DNA
<213>  Artificial Sequence

<220>
<223>  Probe Sequence

<400>  203
ccctttcttt gatggtgctt gcaggttttc taggtagaaa ttatttca                    48


<210>  204
<211>  47
<212>  DNA
<213>  Artificial Sequence

<220>
<223>  Probe Sequence

<400>  204
aaatgtacca atcagcatgc tgtgtctagc tcaagaactc aagctcc                     47

<210>  205
<211>  44
<212>  DNA
<213>  Artificial Sequence

<220>
<223>  Probe Sequence

<400>  205
cgtcacccca aaaagttccc tccatatccc tttgcagtca gttc                        44


<210>  206
<211>  46
<212>  DNA
<213>  Artificial Sequence

<220>
<223>  Probe Sequence

<400>  206
catgaccgca tggtatacct gggcctctca gactacttct tcaaca                      46


<210>  207
<211>  48
<212>  DNA
<213>  Artificial Sequence

<220>
<223>  Probe Sequence

<400>  207
gcattagtat gacagtaggg gggctgttag aattgctgct atactggt                    48


<210>  208
<211>  47
<212>  DNA
<213>  Artificial Sequence

<220>
<223>  Probe Sequence

<400>  208
ccaggataaa gcttccggga aaacagctat tatatcagct tttctga                     47


<210>  209
<211>  47
<212>  DNA
<213>  Artificial Sequence

<220>
<223>  Probe Sequence

<400>  209
ttcctgctac acatgccctg aatgaattgc taaatttcaa aggaaat                     47


<210>  210
<211>  47
<212>  DNA
<213>  Artificial Sequence

<220>
<223>  Probe Sequence

<400>  210
tggcctgtgg ttatcttgga aattggtgat ttatgctaga aagcttt                    47


<210>  211
<211>  49
<212>  DNA
<213>  Artificial Sequence

<220>
<223>  Probe Sequence

<400>  211
gggagaaagc taatgttttc cacaagactg aacaacgtgt atttacacg                  49


<210>  212
<211>  45
<212>  DNA
<213>  Artificial Sequence

<220>
<223>  Probe Sequence

<400>  212
gtgctgggtg catatcatcc aggataatat tctgcccaac tccat                      45


<210>  213
<211>  47
<212>  DNA
<213>  Artificial Sequence

<220>
<223>  Probe Sequence

<400>  213
cccaggatta tgtttgttga cccatctctg acagttagag ccgatat                    47


<210>  214
<211>  49
<212>  DNA
<213>  Artificial Sequence

<220>
<223>  Probe Sequence

<400>  214
gctggacagg agcactttat ctgaagacaa actcatttaa tcatctttg                  49


<210>  215
<211>  47
<212>  DNA
<213>  Artificial Sequence

<220>
<223>  Probe Sequence

<400>  215
agttgcaaaa atggctccat cggtaactca agcttcagaa tgttatg                    47

<210>  216
<211>  46
<212>  DNA
<213>  Artificial Sequence

<220>
<223>  Probe Sequence

<400>  216
tcatcggagt agattccggg tgcctttact ccactgtgac ctcata                    46


<210>  217
<211>  46
<212>  DNA
<213>  Artificial Sequence

<220>
<223>  Probe Sequence

<400>  217
gtgtgctcag gcaattattt tgctaagaat gtgaattcaa gtgcag                    46


<210>  218
<211>  47
<212>  DNA
<213>  Artificial Sequence

<220>
<223>  Probe Sequence

<400>  218
tgggaaagta tcaggagtgc catgattcca atgttttcct tctttta                   47


<210>  219
<211>  43
<212>  DNA
<213>  Artificial Sequence

<220>
<223>  Probe Sequence

<400>  219
agacggcgca gacatgtcag aacaaagtaa ggatctgagc gac                       43


<210>  220
<211>  49
<212>  DNA
<213>  Artificial Sequence

<220>
<223>  Probe Sequence

<400>  220
ccagcattaa gtactgtata tcgccctgta cttggatagg ctggctaac                 49


<210>  221
<211>  47
<212>  DNA

<213>  Artificial Sequence

<220>
<223>  Probe Sequence

<400>  221
aggcaaggag gaggggaatt ttaaaaccat cttatttgaa ctgagag                    47


<210>  222
<211>  47
<212>  DNA
<213>  Artificial Sequence

<220>
<223>  Probe Sequence

<400>  222
tggtgctcta tgctcaatga tggtcttaca cattcctcta gggaaag                    47


<210>  223
<211>  51
<212>  DNA
<213>  Artificial Sequence

<220>
<223>  Probe Sequence

<400>  223
ccctttctat tctgaacaac tgtctccatt tttcaagtgt gagagataag g               51


<210>  224
<211>  46
<212>  DNA
<213>  Artificial Sequence

<220>
<223>  Probe Sequence

<400>  224
cagtcgagac ccagatccac tgaacatctg tgtctttatt ttgctg                     46


<210>  225
<211>  46
<212>  DNA
<213>  Artificial Sequence

<220>
<223>  Probe Sequence

<400>  225
gcagacatcc tgtgaagcag gacctgctga agaggagact ttctat                     46


<210>  226
<211>  48
<212>  DNA
<213>  Artificial Sequence

<220>
<223>  Probe Sequence

<400>  226
tgagaaagct caagattcca aggcctattc aaaaatcact gaaggaaa                    48


<210>  227
<211>  44
<212>  DNA
<213>  Artificial Sequence

<220>
<223>  Probe Sequence

<400>  227
caatccgagt tcccggatga gggaacattc tgcagtataa aggg                        44


<210>  228
<211>  43
<212>  DNA
<213>  Artificial Sequence

<220>
<223>  Probe Sequence

<400>  228
cagcaccaag tctacgggtg ccagatcagt agggcctgtg att                         43


<210>  229
<211>  46
<212>  DNA
<213>  Artificial Sequence

<220>
<223>  Probe Sequence

<400>  229
agcaacagca aatcacgacc actgatagat gtctattctt gttgga                      46


<210>  230
<211>  48
<212>  DNA
<213>  Artificial Sequence

<220>
<223>  Probe Sequence

<400>  230
cttgcccatc tagcactttg gaaatcagta tttaaatgcc aaataatc                    48


<210>  231
<211>  50
<212>  DNA
<213>  Artificial Sequence

<220>
<223>  Probe Sequence

<400>  231
ttttcaggtt tattctttta gcaggtgtag ttaaacgacc tccactgaac                  50


<210>  232

<211>  55
<212>  DNA
<213>  Artificial Sequence

<220>
<223>  Probe Sequence

<400>  232
tgccaaaaat taaagtgcaa tattgtatat ttttaagaac aaatttaaaa tagaa          55


<210>  233
<211>  46
<212>  DNA
<213>  Artificial Sequence

<220>
<223>  Probe Sequence

<400>  233
ttctgaggag gagagagtga gggttttgct attgactgac ttgaac                    46


<210>  234
<211>  39
<212>  DNA
<213>  Artificial Sequence

<220>
<223>  Probe Sequence

<400>  234
ctgggggcgc aaccacccct tccttaggtt gatgtgctt                            39


<210>  235
<211>  47
<212>  DNA
<213>  Artificial Sequence

<220>
<223>  Probe Sequence

<400>  235
acgctgtgcg tttgtcagaa tgaagtatac aagtcaatgt tttcccc                   47


<210>  236
<211>  46
<212>  DNA
<213>  Artificial Sequence

<220>
<223>  Probe Sequence

<400>  236
tgttgaatac ttggccccat gagccatgcc tttctgtata gtacac                    46


<210>  237
<211>  48
<212>  DNA
<213>  Artificial Sequence

<220>

<223>  Probe Sequence

<400>  237
gctatacctc attcacagct ccttgtgagt gtgtgcacag gaaataag                    48


<210>  238
<211>  47
<212>  DNA
<213>  Artificial Sequence

<220>
<223>  Probe Sequence

<400>  238
acactgttgg acctgaccca cactgaatgt agtctttcag tacgaga                     47


<210>  239
<211>  48
<212>  DNA
<213>  Artificial Sequence

<220>
<223>  Probe Sequence

<400>  239
tcctgcaagt aagaatgttt tcacactgag ctattgattt aaccaagc                    48


<210>  240
<211>  46
<212>  DNA
<213>  Artificial Sequence

<220>
<223>  Probe Sequence

<400>  240
gggaggtcag acacgcttca ttatatctcc gtctctttta tggttt                      46


<210>  241
<211>  50
<212>  DNA
<213>  Artificial Sequence

<220>
<223>  Probe Sequence

<400>  241
tggttgtgct tgctttcctt ttttagaaag ttctagaaaa taggaaaacg                  50


<210>  242
<211>  47
<212>  DNA
<213>  Artificial Sequence

<220>
<223>  Probe Sequence

<400>  242
gctccggcag cacctttatc tatggttatg tggatgcagc atataag                     47

<210>  243
<211>  44
<212>  DNA
<213>  Artificial Sequence

<220>
<223>  Probe Sequence

<400>  243
aacctcccag agttagccag cctttcagag ttgaagtcac agct                        44


<210>  244
<211>  46
<212>  DNA
<213>  Artificial Sequence

<220>
<223>  Probe Sequence

<400>  244
atgtgcacct ttgaggctac gggcttctcc aaagacttag gaatct                      46


<210>  245
<211>  46
<212>  DNA
<213>  Artificial Sequence

<220>
<223>  Probe Sequence

<400>  245
atgtggccat taccgtcatt ggcctgtgaa gcattggaca tttata                      46


<210>  246
<211>  47
<212>  DNA
<213>  Artificial Sequence

<220>
<223>  Probe Sequence

<400>  246
ggcctgtgaa aacagaggct tttgcattgt ctcttgacat cagaagt                     47


<210>  247
<211>  44
<212>  DNA
<213>  Artificial Sequence

<220>
<223>  Probe Sequence

<400>  247
agccaaggca gggtggacag tgtgagagag ctagtgtaag ctgt                        44


<210>  248
<211>  43
<212>  DNA
<213>  Artificial Sequence

<220>
<223>  Probe Sequence

<400>  248
acatggttgt gcagggccat gtgtgaagac agcatgagtc tta                43


<210>  249
<211>  47
<212>  DNA
<213>  Artificial Sequence

<220>
<223>  Probe Sequence

<400>  249
tgatgttggt tgtaatggtt gggtttagga tgaaccattt taaggat            47


<210>  250
<211>  47
<212>  DNA
<213>  Artificial Sequence

<220>
<223>  Probe Sequence

<400>  250
gtcccaaagg tggaatacaa ccagaggtct catctctgaa ctttctt            47


<210>  251
<211>  47
<212>  DNA
<213>  Artificial Sequence

<220>
<223>  Probe Sequence

<400>  251
ggcgtctaca gagaccagcc atatggcaga tactgattgt actgtct            47


<210>  252
<211>  46
<212>  DNA
<213>  Artificial Sequence

<220>
<223>  Probe Sequence

<400>  252
tgtttgcctc aaacgctgtg tttaaacaac gttaaactct tagcct             46


<210>  253
<211>  50
<212>  DNA
<213>  Artificial Sequence

<220>
<223>  Probe Sequence

<400>  253

tgccaaaaat aaactcacat gagcacatga cagtctgagc tctataatca 50

<210> 254
<211> 47
<212> DNA
<213> Artificial Sequence

<220>
<223> Probe Sequence

<400> 254
gtgcatggac gactgaacac aatcaactgt gaggaaggag ataaact 47

<210> 255
<211> 46
<212> DNA
<213> Artificial Sequence

<220>
<223> Probe Sequence

<400> 255
ccttgccacg gttctagagc agcgtagaca gctggtaaac tgaaga 46

<210> 256
<211> 44
<212> DNA
<213> Artificial Sequence

<220>
<223> Probe Sequence

<400> 256
catcgagagc gcacacaaga cgccactgta aaaggatcac agat 44

<210> 257
<211> 46
<212> DNA
<213> Artificial Sequence

<220>
<223> Probe Sequence

<400> 257
ggagttccag gagattcaac caggatgttt ctacacctgt gggtta 46

<210> 258
<211> 45
<212> DNA
<213> Artificial Sequence

<220>
<223> Probe Sequence

<400> 258
cagccaccat caaagcccat tcgtaggaaa ttcagaccag aaaac 45

<210> 259
<211> 46

<212>  DNA
<213>  Artificial Sequence

<220>
<223>  Probe Sequence

<400>  259
tttggaaggc attgaagctt gcaccttttc atgtacagca ttaaaa                    46


<210>  260
<211>  45
<212>  DNA
<213>  Artificial Sequence

<220>
<223>  Probe Sequence

<400>  260
gaccaggtct atcagcccct ccgagatcga gatgatgctc agtac                     45


<210>  261
<211>  44
<212>  DNA
<213>  Artificial Sequence

<220>
<223>  Probe Sequence

<400>  261
atccaacaca gccagaaccc gcgattctac cacaagtgac catc                      44


<210>  262
<211>  51
<212>  DNA
<213>  Artificial Sequence

<220>
<223>  Probe Sequence

<400>  262
ttgcctggag aaagagaaga aaatattttt taaaaagcta gtttatttag c              51


<210>  263
<211>  47
<212>  DNA
<213>  Artificial Sequence

<220>
<223>  Probe Sequence

<400>  263
tcacccctga cacaacattt tcagaattcc agacgatact gtgataa                   47


<210>  264
<211>  45
<212>  DNA
<213>  Artificial Sequence

<220>
<223>  Probe Sequence

<400>  264
acggaaacag acccctgctt tcgaatttac atgttcatga tgtgc                    45

<210>  265
<211>  43
<212>  DNA
<213>  Artificial Sequence

<220>
<223>  Probe Sequence

<400>  265
cttcgcccct cccttgtttt atattttatg aagttagtgc ggg                       43

<210>  266
<211>  49
<212>  DNA
<213>  Artificial Sequence

<220>
<223>  Probe Sequence

<400>  266
accaaccaat actcaggaga accctgccta tgaggaatat ataagacca                 49

<210>  267
<211>  47
<212>  DNA
<213>  Artificial Sequence

<220>
<223>  Probe Sequence

<400>  267
tgtttggaaa atcacatcat gcctagaatc tgaaattgaa ttagcaa                   47

<210>  268
<211>  49
<212>  DNA
<213>  Artificial Sequence

<220>
<223>  Probe Sequence

<400>  268
gccaagagaa tcagagaaag atgctgcatt ttataatcaa agcccaaac                 49

<210>  269
<211>  54
<212>  DNA
<213>  Artificial Sequence

<220>
<223>  Probe Sequence

<400>  269
cgttacagta ttctgattat attactgaca cagtcaaaat gattaactgt acaa           54

<210>  270
<211>  47
<212>  DNA
<213>  Artificial Sequence

<220>
<223>  Probe Sequence

<400>  270
ttgtgcctgt gtgttaccat gctaagaatg tctttgttta aagggaa                    47


<210>  271
<211>  46
<212>  DNA
<213>  Artificial Sequence

<220>
<223>  Probe Sequence

<400>  271
gcaacagcaa gctgtagagc gggccaatga taaatcacat tgaatc                     46


<210>  272
<211>  45
<212>  DNA
<213>  Artificial Sequence

<220>
<223>  Probe Sequence

<400>  272
aagccaaagg aactggaggc actgatttaa tgaatttcct gaaga                      45


<210>  273
<211>  43
<212>  DNA
<213>  Artificial Sequence

<220>
<223>  Probe Sequence

<400>  273
tttcctcttg atcgggaact cctgcttctc cttgcctcga aat                        43


<210>  274
<211>  46
<212>  DNA
<213>  Artificial Sequence

<220>
<223>  Probe Sequence

<400>  274
ccaggtacaa tggcagagcc tttccatacc tgtactcaca actagc                     46


<210>  275
<211>  47
<212>  DNA
<213>  Artificial Sequence

<220>
<223>  Probe Sequence

<400>  275
tggtcttctt gcatcgatga tccaacagca acaccatttt taaatta                47


<210>  276
<211>  48
<212>  DNA
<213>  Artificial Sequence

<220>
<223>  Probe Sequence

<400>  276
tgtccctttg atacttgtgc tctgctgaga atgtacagtt tgcattaa              48


<210>  277
<211>  45
<212>  DNA
<213>  Artificial Sequence

<220>
<223>  Probe Sequence

<400>  277
gagaggcagc attgcacagt gaaagaattc tggatatctc aggag                 45


<210>  278
<211>  47
<212>  DNA
<213>  Artificial Sequence

<220>
<223>  Probe Sequence

<400>  278
ttggatgaga ttaggagatc agaggctgga ccttctcttg ataatgc               47


<210>  279
<211>  52
<212>  DNA
<213>  Artificial Sequence

<220>
<223>  Probe Sequence

<400>  279
aattgttgac attcatgtct ctgagttaca aaagtgctaa ttcactacat gt         52


<210>  280
<211>  46
<212>  DNA
<213>  Artificial Sequence

<220>
<223>  Probe Sequence

<400>  280
ggggaaattc aagcagtgtt tcctcaacca gtcacataga actctg                46

<210>  281
<211>  38
<212>  DNA
<213>  Artificial Sequence

<220>
<223>  Probe Sequence

<400>  281
ctgtccagcc gcatggaggg catgatggct tcctacac                                38


<210>  282
<211>  43
<212>  DNA
<213>  Artificial Sequence

<220>
<223>  Probe Sequence

<400>  282
tgtcaatctg tcctcggctg cccttctcat ttgttgatgg gac                          43


<210>  283
<211>  48
<212>  DNA
<213>  Artificial Sequence

<220>
<223>  Probe Sequence

<400>  283
ggagactttc acaagtggtt tccatggaga tagaatgaag cattctgt                     48


<210>  284
<211>  51
<212>  DNA
<213>  Artificial Sequence

<220>
<223>  Probe Sequence

<400>  284
agtagtttct ccaagtactt ttgtgctatc aatgagttct tctcaaaaaa t                 51


<210>  285
<211>  47
<212>  DNA
<213>  Artificial Sequence

<220>
<223>  Probe Sequence

<400>  285
tttctttgct aagccttgca tgcaaaattt gaaattttaa cattggc                      47


<210>  286
<211>  44
<212>  DNA

<213>  Artificial Sequence

<220>
<223>  Probe Sequence

<400>  286
acaaaacgag tccagcgacg aggagagcct gagaaaagag agag                        44


<210>  287
<211>  46
<212>  DNA
<213>  Artificial Sequence

<220>
<223>  Probe Sequence

<400>  287
aaatgagggc ccgtaacaga accagtgtgt gtataacgaa aaccat                      46


<210>  288
<211>  50
<212>  DNA
<213>  Artificial Sequence

<220>
<223>  Probe Sequence

<400>  288
tcacctcagt ctctaattgg ctgtgagtca gtctttcatt tacatagggt                  50


<210>  289
<211>  46
<212>  DNA
<213>  Artificial Sequence

<220>
<223>  Probe Sequence

<400>  289
cgtccagcca agagctcttc atctgctaca agaacatttg aatctt                      46


<210>  290
<211>  47
<212>  DNA
<213>  Artificial Sequence

<220>
<223>  Probe Sequence

<400>  290
gattgcaatg atgatgtcca aggtaagcta ttaaaaggca ggttact                     47


<210>  291
<211>  44
<212>  DNA
<213>  Artificial Sequence

<220>
<223>  Probe Sequence

<400>  291
cttgccagca gcaatcattt ggggaagaat ctacagttgc tgat                        44


<210>  292
<211>  48
<212>  DNA
<213>  Artificial Sequence

<220>
<223>  Probe Sequence

<400>  292
ttcctttggg agaaacctgt tcattccaat cttctaatta cagtggtt                    48


<210>  293
<211>  42
<212>  DNA
<213>  Artificial Sequence

<220>
<223>  Probe Sequence

<400>  293
caggacatca tgagcaggca gcagggagag agcaaccaag ag                          42


<210>  294
<211>  47
<212>  DNA
<213>  Artificial Sequence

<220>
<223>  Probe Sequence

<400>  294
actcggattc ttttgcatga tggggtaaag cttagcagag aatcatg                     47


<210>  295
<211>  44
<212>  DNA
<213>  Artificial Sequence

<220>
<223>  Probe Sequence

<400>  295
gacgtgaagt ctcgggcaaa gcgttatgag aagctggact tcct                        44


<210>  296
<211>  46
<212>  DNA
<213>  Artificial Sequence

<220>
<223>  Probe Sequence

<400>  296
cctctgtgtt cacttcgcct tgctcttgaa agtgcagtat tttctct                     46


<210>  297

<211>  50
<212>  DNA
<213>  Artificial Sequence

<220>
<223>  Probe Sequence

<400>  297
tcaggtgtca tcactgttca aaaggtaagc acatttagaa ttttgttctt                50


<210>  298
<211>  42
<212>  DNA
<213>  Artificial Sequence

<220>
<223>  Probe Sequence

<400>  298
cccttacccc tctctgggcc catgaattcc tggcttggtt ta                        42


<210>  299
<211>  46
<212>  DNA
<213>  Artificial Sequence

<220>
<223>  Probe Sequence

<400>  299
gggaaaacat ccatgctgga ctcctgaaga agttaaatga actgga                    46


<210>  300
<211>  46
<212>  DNA
<213>  Artificial Sequence

<220>
<223>  Probe Sequence

<400>  300
ctccaccaga agggcacact ttcatctaat ttggggtatc actgag                    46


<210>  301
<211>  51
<212>  DNA
<213>  Artificial Sequence

<220>
<223>  Probe Sequence

<400>  301
ttctctcaaa atactaaaca gaggtggttt tattgataag attttggctg t              51


<210>  302
<211>  48
<212>  DNA
<213>  Artificial Sequence

<220>

<223>  Probe Sequence

<400>  302
gcatgctgtt gtacatgatc ctgacaagaa gaaaatgaag ctcaaagt                    48


<210>  303
<211>  40
<212>  DNA
<213>  Artificial Sequence

<220>
<223>  Probe Sequence

<400>  303
gttctcccct ctggcccctg gagagaaggg agcattccta                             40


<210>  304
<211>  46
<212>  DNA
<213>  Artificial Sequence

<220>
<223>  Probe Sequence

<400>  304
tcttggtggg tcaagacttt ctgataaatc agttagcacc atgcat                      46


<210>  305
<211>  47
<212>  DNA
<213>  Artificial Sequence

<220>
<223>  Probe Sequence

<400>  305
ctggaataat ggaaagaaat gggggctttg gagaactagg atgtttc                     47


<210>  306
<211>  45
<212>  DNA
<213>  Artificial Sequence

<220>
<223>  Probe Sequence

<400>  306
gcccacatgg atagcacagt tgtcagacaa gattccttca gattc                       45


<210>  307
<211>  49
<212>  DNA
<213>  Artificial Sequence

<220>
<223>  Probe Sequence

<400>  307
cgccttcctc tttttaagct gttttatgaa aaagacctag aagttcttg                   49

<210>  308
<211>  43
<212>  DNA
<213>  Artificial Sequence

<220>
<223>  Probe Sequence

<400>  308
ctccaagccg atcaccaaga gtaaatcaga agcaaacctc atc                43


<210>  309
<211>  44
<212>  DNA
<213>  Artificial Sequence

<220>
<223>  Probe Sequence

<400>  309
aaagttgtgt aagcgcctgc gttcttctgg gtttggctag atag               44


<210>  310
<211>  46
<212>  DNA
<213>  Artificial Sequence

<220>
<223>  Probe Sequence

<400>  310
atggactgct ttgctggatt ggcactgagc aactttagga aatgtc             46


<210>  311
<211>  47
<212>  DNA
<213>  Artificial Sequence

<220>
<223>  Probe Sequence

<400>  311
tgatgaaata acttggggcg ttgaagagct gtttaatttt aaatgcc            47


<210>  312
<211>  47
<212>  DNA
<213>  Artificial Sequence

<220>
<223>  Probe Sequence

<400>  312
gctcgcccct gttttttgta gaatctcttc atgcttgaca tacctac             47


<210>  313
<211>  46
<212>  DNA
<213>  Artificial Sequence

<220>
<223>  Probe Sequence

<400>  313
cataggtgcc atcgtggttg agacaagtgc aaaaaatgct attaat                    46


<210>  314
<211>  47
<212>  DNA
<213>  Artificial Sequence

<220>
<223>  Probe Sequence

<400>  314
   tgtctgtgtc agacgtacag ccagacatgt tctctattgg cattttt                   47


<210>  315
<211>  44
<212>  DNA
<213>  Artificial Sequence

<220>
<223>  Probe Sequence

<400>  315
caggcctggt gctcagtcgt acgacctgta cctctcaact tttg                      44


<210>  316
<211>  46
<212>  DNA
<213>  Artificial Sequence

<220>
<223>  Probe Sequence

<400>  316
aactcctgcg atcagcttgt gacttacaaa ccttgtttaa aagctg                    46


<210>  317
<211>  48
<212>  DNA
<213>  Artificial Sequence

<220>
<223>  Probe Sequence

<400>  317
tcagtgagag actccaggac tttgacaaaa gcaagcatgt catctatg                  48


<210>  318
<211>  47
<212>  DNA
<213>  Artificial Sequence

<220>
<223>  Probe Sequence

<400>  318

agccacttgc cccagttcat aaccccatta gtgtctaaga agatttc 47

<210> 319
<211> 48
<212> DNA
<213> Artificial Sequence

<220>
<223> Probe Sequence

<400> 319
caccagggac acatttctct gtcttttttg atcagtgtcc tatacatc 48

<210> 320
<211> 47
<212> DNA
<213> Artificial Sequence

<220>
<223> Probe Sequence

<400> 320
gtcagcttgc ccaggttcaa actggaagag agttacactc tcaactc 47

<210> 321
<211> 46
<212> DNA
<213> Artificial Sequence

<220>
<223> Probe Sequence

<400> 321
tgatacccac cgggtctgac attccaagta accagtatgt aactgg 46

<210> 322
<211> 53
<212> DNA
<213> Artificial Sequence

<220>
<223> Probe Sequence

<400> 322
ttgtgcagat agtatttctg attgatgtca tctatcaaga atttcaagag att 53

<210> 323
<211> 46
<212> DNA
<213> Artificial Sequence

<220>
<223> Probe Sequence

<400> 323
aagactgtca ggaagggtcg gagtctgtaa aaccagcata cagttt 46

<210> 324
<211> 51

<212>  DNA
<213>  Artificial Sequence

<220>
<223>  Probe Sequence

<400>  324
aatgggcata aagcttcaca ctagtaacaa aaatggctta actttattac a                51


<210>  325
<211>  47
<212>  DNA
<213>  Artificial Sequence

<220>
<223>  Probe Sequence

<400>  325
tcacatcagg gcaaatgaaa tatccatcaa ctccagcatt tatcatt                     47


<210>  326
<211>  45
<212>  DNA
<213>  Artificial Sequence

<220>
<223>  Probe Sequence

<400>  326
tgcctttttg cctttggtaa cataactctg ggagtcttgg tttat                        45


<210>  327
<211>  47
<212>  DNA
<213>  Artificial Sequence

<220>
<223>  Probe Sequence

<400>  327
ccactggtca tgctgtggaa aatttaatga gaaatctgaa tgcacat                     47


<210>  328
<211>  45
<212>  DNA
<213>  Artificial Sequence

<220>
<223>  Probe Sequence

<400>  328
cattccgctc aaaggtcact gagacttttg cctcacctaa agaga                       45


<210>  329
<211>  50
<212>  DNA
<213>  Artificial Sequence

<220>
<223>  Probe Sequence

<400>  329
tgaggaaatc aaagtgctat tacgaagttc aagatcaaaa aggcttataa                50

<210>  330
<211>  47
<212>  DNA
<213>  Artificial Sequence

<220>
<223>  Probe Sequence

<400>  330
gctttgggga ggacaaaact tgtaagtaca gtcaaggaca agacttg                   47

<210>  331
<211>  43
<212>  DNA
<213>  Artificial Sequence

<220>
<223>  Probe Sequence

<400>  331
acgttccagg gcccaaagcc cagctctttg ttcagttgac tta                       43

<210>  332
<211>  47
<212>  DNA
<213>  Artificial Sequence

<220>
<223>  Probe Sequence

<400>  332
tggccaaatt agatgtgtgc tgaagacaat cagtcactgg gtctata                   47

<210>  333
<211>  48
<212>  DNA
<213>  Artificial Sequence

<220>
<223>  Probe Sequence

<400>  333
tggcttgtca ttctgtacac tgaccttagg catggagaaa attacttg                  48

<210>  334
<211>  49
<212>  DNA
<213>  Artificial Sequence

<220>
<223>  Probe Sequence

<400>  334
tcatgaattt tttaatccca ttgcaaacat tattccaaga gtatcccag                 49

<210>  335
<211>  42
<212>  DNA
<213>  Artificial Sequence

<220>
<223>  Probe Sequence

<400>  335
ccccgtctcc ctcccaactt atacgacctg atttccttag ga                          42


<210>  336
<211>  48
<212>  DNA
<213>  Artificial Sequence

<220>
<223>  Probe Sequence

<400>  336
ccacacagcc aagctgagac tgtggcaatg tgttgagtca tatacatt                    48


<210>  337
<211>  46
<212>  DNA
<213>  Artificial Sequence

<220>
<223>  Probe Sequence

<400>  337
attacctcag tccccgagga cagttttgaa ggacttgttc agttac                      46


<210>  338
<211>  43
<212>  DNA
<213>  Artificial Sequence

<220>
<223>  Probe Sequence

<400>  338
gatgcaccag gcgaggaaac gagacctctt tcgttccttc tag                         43


<210>  339
<211>  46
<212>  DNA
<213>  Artificial Sequence

<220>
<223>  Probe Sequence

<400>  339
atgcctgagg catgagtgac tgtgcatttg agatagtttt ccctat                      46


<210>  340
<211>  50
<212>  DNA
<213>  Artificial Sequence

<220>
<223>  Probe Sequence

<400>  340
tgaaaaagaa caagagctgg acacattaaa aagaaagagt ccatcagatt                50


<210>  341
<211>  43
<212>  DNA
<213>  Artificial Sequence

<220>
<223>  Probe Sequence

<400>  341
cagaaggacc tgggggatgg cgtgtatggc ttcgagtatt acc                       43


<210>  342
<211>  45
<212>  DNA
<213>  Artificial Sequence

<220>
<223>  Probe Sequence

<400>  342
agggctgtca tcaacatgga tatgacattt cacaacagtg actag                     45


<210>  343
<211>  43
<212>  DNA
<213>  Artificial Sequence

<220>
<223>  Probe Sequence

<400>  343
tggctttgca cagaaccagc tagttatttg gaagtaccca acc                       43


<210>  344
<211>  47
<212>  DNA
<213>  Artificial Sequence

<220>
<223>  Probe Sequence

<400>  344
tgtgggttag catcaagttc tccccagggt agaattcaat cagagct                   47


<210>  345
<211>  48
<212>  DNA
<213>  Artificial Sequence

<220>
<223>  Probe Sequence

<400>  345
caatgcaaaa agtattcgct gctgtttaca ttagaaatca cttccagc                  48

<210>  346
<211>  47
<212>  DNA
<213>  Artificial Sequence

<220>
<223>  Probe Sequence

<400>  346
tgtacagtgg aaccatgtga ccatgtcttg tgcttgcaat atagaaa                47


<210>  347
<211>  48
<212>  DNA
<213>  Artificial Sequence

<220>
<223>  Probe Sequence

<400>  347
ggcaaatcct tcaagcaggg ataaaagtcg atcttcaaac attaactt                48


<210>  348
<211>  47
<212>  DNA
<213>  Artificial Sequence

<220>
<223>  Probe Sequence

<400>  348
gggtttgggg gagattttac tcctttcttc aacaactatt cactgga                47


<210>  349
<211>  46
<212>  DNA
<213>  Artificial Sequence

<220>
<223>  Probe Sequence

<400>  349
ttttaggcct ttgggggaat tgatttttat ccacaggtag aaaatg                46


<210>  350
<211>  47
<212>  DNA
<213>  Artificial Sequence

<220>
<223>  Probe Sequence

<400>  350
ctatgcagga aaatagcacc ccccgtgagg actaatccag atacatc                47


<210>  351
<211>  47
<212>  DNA

<213>  Artificial Sequence

<220>
<223>  Probe Sequence

<400>  351
aggtatggcc tcacaagtct tggtctaccc accatatgtt tatcaaa                47


<210>  352
<211>  47
<212>  DNA
<213>  Artificial Sequence

<220>
<223>  Probe Sequence

<400>  352
aggaggtgtc agactgctga agccgactct gaaagtgatc atgaagt                47


<210>  353
<211>  45
<212>  DNA
<213>  Artificial Sequence

<220>
<223>  Probe Sequence

<400>  353
gagggcatgt tgtccatatc cctgtggaat acagaccgtg taact                  45


<210>  354
<211>  48
<212>  DNA
<213>  Artificial Sequence

<220>
<223>  Probe Sequence

<400>  354
tctaatgtgc acggtgtgac tggcagagtg agtttaaaag ctttacga               48


<210>  355
<211>  49
<212>  DNA
<213>  Artificial Sequence

<220>
<223>  Probe Sequence

<400>  355
tgttgccaag ctagtctaca aagcatctga ttttggaagt acatggaat              49


<210>  356
<211>  46
<212>  DNA
<213>  Artificial Sequence

<220>
<223>  Probe Sequence

<400>  356
ttacaccttt cccccctgaa atgtatagaa tccatttgtc atcagg                    46

<210>  357
<211>  46
<212>  DNA
<213>  Artificial Sequence

<220>
<223>  Probe Sequence

<400>  357
aattggtgaa caaaaaatgc ccaaggcttc tcatgtcttt attctg                    46

<210>  358
<211>  49
<212>  DNA
<213>  Artificial Sequence

<220>
<223>  Probe Sequence

<400>  358
ggagaattct ttaggttgtc ccctaaagat tctgaaaaag agaatcaga                 49

<210>  359
<211>  46
<212>  DNA
<213>  Artificial Sequence

<220>
<223>  Probe Sequence

<400>  359
gtagcctcac cattagtggc agcatcatgt aactgagtgg actgtg                    46

<210>  360
<211>  46
<212>  DNA
<213>  Artificial Sequence

<220>
<223>  Probe Sequence

<400>  360
tttgctcaca agccatattg gcccgattag tggtactgtc tgactc                    46

<210>  361
<211>  48
<212>  DNA
<213>  Artificial Sequence

<220>
<223>  Probe Sequence

<400>  361
tgagcttaca acaggtctcg agctggtgga ctcctgtatt aggtcact                  48

<210>  362

<211>  46
<212>  DNA
<213>  Artificial Sequence

<220>
<223>  Probe Sequence

<400>  362
gcatgcagat gtcaaggcag ttaggaagta aatggtgtct tgtaga                    46